# EUROPEAN PATENT APPLICATION

(11) **EP 1 908 827 A2**
(43) Date of publication of application: **09.04.2008**
(21) Application number: 07123326.6
(22) Date of filing: 17.09.2004
(51) Int. Cl.: C12N 15/00, A01K 67/027, C12N 9/12, G01N 33/50, C12N 15/85

(54) **Regulation of cardiac contractility and heart failure propensity**

(30) Priority: 19.09.2003 US 503853 P
(62) Divisional of application: 04809768.7
(71) Applicant: Children's Hospital Medical Center, Cincinnati, OH 45229-3039 (US); UNIVERSITY OF CINCINNATI, Cincinnati, OH 45221 (US)
(72) Inventor: Molkentin, Jeffery Daniel, Cincinnati, OH 45231 (US); Kranias, Evangelia Galani, Cincinnati, OH 45243 (US)
(74) Representative: Hammann, Heinz

(57) **Abstract**

The methods and compositions of the present invention find use in altering expression of PKCα in transgenic animals. The compositions of the invention include isolated transgenic animal cells, transgenic tissue, transgenic animals, and transgenic mice. The transgenic animals of the invention exhibit altered PKCα activity. The methods allow generation of transgenic animals with altered expression of PKCα. The invention allows modulation of cardiac contractility. In particular, the invention provides a method for altering the susceptibility of a transgenic animal to cardiomyopathy. A transgenic animal of the invention finds use in identifying anti-cardiomyopathic compounds.

## Description

### GOVERNMENT GRANT INFORMATION

This invention was made with Government support under NIH Grant Nos. HL62927, HL26057, and HL64018. The United States Government has certain rights in this invention.

### FIELD OF THE INVENTION

This invention relates to modulation of cardiac contractility and cardiomyopathic phenotypes, prevention and treatment of the same, and transgenic mice related to the same.

### BACKGROUND OF THE INVENTION

Heart failure afflicts an estimated 5 million Americans, with approximately 400,000 new individuals diagnosed each year at an annual cost of over $20 billion (Lloyd-Jones et al. (2002) Circulation 106:3068-3072). The predominant therapeutic strategy employed over the past two decades has been based on pharmacological manipulation of cardiac contractility (Remme, W.J. (2001) Cardiovasc. Drugs Ther. 15:375-377; Felker et al (2001) Am. J. Heart 142:393-401; Packer, M. (2001) Am. J. Med. 110 Suppl 7A:81S-94S). Heart failure may be characterized by a progressive loss in contractility, ventricular chamber dilation, increased peripheral vascular resistance, and / or dysregulated fluid homeostasis. Positive inotropic agents were initially employed as a means of enhancing cardiac pump function, yet are now only utilized to acutely bridge patients in severe heart failure since they worsen long-term survival (Felker et al (2001) Am. J. Heart 142:393-401). More recently, pharmacological blockade of β-adrenergic receptors has emerged as the favored treatment for heart failure, although it remains uncertain whether or not β-blockers benefit the myocardium by diminishing cardiac contractility (short-term) or augmenting it (long-term) (Packer, M. (2001)Am. J. Med. 110 Suppl 7A:81S-94S; Bristow M.W. (2000) Circulation 101:558-569; Bouzamondo et al. (2001) Fundam. Clin. Pharmacol. 15:95-109). Two other features associated with the failing human heart are a dysregulation in calcium homeostasis and an increase in neuroendocrine stimulatory agents that signal through both Gαq- and Gαs-coupled receptors.

A variety of human diseases and conditions manifested by cardiac abnormalities or cardiac dysfunction may lead to heart failure. Heart failure is a physiological condition in which the heart fails to pump enough blood to meet the circulatory requirements of the body. The study of such diseases and conditions in genetically diverse humans is difficult and unpredictable. Therefore, there is a need for a model system that facilitates the identification of potential therapeutic agents of cardiomyopathy.

When stimulated by an array of neuro-humoral factors or when faced with an increase in ventricular wall tension, the myocardium undergoes an adaptive hypertrophic response. Cardiac hypertrophy is an adaptive response of the heart to many forms of cardiac disease, including those arising from hypertension, mechanical load, myocardial infarction, cardiac arrhythmias, endocrine disorders, and / or genetic mutations in cardiac contractile protein genes. While the hypertrophic response is initially a compensatory mechanism that augments cardiac output, sustained hypertrophy can lead to heart failure, and sudden death.

The causes and effects of cardiac hypertrophy have been documented, but the underlying mechanisms that couple hypertrophic signals initiated at the cell membrane to the reprogramming of cardiomyocyte gene expression remain poorly understood. Elucidation of these mechanisms is a central issue in cardiovascular biology and is important for designing new strategies for prevention or treatment of cardiac hypertrophy and heart failure.

Studies have implicated intracellular Ca²⁺ as a signal for cardiac hypertrophy. In response to myocyte stretch or increased loads on working heart preparations, intracellular Ca²⁺ concentrations increase (Marban et al. (1987) Proc. Natl. Acad. Sci. USA 84:6005-6009; Bustamante et al. (1991) J. Cardovasc. Pharmacol. 17:S1110-S113; and Hongo et al. (1995) Am. J. Physiol. 269:C690-C697), consistent with a role of Ca²⁺ in coordinating physiological responses with enhanced cardiac output.

Hypertrophic stimuli result in reprogramming of gene expression in the adult myocardium, such that genes encoding fetal protein isoforms like β-myosin heavy chain (MHC) and α-skeletal actin are up-regulated, whereas the corresponding adult isoforms α-MHC and α-cardiac actin, are down-regulated. The natriuretic peptides, atrial natriuretic factor and b-type natriuretic peptide, which decrease blood pressure by vasodilation and natriuresis, are also rapidly up-regulated in the heart in response to hypertrophic signals. (Komuro and Yazaki (1993) Ann. Rev. Physiol. 55:55-75). The mechanisms involved in coordinately regulating these cardiac genes during hypertrophy are unknown.

A number of signaling molecules have been characterized as important transducers of this disease response sequelae, including, but not limited to, specific G-protein isoforms, low molecular weight GTPases (Ras, RhoA, Rac), mitogen-activated protein kinases (MAPK), protein kinase C (PKC), calcineurin, gp130-STAT, insulin-like growth factor-1 receptor, fibroblast growth factor, and transforming growth factorβ, For example, binding of the cell surface receptors for AngII, PE, and ET-1 leads to activation of phospholipase C, resulting in the production of diacylglycerol and inositol triphosphate, mobilization af intracellular Ca²⁺, and activation of protein kinase C. The extent to which these signaling pathways interact during cardiac hypertrophy is unknown (Molkentin et al. (2001) Annu. Rev. Physiol. 63:391-426).

The protein kinase C (PKC) family of calcium and/or lipid-activated serine-threonine kinases functions downstream of nearly all membrane-associated signal transduction pathways (Molkentin et al. (2001) Annu. Rev. Physiol. 63:391-426). Approximately 12 different isozymes comprise the PKC family, which are broadly classified by their activation characteristics. The conventional PKC isozymes (PKCα, βI, βII, and γ) are calcium- and lipid-activated, while the novel isozymes (ε, θ, η, and δ) and atypical isozymes (ζ, 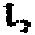 υ, and µ) are calcium independent but activated by distinct lipids (Dempsey et al. (2000) Am. J. Physiol. Lung Mol. Physiol. 279:247-251). Once activated, PKC isozymes translocate to discrete subcellular locations through direct interactions with docking proteins termed RACKs (Receptor for Activated C Kinases), which permit specific substrate recognition and subsequent signal transduction (Mochly-Rosen, D (1995) Science 268:247-251).

Reports have associated PKC activation with hypertrophy, dilated cardiomyopathy, ischemic injury, or mitogen stimulation (DeWindt et al. (2000) J. Biol. Chem. 275:13571-13579; Gu & Bishop (1994) Circ. Res. 75:926-931; Jalili et al. (1999) Am. J. Physiol. 277:H2298-H2304; Takeishi et al (1999) Am. J. Physiol. 276:H53-H62). For example, hemodynamic pressure overload stimulation in rodents promotes translocation of PKCα, β, γ, ε, and θ. In diverse cultured cardiomyocytes, agonists and stress stimuli are also potent activators of PKC isozyme translocation. Isozyme-specific peptide inhibitors have been employed in cultured cardiomyocytes and in transgenic mice to afford greater specificity of PKC inhibition. Specifically, overexpression of a PKCβ C₂ domain peptide in cardiomyocytes blocked phorbol ester-mediated calcium channel activity (Zhang et al. (1997) Circ. Res. 80:720-729), while a PKCε inhibitory or activating peptide affected inotropy and ischemia-induced cellular injury (Gray et al. (1997) J. Biol. Chem. 272:30945-30951; Johnson et al. (1996) J. Biol. Chem. 271:24962-24966; Dorn et al. (1999) Proc. Natl. Acad. Sci USA. 96:12798-12803). Additionally, adenovirus-mediated gene transfer of PKCε into cultured adult rabbit ventricular myocytes augmented basal myocyte contractility and calcium transients. The results in myocytes suggest that PKCε functions to enhance cardiac contractile performance (Baudet et al, (2001) Cardiovasc. Res. 50:486-494).

Phorbol esters exert acute biologic effects on metazoan cells, mostly consistent with immediate activation of multiple PKC isozymes. In cardiac myocytes, PMA is a potent inducer of many PKC isozymes including, but not limited to, PKCα, β, δ, and ε translocation and activation (Braz et al. (2002) J. BioL Chem. 156:905-919). Thus acute PMA administration may be used to examine the immediate, but non-specific effects of PKC translocation on alterations in cardiac inotropy and contractility. Acute phorbol ester administration has been used to assess the hypothesis that PKC isozymes regulate, in part, the contractile performance of the whole heart or isolated myocytes. For example, using isolated chicken ventricular myocytes, PMA treatment produced a concentration and time-dependent decrease in the amplitude of cell shortening, reaching a maximum of a 54% decrease at 1 µM drug (Leatherman et al. (1987) Am. J. Physiol. 253:H205-209), Consistent with this effect, PMA produced a decrease in intracellular calcium concentration and the rate of calcium reuptake. In contrast, PMA pretreatment of papillary muscles from the heart potentiated alpha 1-adenoceptor-mediated positive inotropy, demonstrating the non-selective effects of using PMA (Otani et al. (1988) Circ. Res. 62:8-17). An analysis performed in isolated ventricular myocytes from adult rats showed that PMA has an acute negative contractile effect (Capogrossi et al. (1990) Circ. Res. 66:1143-1155). These authors used steady field stimulation of adult myocytes at 1 Hz in 1 mM calcium, after which PMA (10⁻⁷ M) was applied resulting in a decrease in twitch amplitude to approximately 60% of control. In single cardiac myocytes myofilament responsiveness to calcium was not affected by PMA, but that the negative inotropic effect was due to diminished amplitude of the calcium transient. In contrast to this report, a separate study using a slightly different phorbol ester, 12-O-tetradecanoylphorbol 13-acetate (TPA), showed significant increases in cell shortening and an increase in the rate of change in cell length during relaxation, suggesting enhanced contractility by activation ofPKC isozyme(s) (MacLeod et al. (1991) J. Physiol. 444:481-498). A more elaborate study in both isolated cardiac myocytes and whole guinea-pig hearts showed a significant positive inotropic response with 10⁻¹² M PMA, but a negative inotropic response at concentrations higher than 10⁻¹⁰ M PMA (Ward and Moffat (1992) J. Mol. Cell Cardiol. 24:937-948). The results discussed above suggest that phorbol ester-mediated alterations in cardiac contractility are complex.

Transgenic mice have been generated with altered PKC isozyme expression in the heart. Overexpression of either wild-type or a constitutively active deletion mutant of PKCβ in a mouse heart was reported to induce cardiomyopathy (Wakasaki et al. (1997) Proc. Natl. Acad. Sci. USA 94;9320-9325; Bowman et al (1997) J. Clin. Invest. 100:2189-2195) but more recent investigation has suggested that lower levels of expression or adult onset PKCβ activation benefits ischemic recovery (Tiang et al. (1999) Proc. Natl. Acad. Sci. 96:13536-13541; Huang et al. (2001) Am. J. Physiol. Cell. Physiol. 280:C1114-C1120). Three groups have also reported transgenic mice with altered PKCε or PKCδ activity in the heart. Expression of a PKCε or PKCδ activating peptide in the mouse heart was associated with a physiologic activation of each isozyme and a mild hypertrophic response (Mochly-Rosen *et al.* (2000) *supra*; Chen et al. (2001) Proc. Natl. Acad. Sci. 98:11114-11119) Similarly, overexpression of an activated mutant PKCε cDNA in the mouse heart was reported to induce significant cardiac hypertrophy (Takeishi et al. (2000) Circ. Res. 86:1218-1223), but such a result is likely dependent on the absolute levels of PKCε overexpression and activity (Pass et al. (2001) Am. J. Physiol. Heart Circ, Physiol. 280:H946-H955). While a number of studies have demonstrated associations between various PKC isozymes and cardiac hypertrophy or ischemic injury, the necessary and sufficient functions of specific PKC isozymes are still debated. For example, while transgenic overexpression of PKCβ, δ, or ε in the mouse heart can initiate cardiac hypertrophy, gene targeting for these 3 isoforms did not overtly affect the heart, nor were *PKCβ* null mice defective in their ability to mount a hypertrophic response (Roman et al. (2001) Am. J. Physiol. Heart Circ. Physiol. 280:H2264-H2270). Collectively these results highlight the confusion in the art as to the PKC isozymes' roles as regulators of cardiac contractility. PKCα knock out mice have also been generated by Legites et al. Mol. Endocrinol. 16, 847-858) showing that PKCα enhances insulin signaling through PI3K.

PKCα is the predominant PKC isoform expressed in the small and large mammal heart, yet little is understood of its function in this organ (Pass *et al.* (2001) *supra;* Ping et al. (1997) Circ. Res. 81:404-414). While a number of correlative studies have been published showing associations between PKCα activation and cardiac hypertrophy or heart failure, almost no causal or mechanistic data have been reported. Gain- and loss-of function analysis of PKC isozyme function using cultured neonatal cardiac myocytes and recombinant adenoviruses expressing either wild-type or dominant negative mutants of PKCα, β, δ, and ε has been performed. It was reported that PKCα. regulates the hypertrophic growth of cultured neonatal myocytes in part through ERK1/2, but its role in cardiac contractility is unknown (Braz *et al.* (2002) *supra).* Similarly, antisense phosphorothioate oligonucleotides against PKCα in cultured neonatal cardiac myocytes reduced hypertrophic gene expression following agonist stimulation (Kerke1a et al. (2002) Mol. Pharmacol. 62:1482-1491). However, none, of these observations include a mechanical assessment of PKCα's in vivo.

Little is understood of the role that various PKC isoforms play in potentially regulating cardiac contractility. PKC isoforms are known to directly phosphorylate sarcomeric proteins such as cTnI, which has been reported to affect the rate of maximal ATPase activity due to actinmyosin interactions (de Tombe & Solaro. (2000) Ann. Biomed. Eng. 28:991-1001). However, it remains unclear if PKC-mediated phosphorylation of contractile proteins significantly alters cardiac performance, in contrast to the well characterized effects of PKA.

Thus, a mechanistic assessment of PKCα's *in vivo* role is desirable. It is of importance to develop methods of modulating PKCα activity in cardiac tissue. It is also important to develop a model transgenic system for identifying PKCα modulating and anti-cardiomyopathic compounds and studying cardiomyopathies.

Treatment of heart failure in humans is based, in part, on the underlying causes, if known, and other factors including the severity of the disease, existing medications and other coinciding risk factors (for example, coronary artery disease, hypertension, valvular defects or hyperlipidemia). Advanced heart failure in patients may consist of both acute and chronic presentations, which may require varying treatments. Thus, current heart failure strategies target either acute decompensated heart failure (ADHF) or the chronic remodeling effects of heart failure. Treatment of ADHF is an unmet medical need, serving as the primary diagnosis for approximately 1 million hospital admissions per year in the United States and is the secondary diagnosis for another 2 million hospitalizations *(*DiDomenico RJ et al. (2004) Ann Pharmacother. 38:649-660). ADHF is marked by functional deficits due to acute injury to the heart, e.g., myocardial infarction, arrhythmia, or may be precipitated by complications of chronic heart failure, e.g., progressive LV remodeling, cardiomegaly and myocyte loss (Cleland JG et al. (2001) Prog. Cardiovasc. Dis. 43:433-455). In either case, the patient requires immediate intervention for successful outcomes. Current treatments for ADHF depend largely on symptoms upon presentation to the emergency room, but may include inotropes (such as dobutamine and milrinone), intravenous diuretics (such as furosemide) and / or vasodilators (such as Nesiritide (RTM) in order to improve myocardial performance and maintain sufficient cardiac output (DiDomenico RJ *et al. supra).* The goal of treatments using these drugs is to enhance or restore cardiac contraction and relaxation acutely and provide symptomatic improvement. In addition to ADHF, the drugs mentioned above may be administered in any setting of cardiac dysfunction (such as left ventricular dysfunction as a result of sepsis) when it is deemed medically necessary for survival, regardless of the etiology. In chronic heart failure, the drug regime is distinct and commonly includes agents such as angiotensin-converting enzyme inhibitors, angiotensin receptor blockers, diuretics and/or β-adrenergic receptor blockers, These drugs are not administered for ADHF and some may in fact be counter-productive in this setting (e,g., β-adrenergic receptor blockers). While these drugs provide little or no immediate improvement in cardiac contraction or relaxation, they have been demonstrated to improve survival and cardiac remodeling in heart failure patients (Aronow WS. (2003) Heart Dis. 5:279-294). Therefore, there is a need to identify novel targets and their modulators to provide sufficient acute and chronic benefits in heart failure.

### SUMMARY OF THE INVENTION

The inventions are based on the novel discovery that PKCα regulates cardiac contractility and cardiomyopathy and therefore both acute decompensated heart failure (ADHF) and chronic heart failure. Accordingly, it is believed that modulation of PKCα activity may provide therapeutic means for enhancing cardiac inotropy and ventricular performance. Transgenic animals of the invention are useful in identifying compounds for prophylaxis and treatment of disorders modulated by cardiac contractility, and cardiomyopathy including cardiac hypertrophy. These animals are also useful for investigative purposes, for examining signal transduction pathways involved in response to hypertrophic signals. The invention also details a process for measuring PKCα activation in vivo to screen for pharmacologic modulators of PKCα activity.

Compositions of the invention include transgenic mice, transgenic cells and transgenic tissues. In an embodiment, transgenic mice, cells and tissues of the invention comprise an expression cassette comprising a cardiac tissue-preferred regulatory sequences operably linked to a PKCα nucleotide sequence (SEQ ID NO: 1, NCBI Accession No. X04796) or a fragment or variant thereof. A variant is set forth in SEQ ID NO: 7, and encodes a polypeptide (SEQ ID NO: 8) exhibiting a dominant-negative effect. The cell expressing the expression cassette exhibits altered PKCα expression or activity. In another aspect, the transgenic mouse of the invention exhibits altered cardiac contractility. In another aspect, a mouse of the invention exhibits altered susceptibility to cardiomyopathy.

In another aspect, transgenic mice, transgenic cells and transgenic tissue comprise at least one disrupted PKCα gene. In one aspect, the disruption is sufficient to decrease or eliminate PKCα expression levels. In another aspect, a PKCα null mouse of the invention exhibits altered cardiac contractility. In another aspect, a mouse of the invention exhibits an altered susceptibility to cardiomyopathy.

In one aspect, methods of identifying compounds that modulate cardiac contractility are disclosed, comprising: providing a first and a second cell, tissue, or mouse, expressing a PKCα gene; administering a compound of interest to said first cell; incubating both the first and second cells for a suitable, predefined period of time; measuring the activity of PKCα in said first and said second cell; and identifying those compounds that modulate the activity of PKCα. in said first cell compared to activity in said second cell as modulators of cardiac contractility.

In another aspect, methods of identifying compounds that modulate cardiomyopathy are disclosed, comprising: providing a first and a second cell expressing PKCα protein; administering a compound of interest to said first cell; incubating both the first and second cells for a suitable, predefined period af time; measuring the activity of PKCα in said first and said second cell; and identifying those compounds that modulate the activity of PKCα in said first cell compared to activity in said second cell as modulators of cardiomyopathy.

In another aspect, methods of identifying compounds that modulate PKCα activity are disclosed, comprising: providing a first and a second cell expressing PKCα, protein; administering a compound of interest to said first cell; incubating both the first and second cells for a suitable, predefined period of time; measuring the activity of PKCα in said first and said second cell; and identifying those compounds that modulate the activity of PKCα in said first cell compared to activity in said second cell as modulators of PKCα activity.

For the above-described assays to identify compounds that modulate cardiac contractility, cardiomyopathy, and PKCα activity; any cell expressing suitable levels of PKCα protein could be used, e.g., standard laboratory-derived cell lines, cardiomyocyte cell lines, or any animal-derived primary cells, or tissues. Cells, and tissues from transgenic, or knock out mice; the transgenic animals themselves; and the dominant negative mutants of the invention are suitable for the purpose.

Modulators of PKCα activity include inhibitors or activators of the various PKCα activities, including, but not limited to, the enzymatic activity; the translocation activity; and the binding to various RACKs.

In another aspect, the compounds identified using above-described methods could be further validated using assays that utilize various cell culture, cultured tissues, or animal models of cardiac contractility, or cardiomyopathy as described herein.

In another embodiment, the invention provides a method of preferentially modulating PKCα activity in cardiac tissue. The method comprises providing a transgenic mouse comprising a stably incorporated expression cassette in the genome of at least one cell. The stably incorporated expression cassette comprises a cardiac preferred regulatory sequence operably linked to the PKCα nucleotide sequence set forth in SEQ ID NO: 1 or fragment or variant thereof. Variants of interest include, but are not limited to, dominant negative mutations such as the site directed mutant having the nucleotide sequence set forth in SEQ ID NO: 7. The invention further comprises determining the PKCα expression levels in the cardiac tissue of the mouse. In an aspect of the method, the mouse exhibits altered cardiac contractility. In another aspect, the mouse exhibits an altered susceptibility to cardiomyopathy.

In an embodiment, the invention provides a method of modulating PKCα expression in a mouse. The method comprises providing a transgenic mouse comprising at least one disrupted PKCα gene in the genome of at least one cell. The invention further comprises determining the PKCα expression levels in the mouse.

In an embodiment, the invention provides a method of treating or preventing an acute heart failure resulting from abnormal cardiac contractility in an animal. The method comprises the step of administering a PKCα modulating compound to the animal. In an aspect of the invention, the PKCα modulating compound is administered to the animal's cardiac tissue. In an aspect of the invention, the PKCα modulating compound is a PKCα inhibitor. In an aspect of the invention, the method increases the animal's cardiac contractility. Suitable animals include, but are not limited to, mice, guinea pigs, hamsters, humans, rabbits, dogs, pigs, goats, cows, rats, monkeys, chimpanzees, sheep, and zebrafish.

In an additional embodiment, the invention provides a method of treating or preventing a cardiomyopathy in an animal. The method comprises the step of administering a PKCα modulating compound to the animal. In an aspect of the invention, the PKCα modulating compound is administered to the animal's cardiac tissue. In an aspect of the invention, the PKCα modulating compound is a PKCα inhibitor or agonist. In an aspect of the invention, the method decreases the animal's susceptibility to cardiomyopathy. Suitable animals include, but are not limited to, mice, guinea pigs, hamsters, humans, rabbits, dogs, pigs, goats, cows, rats, monkeys, chimpanzees, sheep, and zebrafish.

PKCα inhibitors that may be used in the treatment of cardiac contractility or cardiomyopathy include, but are not limited to, nucleic acids, antibodies, small molecules, activator and inhibitor peptides, and Ro-32-0432, LY333531 and Ro-31-8220.

The invention also provides kits for performing a method of identifying a PKCα modulating compound, In an aspect of the invention a kit for identifying a PKCα modulating compound comprises a PKCα indicator polypeptide. In an aspect of the invention a kit for identifying a PKCα modulating compound comprises a cell comprising a PKCα indicator polypeptide.

### DESCRIPTION OF SEQUENCE LISTING

| **Name** | **Species** | **SEQ ID NO:** | | **Genbank Accession Number** |
|---|---|---|---|---|
| | | **DNA** | **Protein** | |
| PKCα | *Oryctolagus cuniculus* | 1 | 2 | X04796 |
| PKCα | *Mus musculus* | 3 | 4 | X52685 |
| PKCα | *Homo sapiens* | 5 | 6 | X52479 |
| PKCα dominant negative mutant | *Oryctolagus cuniculus* | 7 | 8 | |
| Deleted exon | *Mus musculus* | 9 | 10 | |
| 5' Long primer | *Mus musculus* | 11 | | |
| 5' short primer | *Mus musculus* | 12 | | |
| 3' Long primer | *Mus musculus* | 13 | | |
| 3' short primer | *Mus musculus* | 14 | | |
| α MHC promoter sequence | *Mus musculus* | 15 | | U7144I |

### BRIEF DESCRIPTION OF THE DRAWINGS

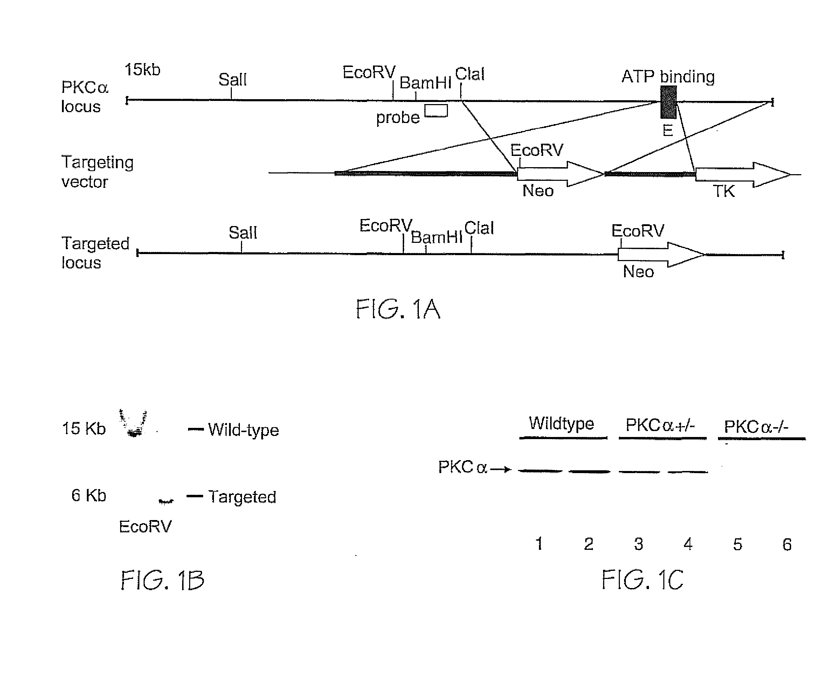

Figure 1 presents generation and characterization of the murine *PKC*α gene disruption transgenic mice. Details of the experiments are described elsewhere herein. Panel A depicts a schematic of the murine *PKCα* genomic locus and the targeting vector used to replace the ATP binding exon (E) with a neomycin resistance gene (neo). The approximate locations of SaII, EcoRV, and ClaI restriction enzyme sites are indicated. The approximate location of the nucleotide sequence used as a genomic probe to identify transgenic mice is also indicated. SEQ ID NO: 9 and 10 provide nucleotide and amino acid sequence of the exon deleted and SEQ ID NOs: 11-14 are the primers used to create the PCR products. Panel B depicts results of a Southern blot assay of embryonic stem cells. Lane 1 contains DNA from a wildtype cell, and Lane 2 contains DNA from a transgenic cell. Panel C depicts results of Western blot analysis of PKCα in protein preparations from hearts of wild-type, heterozygous (PKCα +/-), and PKCα-/transgenic mice. Proteins from wildtype mice are presented in lanes 1-2; proteins from PKCα+/- mice are presented in lanes 3-4; and proteins from *PKCα-*/*-* mice are presented in lanes 5-6.

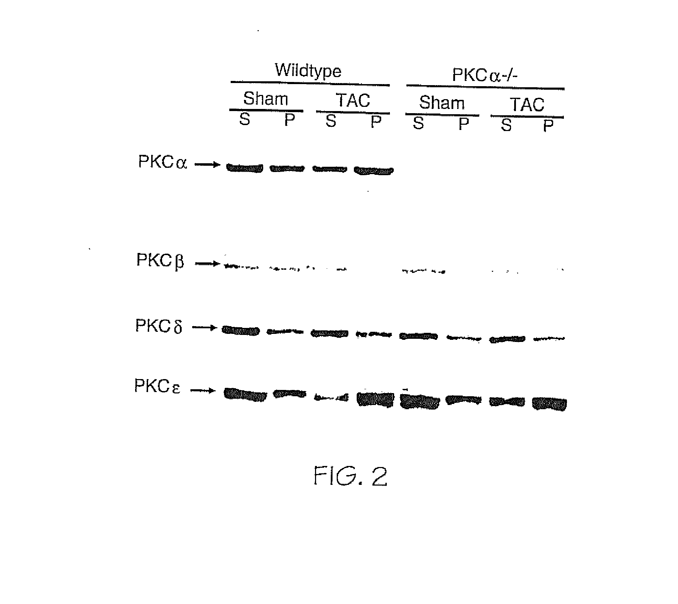

Figure 2 depicts results of Western blot analysis of PKCα, PKCβ, PKCδ, and PKCε in protein preparations from hearts of wildtype and *PKCα-*/*-* mice. Proteins from wildtype mice are presented in lanes 1-4; proteins from *PKCα-*/*-* mice are presented in lanes 5-8. Proteins in lanes I, 2, 5, and 6 were obtained from the hearts of animals that underwent a sham procedure. Proteins in lanes 3, 4, 7, and 8 were obtained from the hearts of animals that underwent transverse aortic constriction (TAC). The proteins were separated into soluble (S) (Lanes 1, 3, 5, and 7) and particulate (P) (Lanes 2, 4, 6, and 8) fractions prior to polyacrylamide gel electrophoresis.

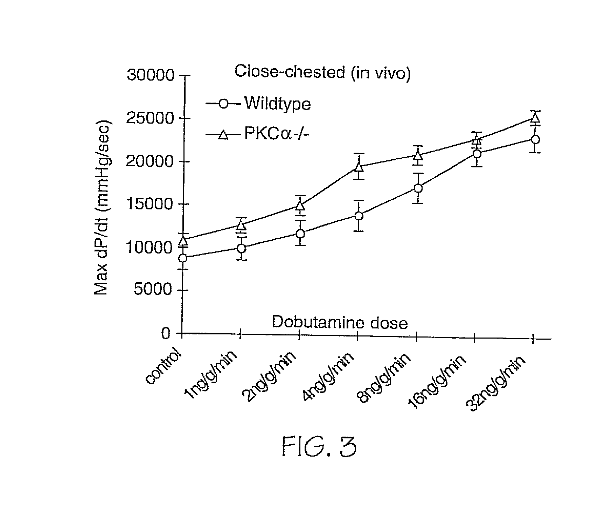

Figure 3 presents results obtained from invasive hemodynamic assessment of ventricular performance as maximal dP/dt in anesthetized close-chested mice at baseline or in response to increasing amounts of dobutamine infusion (β-agonist). Maximal dP/dt increments are in mmHg/sec. The dobutamine dose is indicated as ng dobutamine/ g mouse/ min. Results obtained from wildtype mice are indicated with circles (N=6). Results obtained from *PKCα-l-* mice are indicated with triangles (N=6). Experimental details are described elsewhere herein.

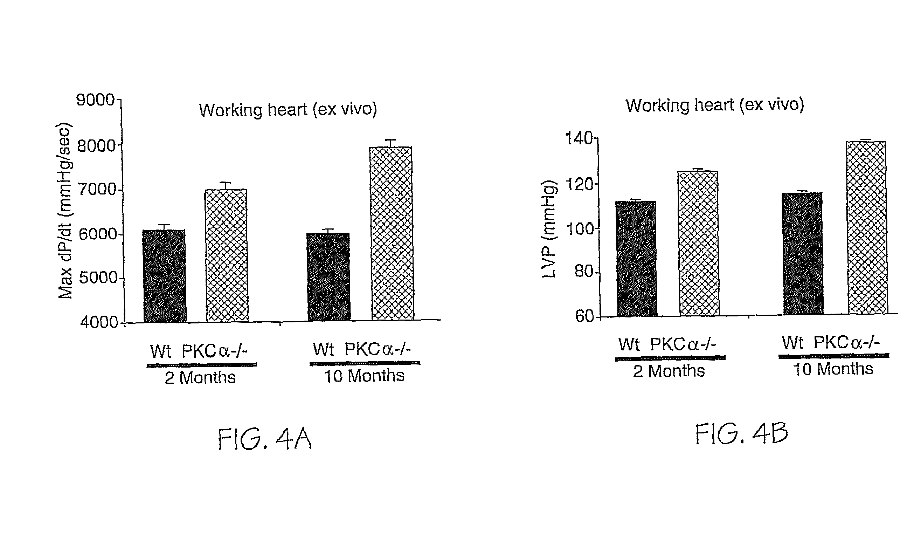

Figure 4 presents results of an analysis of cardiac ventricular performance in wild type (Wt) and PKCα homozygous deletion (PKCα-/-) mice. Results obtained from wildtype mice are indicated with solid bars. Results obtained from *PKCα-*/*-* mice are indicated with cross-hatched bars. In each panel, the first two bars indicate data obtained from 2 month old mice, and the last two bars indicate data obtained from 10 month old mice. (N=4 mice in each group). Panel A presents maximal dP/dt obtained from *ex vivo* working hearts. Panel B presents the left ventricular pressure (LVP) as measured in mmHg.

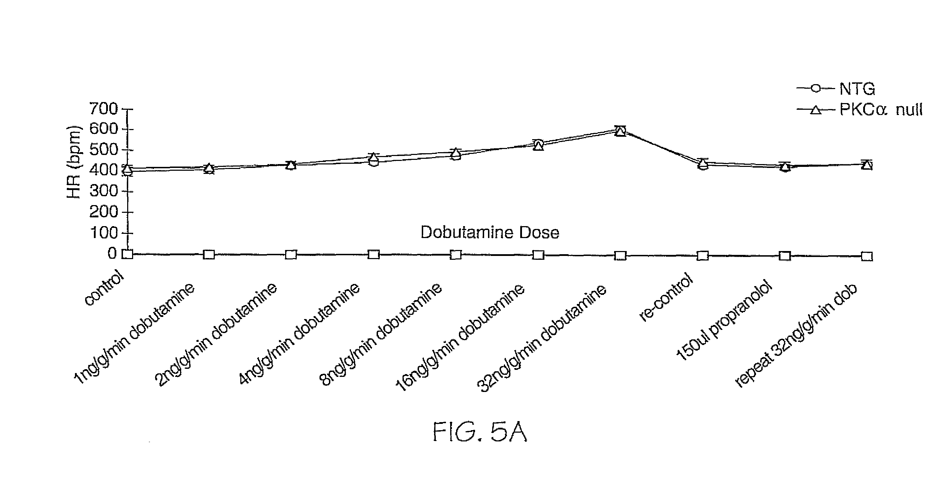

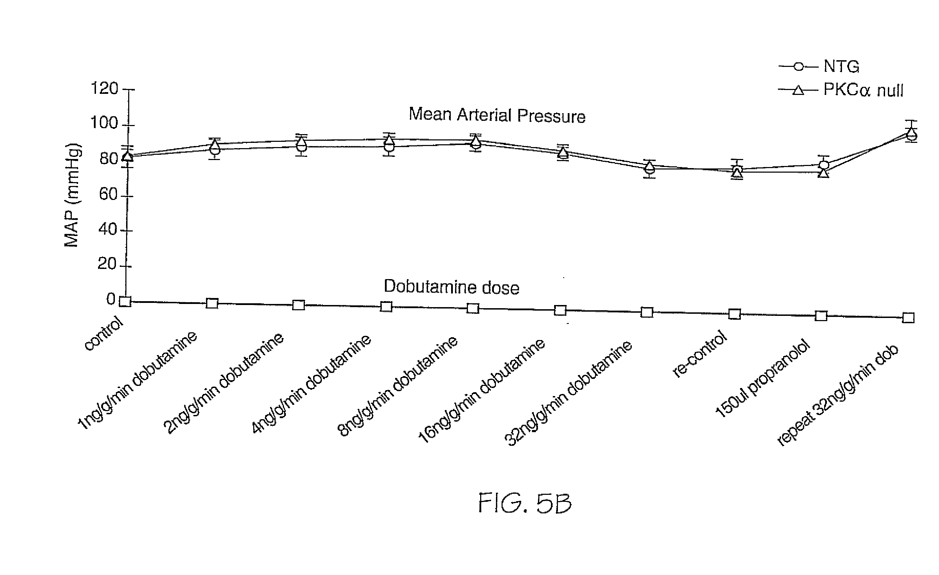

Figure 5 presents results obtained from wildtype (NTG, circles) and PKCα homozygous deletion (PKCα null, triangles) mice. Panel A presents the heart rate (HR) in beats per minute (bpm) in response to increasing dobutamine. Panel B presents the mean arterial pressure (MAP) in mmHg in response to increasing dobutamine. (Heart rate and mean arterial pressure were also assessed in response to propranolol and a repeated dobutamine dose.) Experimental details are described elsewhere herein.

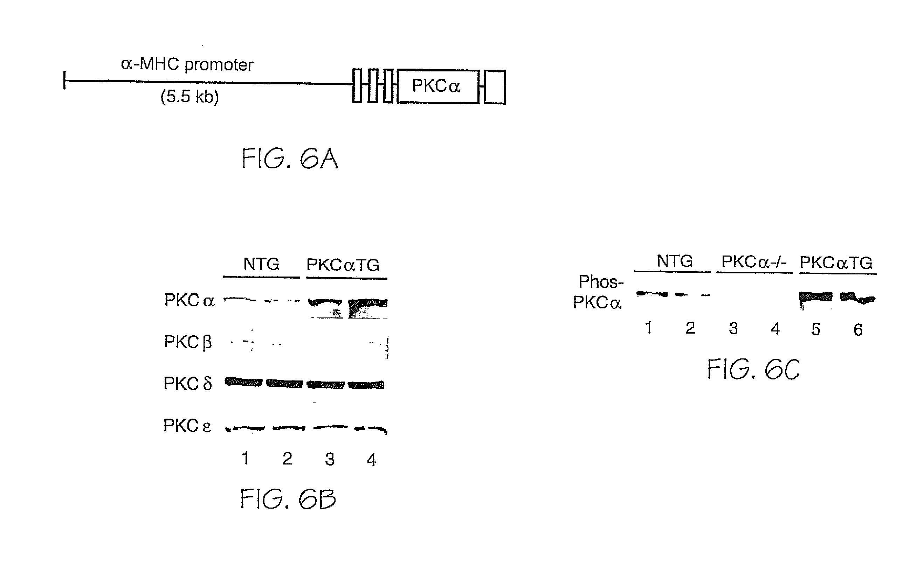

Figure 6 presents generation and characterization of PKCα transgenic mice. Experimental details are described elsewhere herein. Panel A depicts a schematic of the cardiac tissue-preferred (α-myosin heavy chain (α-MHC) promoter (Genbank U71441; SEQ ID NO: 15) operably linked to the rabbit PKCα gene (SEQ ID NO: 1). Panel B depicts results of Western blot analysis of PKC isoforms in protein preparations from hearts of wildtype and *PKCα* transgenic mice. The isoform of interest (PKCα, PKCβ, PKCδ, and PKCε) is indicated on the left hand side of the blots. Lanes 1 and 2 contain proteins from wildtype (NTG) mice; lanes 3 and 4 contain proteins from *PKCα* transgenic mice (PKCα TG). The *PKCα* transgenic mice are also referred to as PKCα overexpressing mice. Panel C depicts results of Western blot analysis with antibodies to the PKCα autophosphorylation site. Proteins were obtained from hearts of non-transgenic mice (lanes 1 and 2); *PKCα-l-* mice (lanes 3 and 4), and *PKCα* overexpressing transgenic mice (lanes 5 and 6).

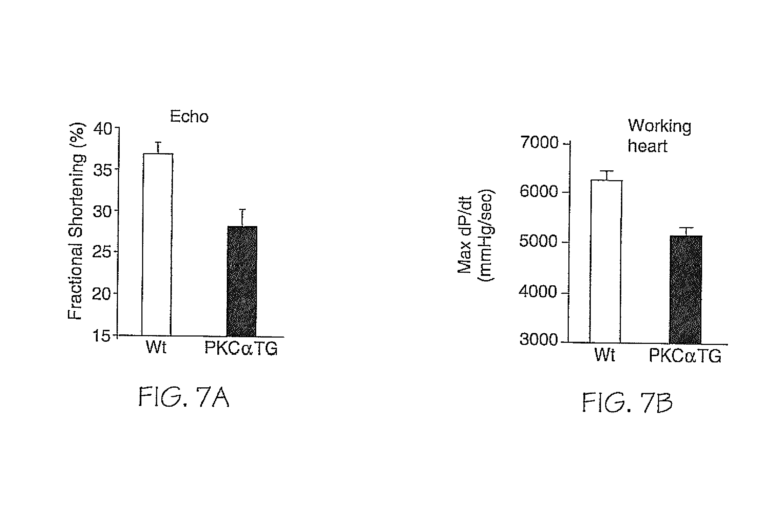

Figure 7 presents results of cardiac ventricular performance assessments. Results obtained from wildtype mice are indicated with a white bar; results obtained from *PKCα* transgenic mice are indicated with a solid bar. Panel A presents the results of an assessment of fractional shortening percentage by echocardiography. Panel B presents results obtained from analysis of ventricular performance by isolated working hearts as maximal dP/dt (Maximum dP/dt).

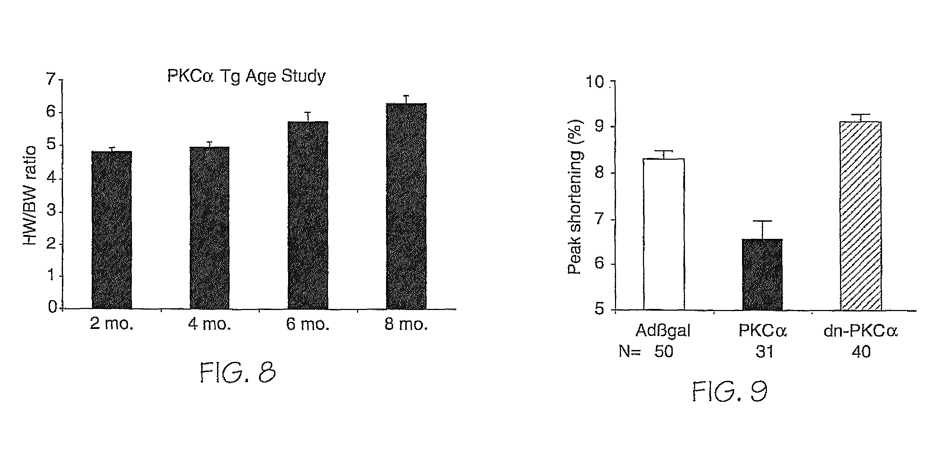

Figure 8 depicts results obtained from heart-weight (HW) to body-weight (BW) ratio analysis of cardiac hypertrophy in unstimulated male *PKC*α transgenic mice. Four mice were assessed at each time point (2, 4, 6, and 8 months).
Figure 9 presents results of a peak shortening assay performed on wildtype adult rat myocytes. The cells were infected with adenoviruses encoding β-galactosidase (Adβgal, white bar), wildtype PKCα (solid bar), and dominant negative PKCα (dn-PKCα, striped bar). The number of cells analyzed is shown below each bar.

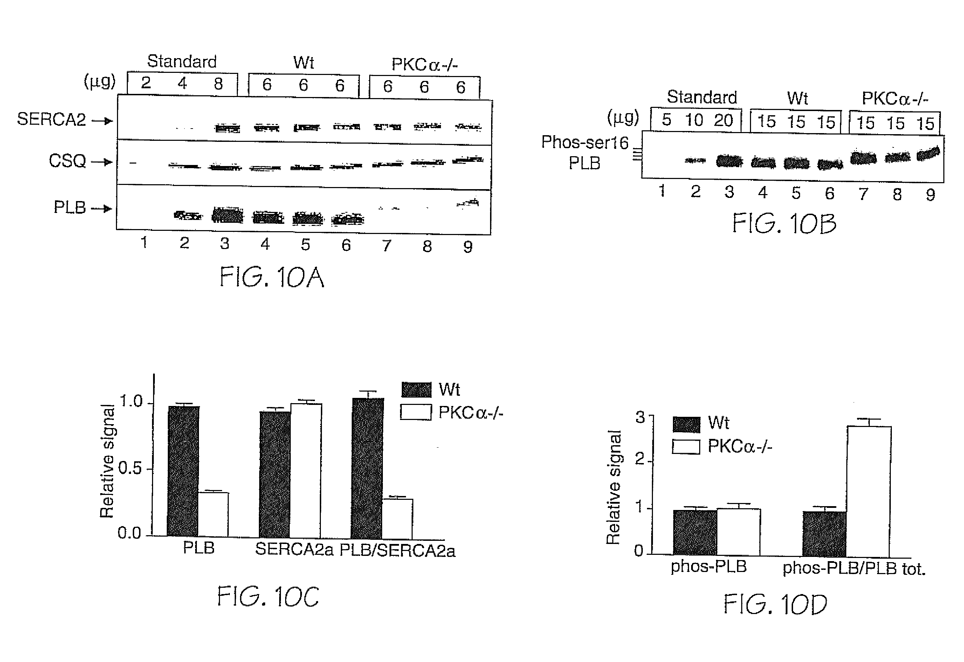

Figure 10 presents results from a series of assays involving alterations in PKCα activity and phospholamban phosphorylation status. Details of the experiments are described elsewhere herein. Panel A depicts results of a Western blot of protein from three wildtype (Wt, Lanes 4-6) and three PKCα-/- (Lanes 7-9) hearts at two months of age probed with antibodies to SERCA2, calsequestrin (CSQ), and phospholamban (PLB). Lanes 1-3 (Standard) were loaded with the indicated amount of protein. The relative quantitation of total phospholamban (PLB) versus SERCA2 is also presented (panel C). Data from wildtype hearts is indicated with solid bars; data from PKCα-/- hearts is indicated with white bars. Panel B depicts the quantitation results of a Western blot of protein from three wildtype (Wt, Lanes 4-6) and three PKCα-/- (Lanes 7-9) hearts probed with PLB serine 16 phospho-specific antibody. Lanes 1-3 (Standard) were loaded with the indicated amount of protein. The relative quantitation of total PLB (PLB tot) versus phosphorylated PLB (phos-PLB) is also presented (panel D). Data from wildtype hearts is indicated with solid bars; data from PKCα-/- hearts is indicated with white bars.

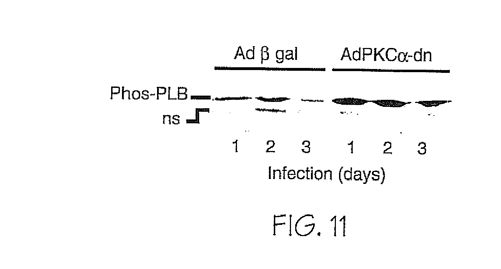

Figure 11 depicts the results of a Western blot of protein from wildtype adult rat ventricular myocytes infected with adenoviruses encoding β-galactosidase (Adβgal) or adenoviruses encoding dominant negative PKCα (AdPKCα-dn) for the indicated days. The blot was probed with PLB serine 16 phospho-specific antibody.

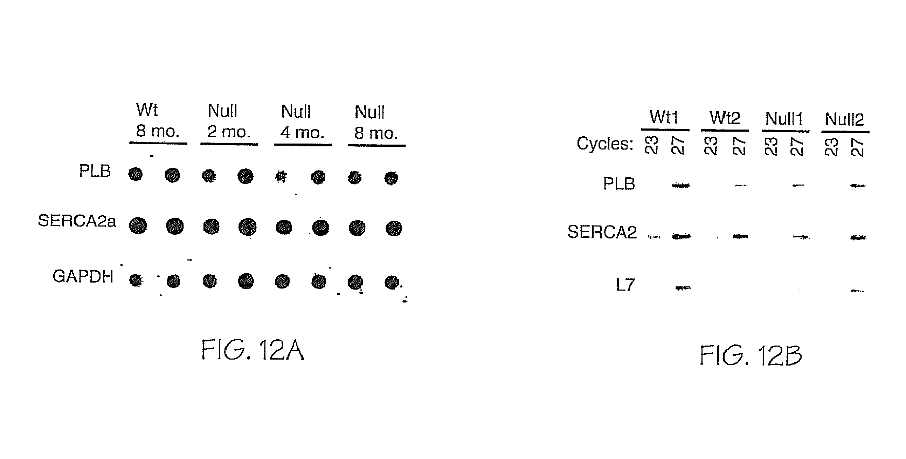

Figure 12, panel A presents the results of RNA dot blot analysis of wildtype (Wt) and PKCα -/- (Null) mice at the indicated ages. The dot blots were probed with phospholamban (PLB), SERCA2, and GAPDH specific probes. Panel B presents the results ofRT-PCR analysis of two wild-type and two PKCα-/- (Null) mice. The number of cycles performed is indicated. Primers specific to PLB, SERCA2a, and ribosomal protein L7 (L7) were used.

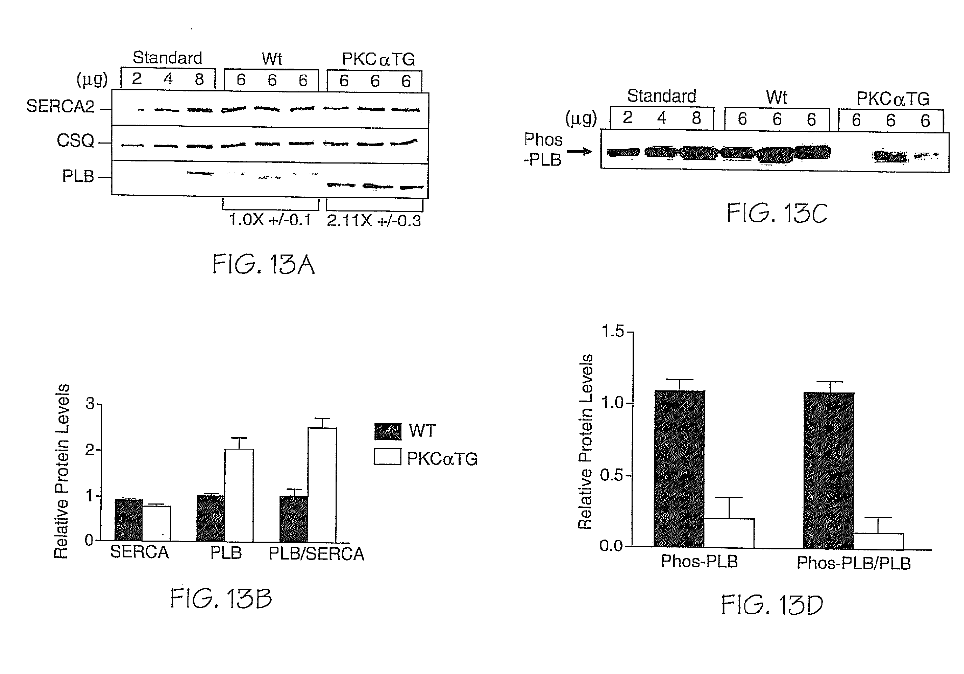

Figure 13 presents results from a series of assays involving alterations in PKCα levels and phospholamban phosphorylation status. Panel A depicts results of a Western blot of protein from three wildtype (Wt) and three PKCα transgenic (PKCα TG) hearts at two months of age probed with antibodies to SERCA2, calsequestrin (CSQ), and phospholamban (PLB). The relative quantification of total phospholamban (PLB) versus SERCA2a is presented in Panel B. Data from wildtype hearts is indicated with solid bars; data from PKCα TG hearts is indicated with white bars. Panel C depicts the results of a Western blot of protein from three wildtype (Wt) and three PKCα transgenic (PKCα TG) hearts probed with PLB serine 16 phospho-specific antibody. The first three lanes (Standard) were loaded with the indicated amount of protein. The relative quantification of total PLB (PLB tot) versus phosphorylated PLB (phos-PLB) is also presented in Panel D. Data from wildtype hearts is indicated with solid bars; data from PKCα TG hearts is indicated with white bars.

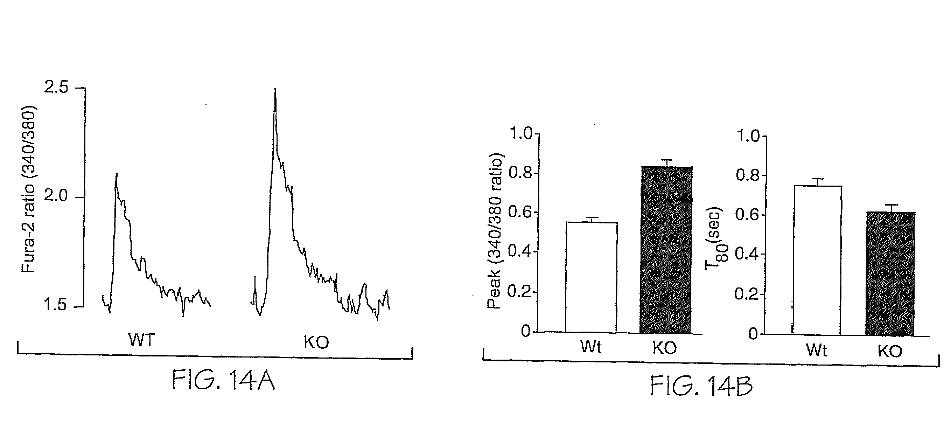

Figure 14 presents the results of a series of assays assessing the calcium transient in *PKCα-*/*-* cardiac myocytes. Details of the experiments are described elsewhere herein. Panel A depicts representative Fura-2 (340/380) emission tracing of calcium transients from an adult wildtype (WT) and *PKCα-*/*-* (KO) cardiomyocyte (2 months of age). Panel B presents the peak calcium release (left) and 80% of relaxation time (T₈₀, right) measured in seconds. Results from myocytes from wildtype mice are indicated by white bars. Results from myocytes from *PKCα-*/mice are indicated by solid bars.

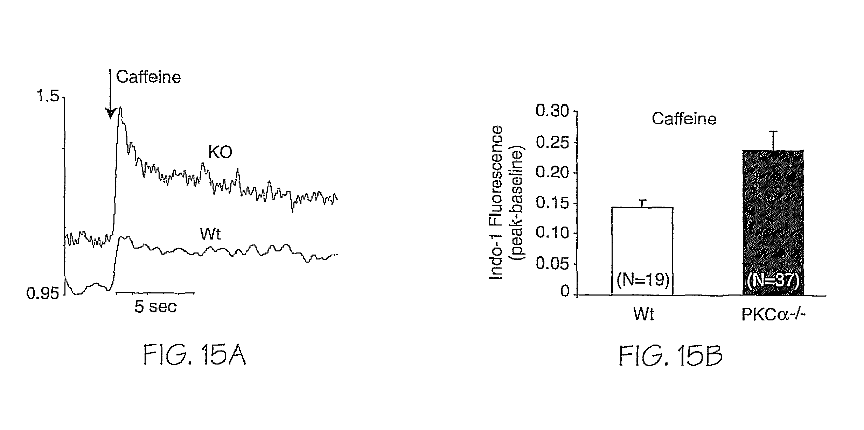

Figure 15, panel A presents representative Indo-1 AM emission tracings from wild-type (wt) and PKCα-/- (KO) myocytes prior to and subsequent to caffeine administration. The point of caffeine stimulation is indicated. Panel B presents results obtained from assessment of caffeine induced Ca²⁺ transients in myocytes. Wild-type myocytes (WT) are indicated with a white bar (n = 19); PKCα -/- (KO) myocytes are indicated with a solid bar (n = 37).

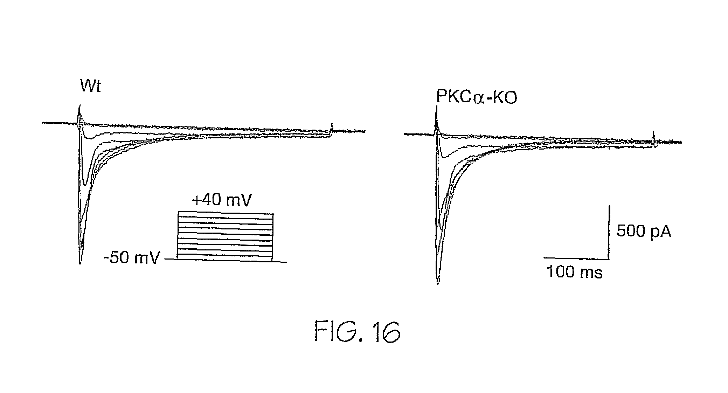

Figure 16 presents traces of mean peak calcium density (*I_{Ca}*) obtained at depolarizing voltage steps from -50 mV to +40 mV in 10 mV increments. Results from wildtype (NTG) cells are in the left trace; results from PKCα-/- (PKCα-KO) are in the right trace.

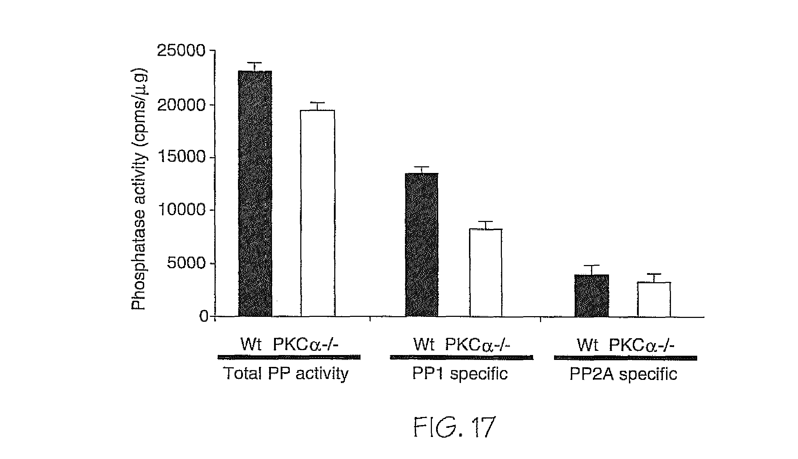

Figure 17 depicts the results of total phosphatase, PP1 specific, and PP2A specific enzymatic assays performed on wild-type (Wt, solid bars) and PKCα-/- mice (PKCα-/-, empty bars).

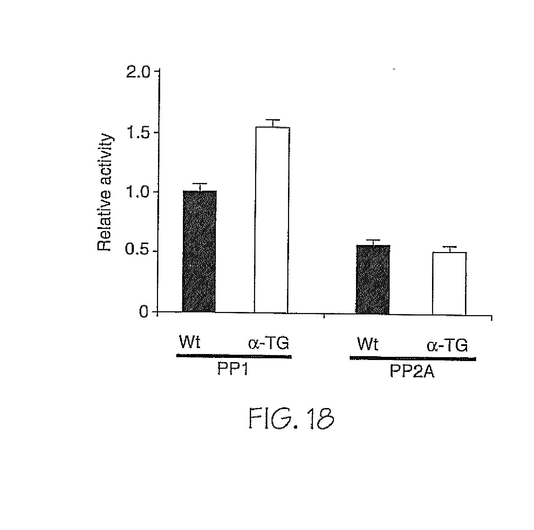

Figure 18 depicts the result of PP1 and PP2A-specific enzymatic assays from wildtype (Wt, solid bars) or PKCα transgenic (overexpressing) hearts (α-TG, empty bars). N=3 separate assays from 3 hearts each.

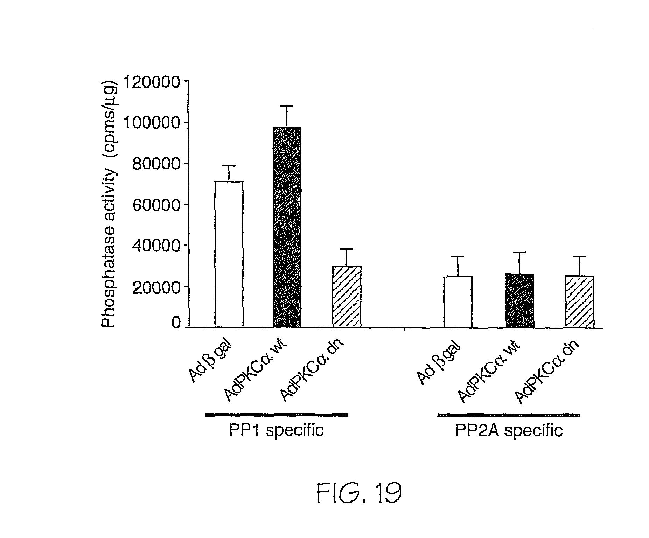

Figure 19 presents the results of PP1- and PP2A-specific enzymatic assays from neonatal cardiomyocytes acutely infected with the indicated adenoviruses: adenovirus encoding β-galactosidase (Adβgal, white bars); PKCα overexpressing adenovirus (AdPKCα wt, solid bars); and PKCα dominant negative adenovirus (AdPKCα dn, striped bars). Phosphatase activity is presented as counts per minute (cpms) per µg protein.

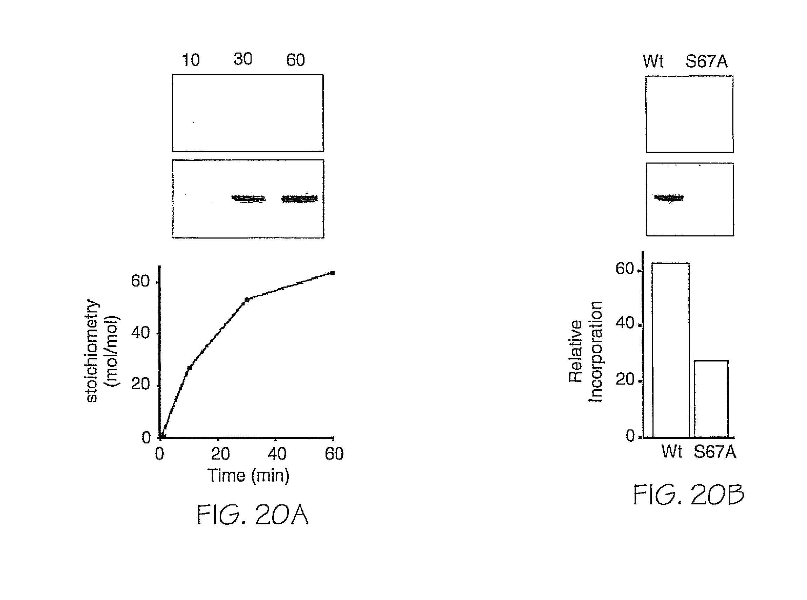

Figure 20, panel A presents an SDS-PAGE of *E*. *coli* purified Inhibitor-1 wildtype protein subjected to phosphorylation with ³²P-ATP and purified protein kinase C. Each lane contains an aliquot from the indicated time point (10, 30, or 60 minutes). The results are summarized in the graph below the gel. The graph depicts the amount of phosphorylated Inhibitor-1 protein at the indicated time points. Panel B presents an SDS-PAGE of *E. coli* purified I-1 wild-type (Wt) or S67A mutant protein subjected to phosphorylation with ³²P-ATP and purified protein kinase C. The graph below the gel indicates the relative amount of phosphorylated Inhibitor-1 wild-type or S67A protein.

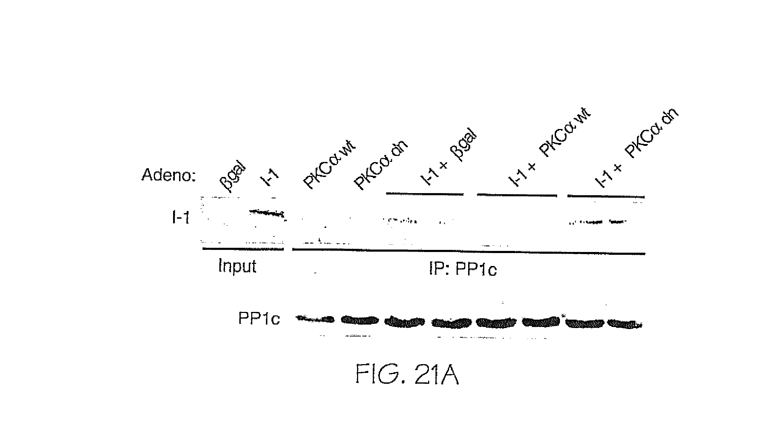

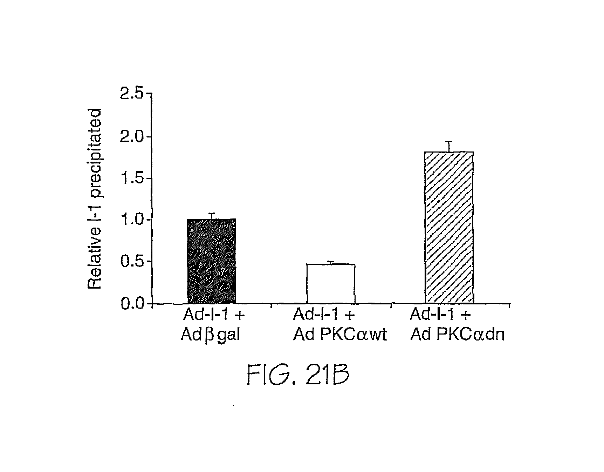

Figure 21, panel A presents a Western blot of extracts from adenoviral-infected neonatal cardiomyocyte cultures incubated with antisera to Inhibitar-1 (I-1). Extracts were prepared from cultures infected with adenovirus expressing β-galactosidase (βgal), Inhibitor-1 (I-1), PKCα wild-type (PKCα wt), PKCα dominant negative mutant (PKCα dn), Inhibitor-1 and β-galactosidase (I-1 + β-gal), Inhibitor-1 and PKCα wild-type (I-1 + PKCα wt), and Inhibitor-I and PKCα dominant negative (I-1 + PKCα dn). The extracts were immunoprecipitated with PPIc. The immunopreciptants were resuspended, electrophoresed, transferred to a membrane, and hybridized with anti-I-1 antisera. A membrane strip containing the PP1c protein band was hybridized with PP1c antisera (shown below the I-1 treated Western blot). The hybridized proteins were quantified and the results summarized in Panel B. Panel B depicts the relative amounts of 1-1 precipitated from each extract: Inhibitor-1 and β-galactosidase (Ad-I-1 + Ad-β-gal, solid bar), Inhibitor-1 and PKCα wild-type (Ad-I-1 + Ad PKCα wt, empty bar), and Inhibitor-1 and PKCα dominant negative (Ad-I-1 + AdPKCα dn, striped bar).

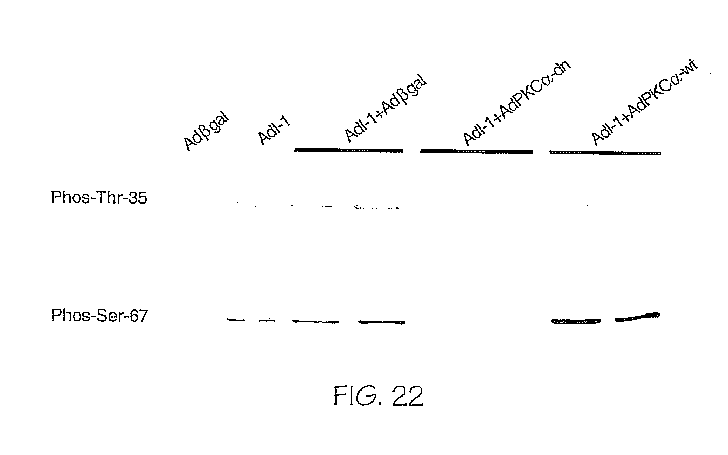

Figure 22 presents a Western blot with I-1 phospho-specific antibodies against threonine-35 and serine-67 from adenoviral-infected neonatal cardiomyocyte cultures.

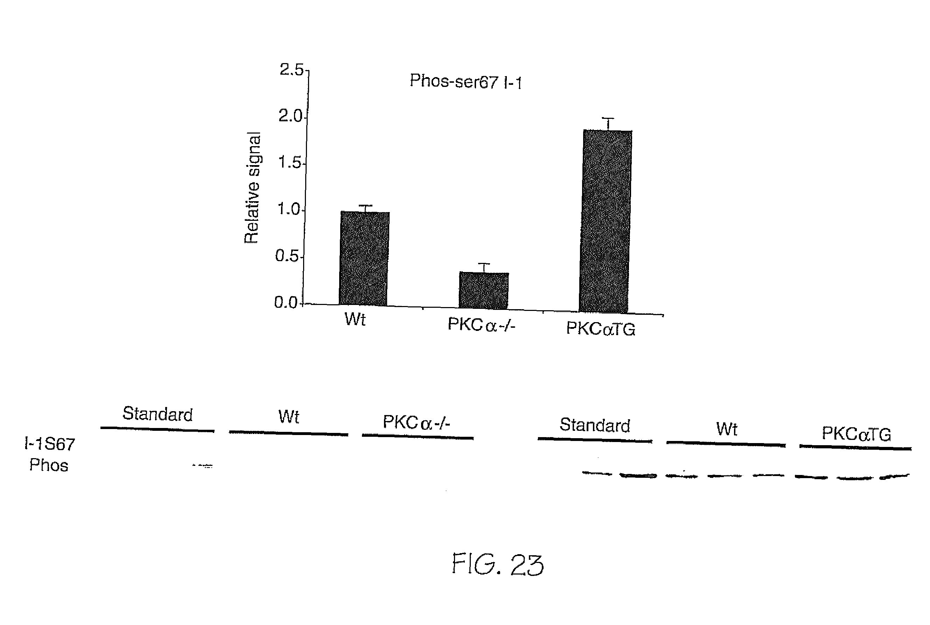

Figure 23 presents quantification of independent Western blots for I-1 phospho-serine 67 from wildtype, *PKCα-*/*-* and PKCα transgenic hearts. Typical Western blots are shown beneath the graph.

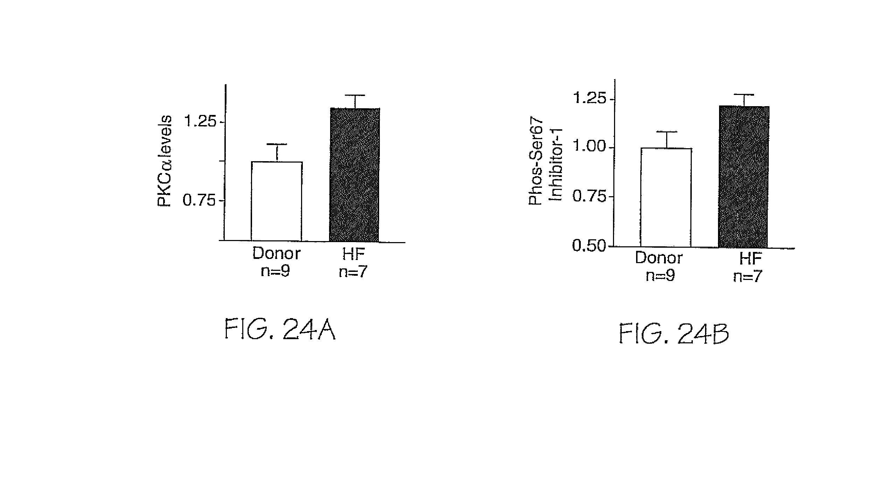

Figure 24, Panel A presents a quantification of western blotting for total PKCα protein levels in "normal" human donor hearts (empty bars, Donor) or dilated cardiomyopathic hearts (solid bars, HF) in failure. Panel B presents western blot quantification between PKCα levels and I-1 serine-67 phosphorylation in "normal" donor hearts (empty bars, Donor) and failing hearts (solid bars, HF).

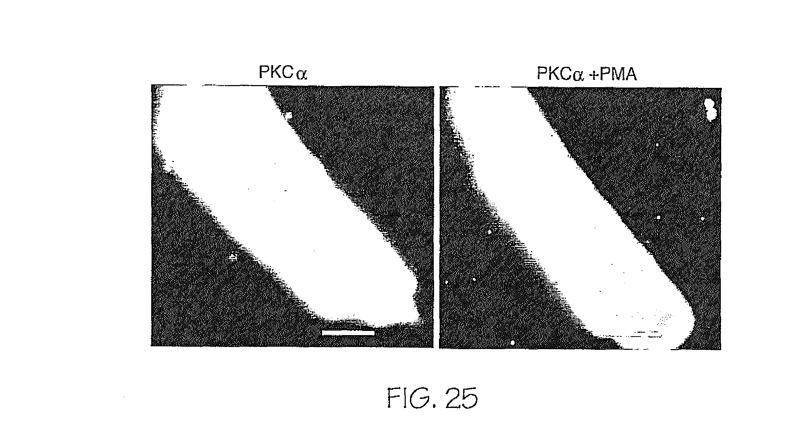

Figure 25 presents confocal micrographs of PKCα protein localization in adult rat cardiac myocytes at baseline (PKCα) or after PMA (PKCα, + PMA) stimulation.

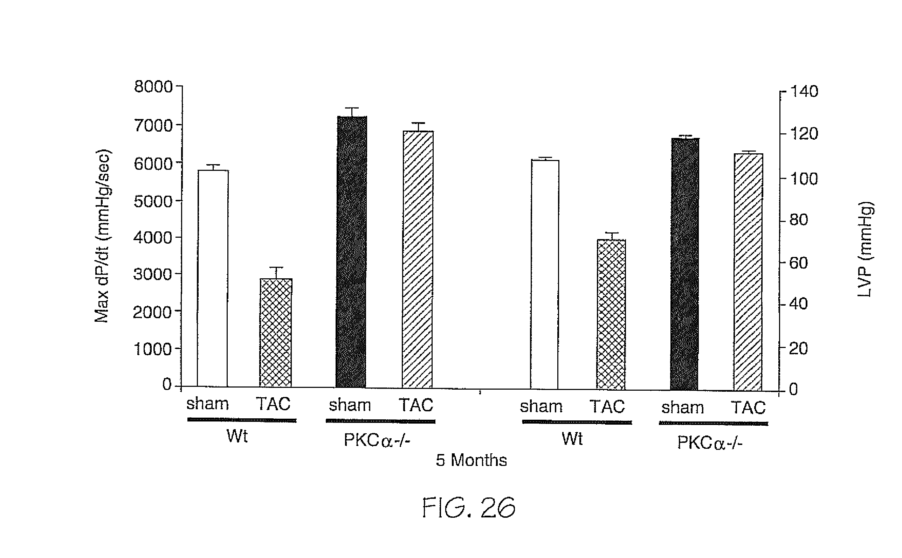

Figure 26 depicts the results of an assessment of heart function and in wildtype (Wt) and *PKCα-*/*-* mice twelve weeks after either a TAC procedure or sham operation. Results obtained from wildtype, sham-operated mice are indicated with empty bars; results obtained from *PKCα-*/*-*, sham-operated mice are indicated with solid bars, results obtained from wildtype, TAC mice are indicated with a cross-hatched bar, and results obtained from *PKCα-l-*, TAC mice are indicated with a striped bar. The left side of the graph presents results of *ex vivo* working heart preparations (Maximum dP/dt measured in mmHg/sec). The right side of the graph presents the left ventricular pressure (LVP) in mmHg.

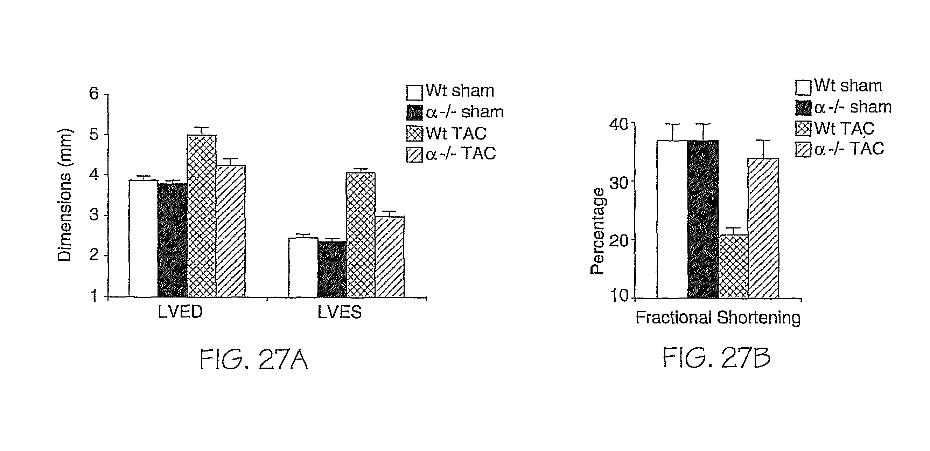

Figure 27 depicts the results of an assessment of heart function and hypertrophy in wildtype (Wt) and *PKCα-*/*-* mice twelve weeks after either a transverse aortic constriction (TAC) procedure or sham operation. Results obtained from wildtype, sham-operated mice are indicated with empty bars; results obtained from *PKCα-*/*-*, sham-operated mice are indicated with solid bars, results obtained from wildtype, TAC mice are indicated with a cross-hatched bar, and results obtained from *PKC*α*-*/*-*, TAC mice are indicated with a striped bar. Panel A presents the left ventricular end diastolic (LVED) and left ventricular end systolic (LVES) dimensions in mm. Panel B presents results of echocardiography analysis of fractional shortening (FS).

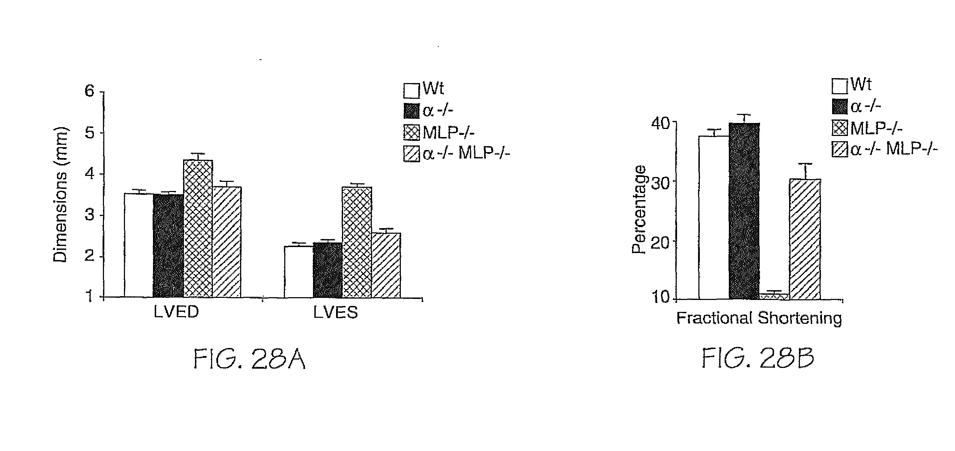

Figure 28 depicts the results of an assessment of heart function, hypertrophy, and gross heart morphology in wildtype (Wt), *MLP-l-,* and *MLP-l PKCα-l-* mice. Results obtained from wildtype mice are indicated with white bars; results obtained from *PKCα-*/*-* mice are indicated with solid bars, results obtained from *MLP-*/*-* mice are indicated with a hatched bar, and results obtained from *PKCα-*/*-, MLP-*/*-* mice are indicated with a striped bar. Panel A presents the left ventricular end diastolic (LVED) and left ventricular end systolic (LVES) dimensions in mm. Panel B presents results of echocardiography analysis of fractional shortening (FS).

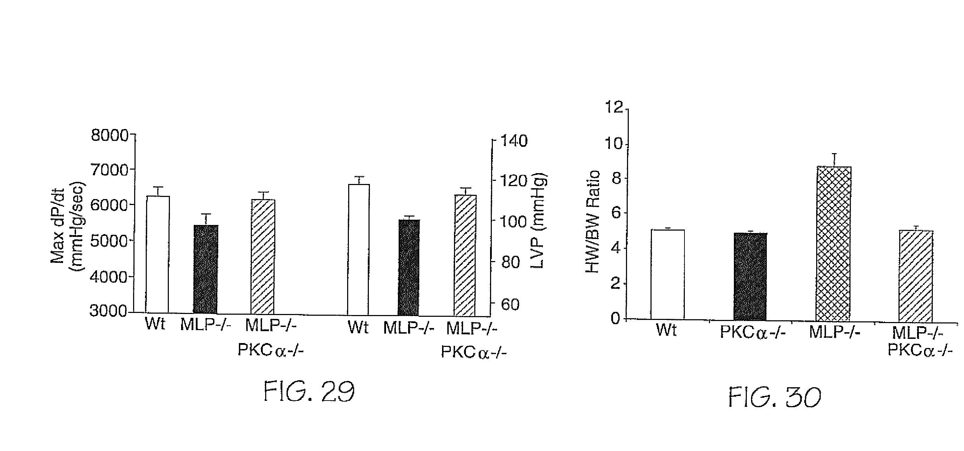

Figure 29 depicts the results of an assessment of heart function, hypertrophy, and gross heart morphology in wildtype (Wt), *MLP*-/-, and *MLP-*/*- PKCα-l* mice. Results obtained from wildtype mice are indicated with white bars; results obtained from *MLP-1-* mice are indicated with solid bars, and results obtained from *PKCα-*/*-, MLP-*/*-* mice are indicated with a striped bar. The left side of the graph presents results of ex vivo working heart preparations (Maximum dP/dt measured in mmHg/sec). The right side of the graph presents the left ventricular pressure (LVP) in mmHg.
Figure 30 presents heart weight (HW) to body weight (BW) ratios (N=4 for each group). Results obtained from wildtype mice are indicated with white bars; results obtained from *PKCα-*/- mice are indicated with solid bars, results obtained from *MLP-*/*-* mice are indicated with a hatched bar, and results obtained from *PKCα-l-, MLP-*/*-* mice are indicated with a striped bar.

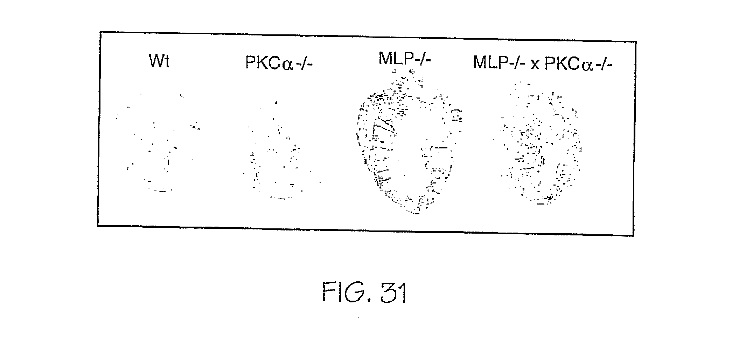

Figure 31 presents gross heart morphology assessed by Hematoxylin and Eosin staining of heart histological sections in wildtype (Wt), *PKCα-l-, MLP-*/*-,* and *MLP-*/*- PKCα-*/- mice.

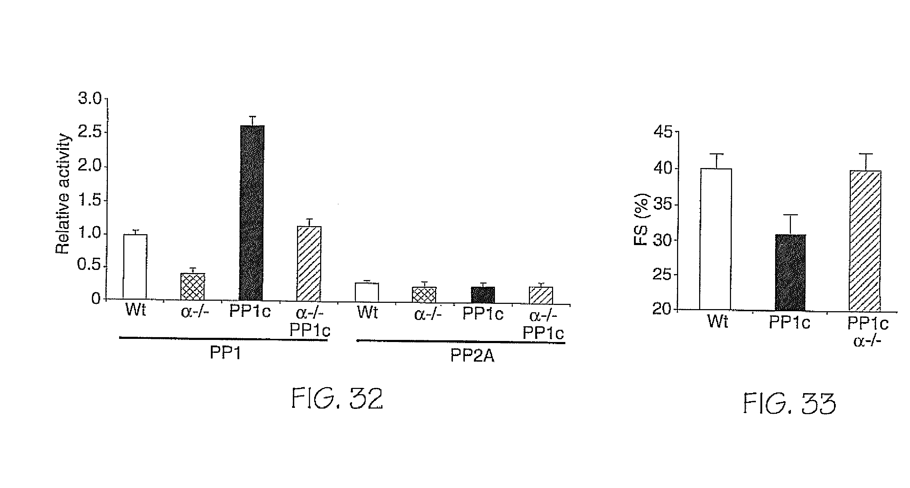

Figure 32 presents results of PP1- and PP2A-specific phosphatase assays from the hearts of adult wildtype (Wt, empty bar), *PKC*α-/- (α-/-, cross-hatch bar), PP1c transgenic (solid bar), and *PKC*α-/- x PP1c (striped bar) mice (N=4 mice in each group).
Figure 33 presents echocardiographic assessment of fractional shortening (FS) from the indicated groups of mice (N=4 each): Wildtype (Wt, empty bar); PP1c transgenic (PP1c, solid bar); and PKCα-/- X PP1c (PP1-c α-/-, striped bar).

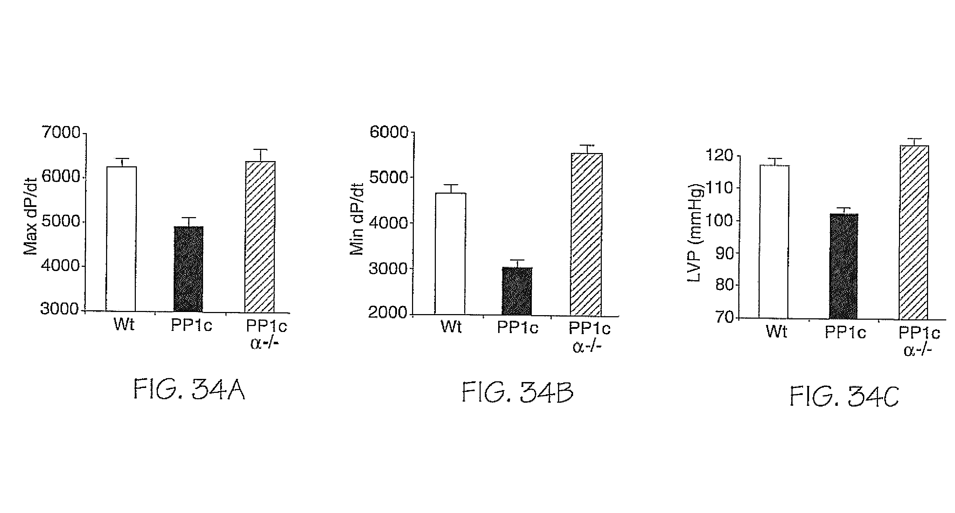

Figure 34 presents *ex vivo* working heart assessment of ventricular performance in wildtype (wt, white bar); PP1c (PP1c, black bar); and PKCα-/- X PP1c (PP1c α-/-, striped bar). Panel A presents the maximum dP/dt. Panel B presents the minimum dP/dt. Panel C presents the left ventricular pressure (LVP) in mmHg.

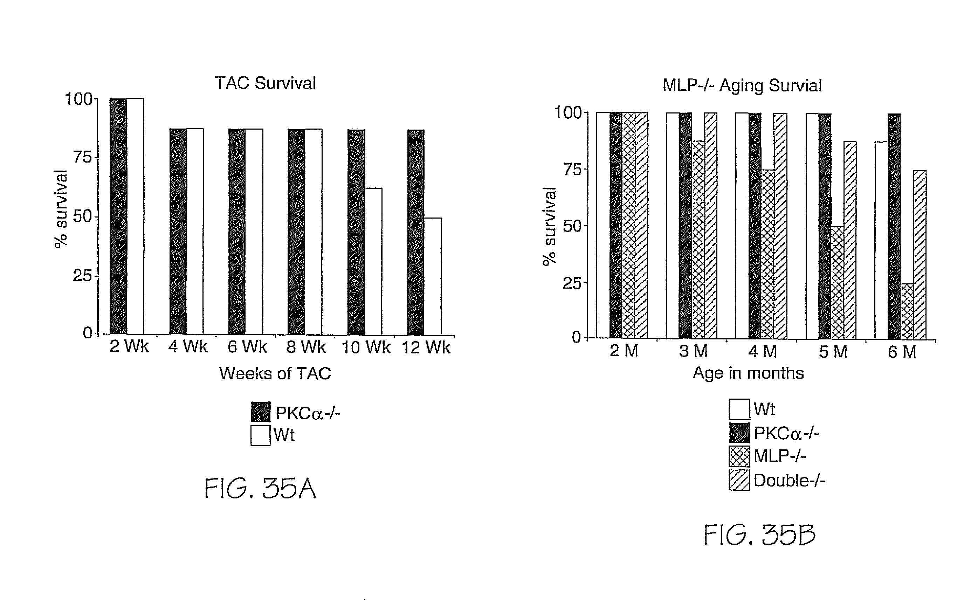

Figure 35 presents an analysis of mortality in two heart failure models. Panel A presents percent survival of wild-type (Wt, empty bars) and PKCα-/- (PKCα-/-, solid bars) at the indicated time points after a TAC operation. Panel B presents percent survival of wild-type (Wt, empty bars), PKCα-/- (PKCα-/-, solid bars), MLP-/- (MLP-/-, hatched bars), and PKCα-/-/MLP-/(Double, striped bars) mice at the indicated ages.

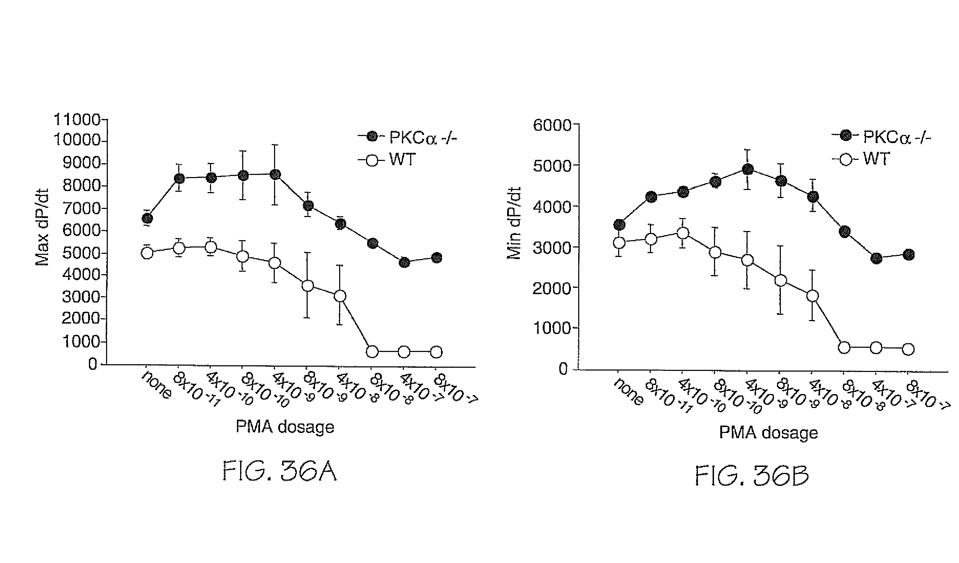

Figure 36 presents maximum (Panel A) and minimum (Panel B) dP/dt values obtained from isolated hearts infused with phorbol myristate acetate (PMA). Results obtained from wild-type hearts are indicated with empty circles; results obtained from PKCα-/- hearts are indicated with solid circles. Four hearts were analyzed in each group, and the error bars represent standard error of the mean. The PMA dosages are indicated.

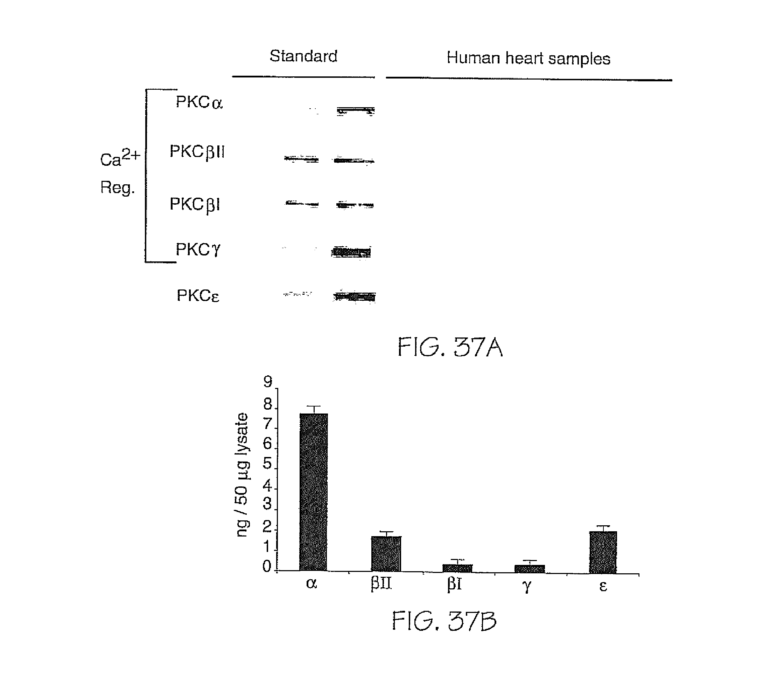

Figure 37 presents results of a Western blot analysis of the indicated PKC isoforms (PKCα, PKCβI, PKCβII, PKCγ, and PKCε) in the normal human heart. The Ca²⁺-regulated isozymes are bracketed. In Panel A the left three lanes contain recombinant protein standards generated in bacteria (standard). The right six lanes contain proteins from six normal human hearts (Human heart samples). Panel B presents a quantification of the amounts of each isozyme relative to the total protein content of the samples. The amount of each isozyme is indicated in ng/ 50 µg of total lysate. The PKC isoform of interest is indicated below each bar. The error bars represent the standard error of the mean.

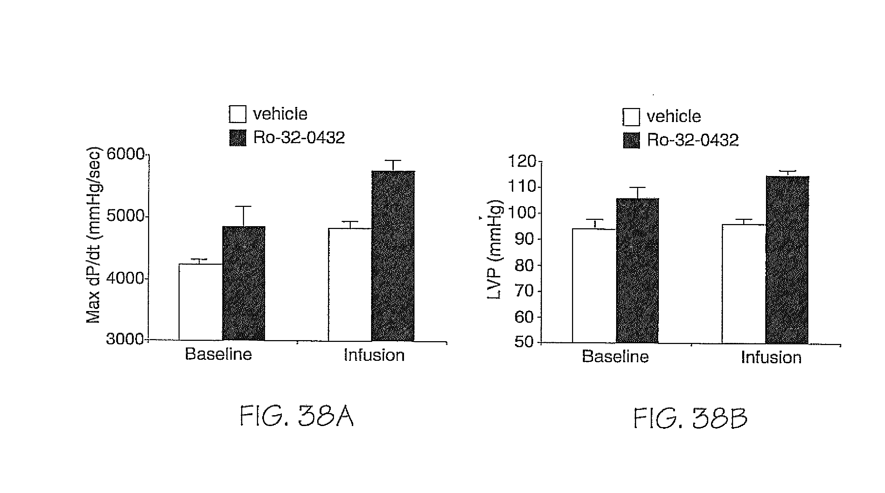

Figure 38 presents results obtained from an assessment of acute cardiac contractility in *ex vivo* working heart preparations. Results obtained from the control group of mice are indicated with empty bars; results obtained from the Ro-32-0432 treated mice are indicated with solid bars. Baseline results are indicated. The data obtained upon infusion of Ro-32-0432 or the vehicle control are indicated (Infusion). Values throughout the concentration time course (7 minutes per 10 different incremental concentrations) were summated for statistical purposes, representing an average dosage of approximately 1 X 10⁻⁸ M. Only the Ro-32-0432-infused group showed a statistically significant increase (p <0.05).

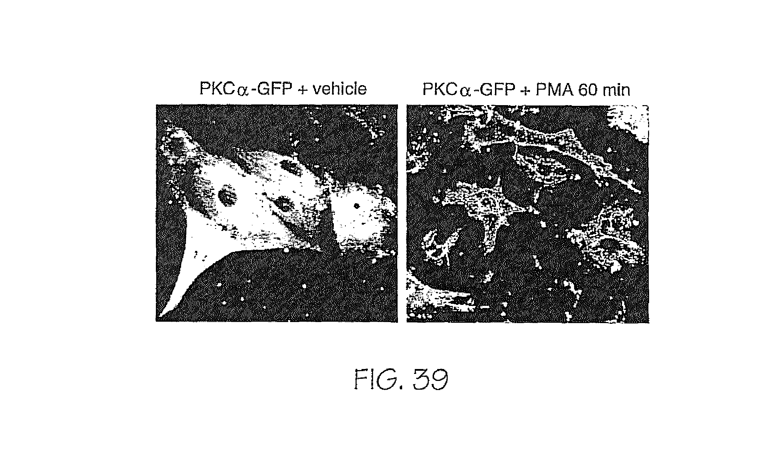

Figure 39 presents confocal micrographs of PKCα indicator polypeptide (PKCα-GFP) in cultured cells treated with DMSO (PKCα-GFP + vehicle) or with PMA (PKCα-GFP + PMA 60 minutes).

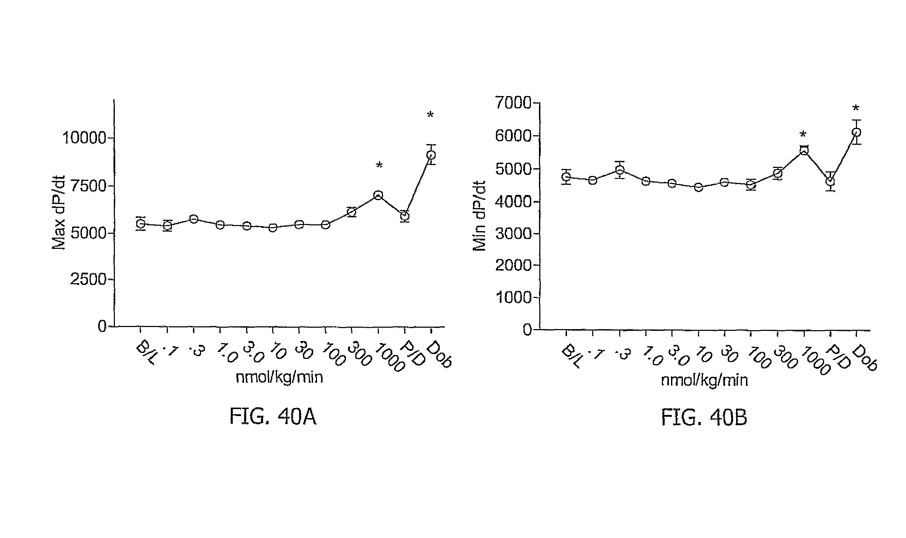

Figure 40 presents results obtained from an assessment of acute cardiac inotropic and lusitropic function after infusion of LY333531 at the indicated doses, represented by maximum dP/dt (Figure 40A) and minimum dP/dt (Figure 40B), respectively, *in vivo* in normal Sprague-Dawley and Lewis rats.

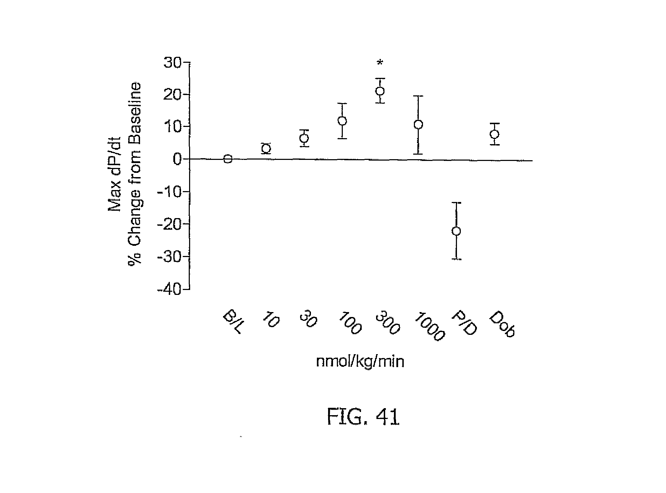

Figure 41 shows the percent increase in maximum dP/dt from baseline (B/L) following infusion of Ro-31-8220 in a rat model of myocardial infarction.

### DETAILED DESCRIPTION OF THE INVENTION

The present invention provides for modulation of cardiac contractility in acute heart failure, and cardiomyopathy in heart failure in general. Compositions of the invention include transgenic animals comprising either a PKCα nucleotide sequence or animals with a disruption in a PKCα nucleotide sequence. The invention further comprises cells and tissues isolated from these mice. The invention provides methods of modulating PKCα activity, PKCα expression levels, cardiac contractility, and susceptibility to cardiomyopathies, and acute heart failure. The invention provides kits for performing the methods of identifying PKCα modulating compounds.

The invention relates to compositions and methods drawn to PKCα gene (SEQ ID NO: 1). In an embodiment, an animal is stably transformed with an expression cassette comprising a cardiac-preferred regulatory sequences operably linked to a PKCα. nucleotide sequence. In an embodiment, an animal of the invention is stably transformed with an expression cassette comprising a cardiac-preferred regulatory sequences operably linked to a fragment or variant of the PKCα nucleotide sequence such as the dominant negative variant set forth in SEQ ID NO: 7. In another embodiment, an animal of the invention is stably transformed with an isolated nucleic acid molecule that disrupts the native PKCα nucleotide sequence such that PKCα expression levels are decreased. In one aspect, the cardiac-preferred regulatory sequences are cardiac-preferred promoter sequences.

In an embodiment, the genome of a germ-line cell of a transgenic animal comprises the nucleotide sequence of interest. A transgenic cell is a cell isolated from a transgenic animal of the invention comprising at least one expression cassette or disruption cassette. Transgenic tissue, e.g. cardiac tissue, is tissue comprising transgenic cells.

In embodiments involving disruption cassettes, the nucleotide sequence of interest may be flanked by nucleotide sequences that naturally occur in the genomic DNA of the cell into which the nucleic acid molecule is transformed.

Fragments and variants of the PKCα nucleotide sequence and protein encoded thereby are also encompassed by the present invention. By "fragment" is intended a portion of the nucleotide sequence or a portion of the amino acid sequence and hence protein encoded thereby. Fragments of a nucleotide sequence may encode protein fragments that retain the biological activity of the native protein and hence exhibit a PKCα activity. Alternatively, fragments of a nucleotide sequence are useful as hybridization probes. A biologically active portion of a PKCα can be prepared by isolating a portion of one of the PKCα nucleotide sequences of the invention, expressing the encoded portion of the PKCα protein (e.g., by recombinant expression *in vitro*), and assessing the activity of the encoded portion of the PKCα protein.

One of skill in the art would also recognize that PKCα genes and proteins from a species other than those listed in the sequence listing, particularly mammalian species, would be useful in the present invention. One of skill in the art would further recognize that by using probes from the known species' sequences, cDNA or genomic sequences homologous to the known sequence could be obtained from the same or alternate species by known cloning methods. Such PKCα homologs and orthologs are included in the definition of PKCα gene and proteins of the invention.

Thus, a fragment of a protein kinase C-α nucleotide sequence may encode a biologically active portion of a protein kinase C-α (PKCα) or it may be a fragment that can be used as a hybridization probe or PCR primer using methods disclosed below. A biologically active portion of a PKCα can be prepared by isolating a portion of one of the PKCα nucleotide sequences of the invention, expressing the encoded portion of the PKCα protein (e.g., by recombinant expression *in vitro),* and assessing the activity of the encoded portion of the PKCα protein. Nucleic acid molecules that are fragments of a protein kinase C-α nucleotide sequence comprise at least 16, 20, 50,75, 100, 150, 200, 250, 300, 350, 400, 450, 500, 550, 600, 650, 700, 800, 900, 1,000, 1,100, 1,200, 1,300, 1400, 1450, 1500, 1550, 1600, 1650, 1700, 1750, 1800, 1850, 1900, 1950, 2000, 2050, 2100, 2150, 2200, 2250, 2300, 2350, 2400, 2450, 2500, 2550, 2600, 2650, 2700, 2750, 2800, 2850, 2900, 2950, 3000, 3050, 3100, 3150, 3200, 3250, 3300, 3350, 3400, 3450, 3500,or 3524 nucleotides, or up to the number of nucleotides present in a full-length protein kinase C-α nucleotide sequence disclosed herein, or that contain additional sequences from the PKCα genomic locus alone or in combination with the sequence discussed above..

By "variants" are intended substantially similar sequences. For nucleotide sequences, conservative variants include those sequences that, because of the degeneracy of the genetic code, encode the amino acid sequence of one of the PKCα polypeptides of the invention. Naturally occurring allelic variants such as these can be identified with the use of known molecular biology techniques, as, for example, with polymerase chain reaction (PCR) and hybridization techniques that are known in the art. In order to isolate orthologs and other variants generally stringent hybridization conditions are utilized mainly dictated by specific sequence, sequence length, GC content and other parameters. Variant nucleotide sequences also include synthetically derived nucleotide sequences, such as those generated, for example, by using site-directed mutagenesis. Variants may also contain additional sequences from the genomic locus alone or in combination with other sequences.

Variant proteins may be derived from the native protein by deletion (so-called truncation) or addition af one or more amino acids; deletion or addition of one or more amino acids; or substitution of one or more amino acids at one or more sites in the native protein. Variant proteins encompassed by the present invention may or may not retain biological activity. Such variants may result from, for example, genetic polymorphism or from human manipulation. An exemplary variant PKCα protein is encoded by the nucleotide sequence set forth in SEQ ID NO: 7. The variant protein encoded by the nucleotide sequence set forth in SEQ ID NO: 7 exhibits dominant negative effects.

The proteins of the invention may be altered in various ways including amino acid substitutions, deletions, truncations, and insertions. For example, amino acid sequence variants of the PKCα proteins can be prepared by mutations in the DNA. Methods for mutagenesis and nucleotide sequence alterations are known in the art. See, for example, Kunkel (1985) Proc. Natl. Acad. Sci. USA 82:488-492; Kunkel et al. (1987) Methods in Enzymol. 154:367-382; U.S. Patent No. 4,873,192; Walker and Gaastra, eds. (1983) Techníques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.).

Variant nucleotide sequences and proteins also encompass sequences and proteins derived from a mutagenic and recombinogenic procedure such as DNA shuffling. With such a procedure, one or more different PKCα coding sequences can be manipulated to create a new PKCα possessing the desired properties. In this manner, libraries of recombinant polynucleotides are generated from a population of related sequence polynucleotides comprising sequence regions that have substantial sequence identity and can be homologously recombined *in vitro* or *in vivo*. For example, using this approach, sequence motifs encoding a domain of interest may be shuffled between the PKCα gene of the invention and other known PKCα genes to obtain a new gene coding for a protein with an altered property of interest e.g. a dominant negative mutation (Ohba et al. (1998) Mol. Cell. Biol. 18:51199-51207, Matsumoto et al. (2001) J. Biol. Chem. 276:14400-14406). Strategies for such DNA shuffling are known in the art.

The "percentage of sequence identity" or "sequence identity" is determined by comparing two optimally aligned sequences or subsequences over a comparison window or span, wherein the portion of the sequence in the comparison window may optionally comprise additions or deletions (i.e., gaps) as compared to the reference sequence (which does not comprise additions or deletions) for optimal alignment of the two sequences. The percentage is calculated by determining the number of positions at which the identical residue (*e*.*g*., nucleic acid base or amino acid residue) occurs in both sequences to yield the number of matched positions, dividing the number of matched positions by the total number of positions in the window of comparison and multiplying the result by 100 to yield the percentage of sequence identity.

Percentage sequence identity can be calculated by the local homology algorithm of Smith & Waterman, Adv. Appl. Math. 2:482-485 (1981); or by the homology alignment algorithm of Needleman & Wunsch, J. Mol. Biol. 48:443-445 (1970); either manually or by computerized implementations of these algorithms (GAP & BESTFIT in the GCG Wisconsin Software Package, Genetics Computer Group).

A preferred method for determining homology or sequence identity is by BLAST (Basic Local Alignment Search Tool) analysis using the algorithm employed by the programs blastp, blastn, blastx, tblastn and tbIastx (Karlin et al. (1990) Proc. Natl. Acad. Sci. USA 87, 2264-2268 and Altschul, (1993) J. Mol. Evol. 36, 290-300), which are tailored for sequence similarity searching. The approach used by the BLAST program is to first consider similar segments between a query sequence and a database sequence, then to evaluate the statistical significance of all matches that are identified and finally to summarize only those matches which satisfy a preselected threshold of significance. The search parameters for histogram, descriptions, alignments, expect (*i*.*e*., the statistical significance threshold for reporting matches against database sequences), cutoff, matrix and filter are generally set at the default-scoring matrix BLOSUM62 for blastp, blastx, tblastn, and tblastx (Henikoff et al. (1992) Proc. Natl, Acad. Sci. USA 89, 10915-10919).

As described herein, PKCα genes and proteins, their allelic and other variants (e.g. splice variants), their homologs and orthologs from other species and various fragments and mutants will exhibit sequence variations. Typically, these sequences may exhibit at least about 75% sequence identity, preferably at least about 80% sequence identity, more preferably at least about 90% sequence identity and more preferably at least about 95% sequence identity to the genes and proteins of the invention.

The PKCα sequences of the invention are provided in expression cassettes for expression in the animal of interest. The cassette will include 5' and 3' regulatory sequences operably linked to a PKCα sequence of the invention. By "operably linked" is intended the transcription and translation of the heterologous nucleotide sequence is under the influence of the regulatory sequences. In this manner, the nucleotide sequences for the PKCα nucleotide sequences of the invention may be provided in expression cassettes along with cardiac tissue-preferred promoters for expression in the animal of interest, more particularly in the heart of the animal.

Such an expression cassette is provided with at least one restriction site for insertion of the nucleotide sequence to be under the transcriptional regulation of the regulatory regions. The expression cassette may additionally contain selectable marker genes.

The expression cassette will include in the 5'-to-3' direction of transcription, a transcriptional and translational initiation region, and a heterologous nucleotide sequence of interest. In addition to containing sites for transcription initiation and control, expression cassettes can also contain sequences necessary for transcription termination and, in the transcribed region a ribosome-binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook et al. (1989) Molecular Cloning: A Laboratory Manual 2nd. ed., Cold Spring Harbor Laboratory Press, Cold Spring Harbor, N.Y.).

The expression cassette comprising the PKCα sequence of the present invention operably linked to a promoter nucleotide sequence may also contain at least one additional nucleotide sequence for a gene to be co-transformed into the organism. Alternatively, the additional sequence(s) can be provided on another expression cassette.

The regulatory sequences to which the polynucleotides described herein can be operably linked include promoters for directing mRNA transcription. These include, but are not limited to, the left promoter from bacteriophage λ, the lac, TRP, and TAC promoters from *E*. *coli,* the early and late promoters from SV40, the CMV immediate early promoter, the adenovirus early and late promoters, and retrovirus long-terminal repeats.

It is recognized that a PKCα nucleotide sequence of the invention can be operably linked to any cardiac tissue preferred promoter and expressed in cardiac tissue. By "cardiac tissue" is intended any tissue obtained from the heart, including but not limited to, tissues developmentally related to the heart such as the pulmonary myocardium.

It is recognized that to increase transcription levels or to alter tissue specificity, enhancers and/or tissue-preference elements may be utilized in combination with the promoter. For example, quantitative or tissue specificity upstream elements from other cardiac-preferred promoters may be combined with the α-MHC promoter region used to generate the PKCα overexpressing mice to augment cardiac-preferred transcription. Such elements have been characterized, for example, the murine TIMP-4 promoter, A and B-type natriuretic peptide promoters, human cardiac troponin I promoter, mouse S100A1 promoter, salmon cardiac peptide promoter, GATA response element, inducible cardiac preferred promoters, rabbit β-myosin promoter, and mouse α-myosin heavy chain promoter (Rahkonen, et al. (2002) Biochim Biophys Acta 1577:45-52; Thuerauf and Glembotski (1997) J. Biol. Chem. 272:7464-7472; LaPointe et al (1996) Hypertension 27:715-722; Grepin et al. (1994) Mol. Cell Biol. 14:3115-29; Dellow, et al. (2001) Cardiovasc. Res.50:3-6; Kiewitz, et al. (2000) Biochim Biophys Acta 1498:207-19; Majalahti-Palviainan, et al (2000) Endocrinology 141:731-740; Charron et al. (1999) Molecular & Cellular Biology 19:4355-4365; Genbank U71441; U.S. Provisional Patent Application No:60/393,525 and 60/454,947; and U.S. Patent Application No. 10/613,728).

A variety of cardiac tissue preferred promoter elements have been described in the literature and can be used in the present invention. These include, but are not limited to, tissue preferred elements from the following genes: myosin light chain-2, α-myosin heavy chain, AE3, cardiac troponin C, and cardiac α-actin. See, e.g. Franz et al (1997) Cardiovasc. Res. 35:560-566; Robbins et al. (1995) Ann. N.Y. Acad. Sci. 752:492-505; Linn et al, (1995) Circ. Res. 76:584-591; Parmacek et al, (1994) Mol Cell Biol. 14:1870-1885; Hunter et al. (1993) Hypertension 22:608-617; and Sartorelli et al. (1992) Proc. Natl. Acad. Sci. USA 89:4047-4051.

In other embodiments, the coding region is operably linked to an inducible regulatory element or elements. A variety of inducible promoter systems has been described in the literature and can be used in the present invention. A known and useful conditional system is the binary, tetracycline-based system, which has been used in both cells and animals to reversibly induce expression by the addition or removal of tetracycline or its analogues. Another example of such a binary system is the cre/loxP recombinase system of bacteriophage P1, For a description af the cre/loxP recombinase system, see, e.g., Lakso et al. (1992) PNAS 89:6232-6236.

Another class of promoter elements are those which activate transcription of an operably linked nucleotide sequence of interest in response to hypoxic conditions. These include promoter elements regulated at least in part by hypoxia inducible factor-1. Hypoxia response elements include, but are not limited to, the erythropoietin hypoxia response enhancer element (HREE1), the muscle pyruvate kinase HRE; the β-enolase HRE; and endothelin-1 HRE element, and chimeric nucleotide sequence comprising these sequences. See Bunn and Poynton (1996) Physiol. Rev. 76:839-885; Dachs and Stratford (1996) Br. J. Cancer 74:S126-S132; Guillemon and Krasnow (1997) Cell 89:9-12; Firth et al. (1994) Proc, Natl. Acad. Sci. 91:6496-6500; Jiang et al. (1997) Cancer Res. 57:5328-5335; U.S. Patent No. 5,834,306).

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

Where appropriate, the PKCα nucleotide sequence of the present invention and any additional nucleotide sequence(s) may be optimized for increased expression in the transformed animal. That is, these nucleotide sequences can be synthesized using species preferred codons for improved expression, such as mouse-preferred codons for improved expression in mice. Methods are available in the art for synthesizing species-preferred nucleotide sequences, See, for example, Wada et al. (1992) Nucleic Acids Res, 20 (Suppl.), 2111-2118; Butkus et al. (1998) Clin Exp Pharmacol Physiol Suppl. 25:S28-33; and Sambrook et al. (1989) Molecular Cloning: A Laboratory Manual 2nd. ed., Cold Spring Harbor Laboratory Press, Cold Spring Harbor, N.Y.

Additional sequence modifications are known to enhance gene expression in a cellular host. These include elimination of sequences encoding spurious polyadenylation signals, exon-intron splice site signals, transposon-like repeats, and other well-characterized sequences that may be deleterious to gene expression. The G-C content of the heterologous nucleotide sequence may be adjusted to levels average for a given cellular host, as calculated by reference to known genes expressed in the host cell. When possible, the sequence is modified to avoid predicted hairpin secondary mRNA structures.

In those instances where it is desirable to have the expressed product of the heterologous PKCα nucleotide sequence directed to a particular organelle, particularly the mitochondria, the nucleus, the endoplasmic reticulum, or the Golgi apparatus; or secreted at the cell's surface or extracellularly; the expression cassette may further comprise a coding sequence for a transit peptide. Such transit peptides are known in the art and include, but are not limited to, the transit peptide for the acyl carrier protein, the small subunit of RUBISCO, and the like.

Disruption cassettes are used to interrupt and/or remove a sequence of interest from the genome of an animal cell in order to generate a "knock-out," "deletion," or "null" mutant. By "targeting vector" and "disruption cassette" is intended an isolated nucleic acid molecule comprising a 5' flanking region, a disruption region, and a 3' flanking region. Disruption cassettes and methods of their use are known in the art. See Doetschman et al. (1987) Nature 330:576-578; Doetschman et al. (1988) Proc. Natl, Acad. Sci 85:8583-87; Schwartz et al (1991) Proc. Natl. Acad. Sci.88:10416-20*;* Oliver et al (1997) Proc. Natl, Acad. Sci. 94:14730-14735; Nagy et al Ed. (2003) Manipulating the Mouse Embryo Cold Spring Harbor Press, Cold Spring Harbor, NY.

Reporter genes or selectable marker genes may be included in the expression cassettes. Examples of suitable reporter genes known in the art can be found in, for example, Ausubel et al. (2002) Current Protocols in Molecular Biology. John Wiley & Sons, New York, NY. Selectable marker genes for selection of transformed cells or tissues can include genes that confer antibiotic resistance. Other genes that could serve utility in the recovery of transgenic events but might not be required in the final product would include, but are not limited to, examples such as GUS (β-glucuronidase), fluorescence proteins (e.g. GFP), CAT; and luciferase.

Delivery vehicles suitable for incorporation of a polynucleotide for introduction into a host cell include, but are not limited to, viral vectors and non-viral vectors (Verma and Somia (1997) Nature 389:239-242).

A variety of non-viral vehicles for delivery of a polynucleotide are known in the art and are encompassed in the present invention. An isolated nucleic acid molecule can be delivered to a cell as naked DNA (WO 97/40163). Alternatively, a polynucleotide can be delivered to a cell associated in a variety of ways with a variety of substances (forms of delivery) including, but not limited to, cationic lipids; biocompatible polymers, including natural and synthetic polymers; lipoproteins; polypeptides; polysaccharides; lipopolysaccharides; artificial viral envelopes; metal particles; protein transduction domains, and bacteria. A delivery vehicle can be a microparticle. Mixtures or conjugates of these various substances can also be used as delivery vehicles. A polynucleotide can be associated non-covalently or covalently with these forms of delivery. Liposomes can be targeted to a particular cell type, e.g., to a cardiomyocyte.

Viral vectors include, but are not limited to, DNA viral vectors such as those based on adenoviruses, herpes simplex virus, poxvirus such as vaccinia virus, and parvoviruses, including adeno-associated virus; and RNA viral vectors, including but not limited to, the retroviral vectors. Retroviral vectors include murine leukemia virus, and lentiviruses such as human immunodeficiency virus. See Naldini et al. (1996) Science 272:263-267.

Non-viral delivery vehicles comprising a polynucleotide can be introduced into host cells and/or target cells by any suitable method known in the art, such as transfection by the calcium phosphate coprecipitation technique; electroporation; electropermeablization; liposome-mediated transfection; ballistic transfection; biolistic processes including microparticle bombardment, jet injection, and needle and syringe injection, or by microinjection. Numerous methods of transfection are known to the skilled artisan.

Viral delivery vectors can be introduced into cells by infection. Alternatively, viral vectors can be incorporated into any of the non-viral delivery vectors described above for delivery into cells. For example, viral vectors can be mixed with cationic lipids (Hodgson and Solaiman (1996) Nature Biotechnol. 14:339-342); or lamellar liposomes (Wilson et al. (1977) Proc, Natl. Acad. Sci. 74:3471-3475; and Faller et al, (1984) J Virol. 49:269-272)*.*

For *in vivo* delivery, the vector can be introduced into an individual or organism by any method known to the skilled artisan.

Any of the regulatory or other sequences useful in expression vectors can form part of the transgenic sequence. This includes intronic sequences and polyadenylation signals, if not already included. In one embodiment, the animal cell can be a fertilized oocyte or embryonic stem cell that can be used to produce a transgenic animal comprising at least one stably transformed expression cassette comprising the nucleotide sequence of interest. Alternatively, the host cell can be a stem cell or other early tissue precursor that gives rise to a specific subset of cells and can be used to produce transgenic tissues in an animal. See also Thomas et al., (1987) Cell 51:503 for a description of homologous recombination vectors. The vector is introduced into an embryonic stem cell line (e.g., by electroporation) and cells in which the introduced gene has recombined with the genome are selected (see e.g,, Li, E. et al. (1992) Cell 69:915). The selected cells are then injected into a blastocyst of an animal (e.g., a mouse) to form aggregation chimeras (see e.g., Bradley, A. in Teratocarcinomas and Embryonic Stem Cells: A Practical Approach, E. J. Robertson, ed. (IRL, Oxford, 1987) pp. 113-152). A chimeric embryo can then be implanted into a suitable pseudopregnant female foster animal and the embryo brought to term. Progeny harboring the recombined DNA in their germ cells can be used to breed animals in which all cells of the animal contain the recombined DNA by germ line transmission of the transgene. Methods for constructing homologous recombination vectors and homologous recombinant animals are described further in Bradley, A. (1991) Current Opinion in Biotechnology 2:823-829 and in PCT International Publication Nos. WO 90/11354; WO 91/01140; and WO 93/04169.

Methods for generating transgenic animals via embryo manipulation and microinjection, particularly animals such as mice, have become conventional in the art and are described, for example, in U.S. Pat. Nos. 4,736,866; 4,870,009; 4,873,191; 6,201,165 and in Nagy et al Ed. (2003) Manipulating the Mouse Embryo Cold Spring Harbor Press, Cold Spring Harbor, NY).

Clones of the non-human transgenic animals described herein can also be produced according to the methods described in Wilmut et al, (1997) Nature 385:810-813 and PCT International Publication Nos. WO 97/07668 and WO 97/07669. In brief, a cell, e.g., a somatic cell, from the transgenic animal can be isolated and induced to exit the growth cycle and enter Gₒ phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyst and then transferred to a pseudopregnant female foster animal. The offspring barn of this female foster animal will be a clone of the animal from which the cell, e.g., the somatic cell, is isolated.

Other examples of transgenic animals include non-human primates, sheep, dogs, pigs, guinea pigs, hamsters, cows, goats, rabbits, and rats. Methods for providing transgenic rabbits are described in Marian et al. (1999) J. Clin. Invest. 104:1683-1692 and James et al. (2000) Circulation 101:1715-1721.

By "PKCα activity" is intended any activity exhibited by the wild-type PKCα described herein. Such activities include, but are not limited to, kinase activity, receptor of activated C kinase (RACK) binding activity, expression, translocation from the cytosolic fraction to the particulate fraction, and translocation to the sarcolemma. Modulation of PKCα activity includes but is not limited to modulation of a PKCα activity such as kinase activity, RACK binding, or modulation of PKCα expression levels or cellular distribution.

Methods of assaying kinase activity are known in the art and include, but are not limited to, immunoprecipitation with antibodies to phosphor-peptides; fluorescence polarization; filter binding assays with radioisotopes, scintillation proximity assays, 96 well assays with conjugated antibodies; time resolved fluorescent assays, thin layer chromatography; immunoprecipitation and immune complex assays; non-trichloroacetic acid phosphoamino acid determinations; and protein kinase assays. See Braz et al, (2002) J. Cell Biol. 156:905-919; Ping et al. (1999) Am. J. Physiol. 276:H1468-H1481; U.S. Patent Application No;20030036106; U.S. Patent No:5447860; Walker, John, ed. (2002) Protein Protocols on CD-ROM v. 2; and Ausubel et al., eds. (1995) Current Protocols in Molecular Biology, (Greene Publishing and Wiley-Interscience, New York).

Methods of analyzing PKCα association with RACKs are known in the art and include, but are not limited to, ELISA, protein interactive trapping, X-ray crystallography, NMR, ultracentrifugation, immunoprecipitation, co-immunoprecipitation, cross-linking, yeast two-hybrid assays, and affinity chromatography. See for example Mochly-Rosen (1995) Biochem Soc. Trans. 23(3):596-600; Walker, John, ed. (2002) Protein Protocols on CD-ROM v. 2; and Ausubel et al., eds. (1995) Current Protocols in Molecular Biology, (Greene Publishing and Wiley-Interscience, New York).

The invention provides methods of monitoring translocation of a PKCα indicator polypeptide. By "indicator polypeptide" is intended any polypeptide suitable for monitoring subcellular location. Suitable indicator polypeptides include fusion polypeptides containing reporter genes described earlier, such as, but not limited to, fluorescent proteins (e.g. GFP), β-galactosidase, c-jun, c-myc; affinity polypeptide tags (such as His tags), radiolabeled polypeptides, biotin labeled polypeptides, antigen labeled polypeptides, and dye labeled polypeptides. Suitable indicator polypeptides include antibodies specific to the polypeptide of interest.

In an embodiment, the invention provides a method of altering PKCα expression in an animal. In an embodiment, PKCα expression is modulated throughout the animal (e.g. the disruption mutant). In an embodiment PKCα expression is modulated in a cardiac preferred manner. By "cardiac-preferred" is intended that expression of the heterologous PKCα is most abundant in cardiac tissue, while some expression may occur in other tissue types, particularly in tissues developmentally related to cardiac tissue.

Methods of determining expression levels are known in the art and include, but are not limited to, qualitative Western blot analysis, immunoprecipitation, radiological assays, polypeptide purification, spectrophotometric analysis, Coomassie staining of acrylamide gels, ELISAs, RT-PCR, 2-D gel electrophoresis, microarray analysis, *in situ* hybridization, chemiluminescence, silver staining, enzymatic assays, ponceau S staining, multiplex RT-PCR, immunohistochemical assays, radioimmunoassay, colorimetric analysis, immunoradiometric assays, positron emission tomography, Northern blotting, fluorometric assays and SAGE. See, for example, Ausubel et al, eds. (2002) Current Protocols in Molecular Biology, Wiley-Interscience, New York, New York; Coligan et al (2002) Current Protocols in Protein Science, Wiley-Interscience, New York, New York; and Sun et al. (2001) Gene Ther. 8; 1572-1579.

It is recognized that the PKCα nucleotide sequences may be used with their native promoters to increase or decrease expression resulting in a change in phenotype in the cardiac tissue of the transformed animal.

Transgenic animals that exhibit altered cardiac preferred expression of PKCα are useful to conduct assays that identify compounds that affect cardiac function such as, but not limited to, cardiac contractility. Assays to determine cardiac contractility are known in the art and include, but are not limited to, shortening assays, peak shortening, time to peak, time to ½ maximal relaxation, contracting and relaxing rate assays, changes in cardiac chronotropy, changes in cardiac lusitropy, and gross heart contraction assays. The altered cardiac-preferred expression of the PKCα expression may result in altered susceptibility to a cardiomyopathy. In an aspect of the invention, the invention provides methods of acutely modulating cardiac contractility. In another aspect of the invention, the invention provides methods of acutely modulating a cardiomyopathy. An acute modulation or alteration begins within 1 second; 10 seconds; 30 seconds; 1, 2, 3, 4, 5, 6, 7, 8, 9, 10, 11, 12, 13, 14, 15, 20, 25, 30, 35, 40, 45, 50, 55, 60 minutes; 2, 3, 4, 5, 6, 7, 8, 9, 10, 11, 12, 13, 14, 15, 16, 17, 18, 19, 20, 21, 22, 23, 24 hours; 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days after administration of the PKCα modulating agent. The duration of the modulation ranges from short durations such as, but not limited to, nanosecond, second, and minute increments; intermediate durations such as, but not limited to, hour, day, and week increments; to long durations such as, but not limited to, month and year increments, up to and including the recipient's lifespan.

In the context of the present invention, "cardiac contractility " or "myocardial contractility" are defined as measures of cardiac function, which may include but are not limited to cardiac output, ejection fraction, fractional shortening, cardiac work, cardiac index, chronotropy, lusitropy, velocity of circumferential fiber shortening, velocity of circumferential fiber shortening corrected for heart rate, stroke volume, rates of cardiac contraction or relaxation, the first derivatives of interventricular pressure (maximum dP/dt and minimum dP/dt), ventricular volumes, clinical evaluations of cardiac function (for example, stress echocardiography and treadmill walking) and variations or normalizations of these parameters. These parameters may be measured in humans or animals alike to assess myocardial function and assist in diagnosis and prognosis of heart disease.

A "cardiomyopathy" is any disorder or condition involving cardiac muscle tissue or cardiac dysfunction. Disorders involving cardiac muscle tissue include, but are not limited to, myocardial disease, including but not limited to dilated cardiomyopathy, hypertrophic cardiomyopathy, restrictive cardiomyopathy, myocardial stunning, and myocarditis; heart failure; acute heart failure; rheumatic fever; rhabdomyoma; sarcoma; congenital heart disease, including but not limited to, left-to-right shunts-late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia; disorders involving cardiac transplantation; arterial hypertension; peripartum cardiomyopathy; alcoholic cardiomyopathy; tachycardias; supraventricular tachycardia; bradycardia; atrial flutter; hydrops fetalis; arrhythmias; extrasystalic arrhythmia; fetal cardiac arrhythmia; endocarditis; atrial fibrillation; idiopathic dilated cardiomyopathy; Chagas' heart disease; long QT syndrome; Brugada syndrome; ischemia; hypoxia; ventricular fibrillation; ventricular tachycardia; restenosis; congestive heart failure; syncope; arrythmias; pericardial disease; myocardial infarction; unstable angina; stable angina; and angina pectoris, viral myocarditis, and non-proliferating cell disorders involving cardiac muscle tissue.

By "altered susceptibility" is intended that a transgenic animal of the invention differs from a non-transgenic animal in the extent to which the transgenic animal of the invention exhibits a cardiomyopathic phenotype. The cardiomyopathic phenotype may present during any stage of development including, but not limited to, embryonically, post-natally, in the adult, and as the animal nears end of lifespan. In an embodiment, the cardiomyopathic phenotype may be induced by external stimuli such as, but not limited to, diet, exercise, chemical treatment, or surgical procedure.

Cardiomyopathic phenotypes include, but are not limited to, hypertrophy; morphology, such as interventricular septal hypertrophy; left ventricular-end systolic maximum dP/dt or end-diastolic dimension(r); papillary muscle dimension; left-ventricular outflow tract obstruction; midventricular hypertrophy; apical hypertrophy; asymmetrical hypertrophy; concentric enlarged ventricular mass; eccentric enlarged ventricular mass; sarcomere structure; myofibril function; receptor expression; heart rate; ventricular systolic pressure; ventricular diastolic pressure; aortic systolic pressure; aortic diastolic pressure; contractility; interstitial fibrosis; cardiomyocyte disarray; Ca²⁺ sensitivity; Ca¹⁺ release; Ca²⁺ uptake; catecholine sensitivity; α-adrenergic sensitivity; beta-adrenergic sensitivity; dobutamine sensitivity; thyroxine sensitivity; angiotensin-converting enzyme inhibitor sensitivity; amiodarone sensitivity; lidocaine sensitivity; glycoprotein receptor antagonist sensitivity; anabolic steroid sensitivity; carnitine transport irregularities; left ventricular dilation, reduced laft ventricular ejection fraction; left atrial dilatation; diuretic sensitivity; volemia; ischemia; leukocyte flow properties; the polymorphonuclear leukocyte (PMN) membrane fluidity; PMN cytosolic Ca²⁺ content; high interventricular septal defects, rosette inhibition effect; contractile force transmission; myocardial fiber disarray; increased chamber stiffness; impaired relaxation; small-vessel disease; dyspnea; angina; presyncope; tachycardia; syncope; lethargy; respiratory distress; ruffled fur; hunched posture; peripheral edema; ascites; hepatomegaly; edematous lung; cardiomegaly; organized thrombi formation; heart weight/body weight ratio; rate of pressure development, rate of pressure fail, cell twitch measurement and the like. See, for example, Braunwald et al. (2002) Circulation 106:1312-1316; Wigle et al. (1995) Circulation 92:1680-1692; and Pi & Walker (2000) Am. J. Physiol. Heart Circ. Physiol 279:H26-H34; hereby incorporated by reference in their entirety.

Methods for measuring cardiomyopathic phenotypes are known in the art and include, but are not limited to, trans-thoracic echocardiography, transesophageal echocardiography, exercise tests, urine/catecholamine analysis, BIAs, light microscopy, heart catheterization, dynamic electrocardiography, Langendorff hanging heart preparation, working heart preparation, MRI, multiplex RT-PCIZ, positron emission tomography, angiography, magnetic resonance spin echo, short-axis MRI scanning, Doppler velocity recordings, Doppler color flow imaging, stress thallium studies, cardiac ultrasound, chest X-ray, oxygen consumption test, electrophysiological studies, auscultation, scanning EM, gravimetric analysis, hematoxylin and eosin staining, skinned fiber analysis, transmission electron microscopy, immunofluorescent analysis, trichrome staining, Masson's trichrome staining, Van Kossa staining, 2-D echocardiography, cardiotocography, baseline M-mode echocardiography, and myocardial lactate production assays. See, for example, Braz et al. (2002) J. Cell. Biol. 156:905-919; Braunwald et al. (2002) Circulation 106:1312-1316; Sohal et al. (2001) Circulation Res. 89:20-25; Nagueh et al. (2000) Circulation 102:1346-1350; Sanbe et al. (2001) J. Biol. Chem. 276:32682-32686; Sanbe et al. (1999) J. Biol. Chem. 274:21085-21094; Wigle et al. (1995) Circulation 92:1680-1692; Pi & Walker (2000) Am. J. Physiol. Heart Circ. Physiol 279:H26-H34; and Wang et al. (2001) Am. J. Physiol, Heart Circ. Physiol. 269:H90-H98 hereby incorporated by reference in their entirety.

The term "treatment" is used herein to mean that, at a minimum, administration of a compound of the present invention mitigates a disease or a disorder in a host, preferably in a mammalian subject, more preferably in humans. Thus, the term "treatment" includes: preventing an infectious disorder from occurring in a host, particularly when the host is predisposed to acquiring the disease, but has not yet been diagnosed with the disease; inhibiting the infectious disorder; and/or alleviating or reversing the infectious disorder. Insofar as the methods of the present invention are directed to preventing disorders, it is understood that the term "prevent" does not require that the disease state be completely thwarted. (See Webster's Ninth Collegiate Dictionary.) Rather, as used herein, the term preventing refers to the ability of the skilled artisan to identify a population that is susceptible to disorders, such that administration of the compounds of the present invention may occur prior to onset of a disease. The term does not imply that the disease state be completely avoided.
Identification of PKCα inhibitors for the treatment of impaired cardiac contraction and relaxation can be identified by known methodologies. PKCα inhibitors could be identified by assessing the enzymatic activity of PKCα. This may be accomplished by using a number of commercially available kits. Some of these kits use "labeled" substrates, including, but not limited to luminescent, fluorescent, radioactive or other measurable and quantifiable endpoints. Alternatively, as set forth in this invention, the PKCα protein itself could be attached to a traceable marker, including, but not limited to luminescent, fluorescent or radioactive ion or molecule in order to determine the distribution and activity of PKCα in isolation or in a cell or tissue. As PKCα has many known substrates in the cell, PKCα activity could be assessed by measuring the phosphorylation or dephosphorylation of PKCα substrates. PKCα substrates phosphorylation/dephosphorylation status may be measured using labeled or unlabeled phosphorylation site-specific antibodies, luminescent, fluorescent, radioactive biological labels or other means to assess PKCα activity against its substrates. Alternatively, the redistribution of the substrate(s) may also serve as a means of measuring its response to alterations in PKCα activity. In the case where the substrate is a kinase, phosphatase or other enzyme, the activity of the substrate may be measured by established techniques.

Identification of PKCα inhibitors that would be beneficial in humans with cardiac dysfunction may be accomplished using isolated cells or isolated tissues in which it has been determined that PKCα is present. For instance, PKCα inhibitors may be tested in isolated cells, preferably cardiomyocytes, from mammals or other organisms and determine the effect of PKCα inhibitors by measuring the percent shortening of the cell (%FS): the rates of shortening or relengthening (±dL/dt), by standard techniques (Chaudhri B et al. (2002) Am J Physiol Heart Circ Physiol. 283:H2450-H2457). Alternatively, muscle(s), preferably of cardiac origin, may be isolated and measurements of contractile function assess in the presence and absence of PKCα inhibitors, by standard techniques (Slack JP et al, (1997) J Biol Chem. 272:18862-18868). PKCα inhibitors may be identified as outlined in the present invention by measuring acute hemodynamics, including heart rate, blood pressure, rates of contraction and relaxation (+dP/dt, and -dP/dt), left ventricular pressure and derivations of these parameters. PKCα inhibitors may be identified by these methods in suitable, normal animals including, but not limited to, various genetic strains of mice, rats, guinea pigs, hamsters, humans, rabbits, dogs, pigs, goats, cows, monkeys, chimpanzees, sheep, hamsters and zebrafish. PKCα inhibitors could be identified by these methods in suitable, animal models of heart failure or cardiac dysfunction including, but not limited to, various genetic strains of transgenic or knockout mice, such as the MLP^{(-/-)} KO mice, type-1 serine/threonine phosphatase overexpressing mice (PPIc), and PKCα overexpressing transgenic mice. In addition, PKCα inhibitors may be identified in spontaneous or natural models of heart failure and cardiac dysfunction due to a genetic or multiple genetic defects, including but not limited to the spontaneous hypertensive heart failure rat or the Dahl salt sensitive rat. In addition, PKCα inhibitors may be identified in surgically induced models of cardiac dysfunction including, but not limited to, myocardial infarction models, coronary microembolism model, aortic constriction model, arteriovenous fistula model or other pressure or volume overload models in rats, guinea pigs, rabbits, dogs, pigs, goats, cows, monkeys, chimpanzees, sheep, hamsters and zebrafish.

In an embodiment, a transgenic animal, tissue, or cell of the invention may be used to identify PKCα modulating compounds. A "PKCα modulating compound" is a compound that modulates a PKCα activity. PKCα modulating compounds include, but are not limited to, diacylglycerols, phosphatidylserine, Ca++; PMA, CGP54345, bisindolylmaleimide, AAP10, staurosporine, H-7 (Sigma Co.), diazoxide, DiC₈, arachidonic acid, Gö-6976 (PKC and including PKCα), CGP 54345, HBDDE (also PKCγ), and Ro-32-0432 (also PKCβ). Methods for assaying PKCα activity are described elsewhere herein. Any method of assaying a PKCα activity known in the art may be used to monitor the effects of the compound of interest on a transgenic animal of the invention.

PKCα inhibitors include, but are not limited to, kinase inhibitors, protein kinase C inhibitors, and PKCα specific inhibitors. By "kinase inhibitor" is intended a compound that inhibits multiple kinases including PKCα. By "protein kinase C inhibitor" is intended a compound preferentially inhibits activities of a protein kinase C as compared to its effect on other kinases. By "PKCα specific inhibitor" is intended a compound that reduces a PKCα activity more than it reduces an activity of another kinase, including other protein kinase C isozymes. Known PKCα inhibitors include, but are not limited to, nucleic acid molecules having antisense nucleotide sequences and antisense molecules commercially available from Isis Pharmaceuticals and dominant negative mutations of PKCα, such as the lysine 368 arginine mutation (Braz et al. (2002) J. Cell. Biol. 156:905-919).

Antisense constructions complementary to at least a portion of the messenger RNA (mRNA) for a PKCα nucleotide sequence can be constructed. Antisense nucleotides are constructed to hybridize with the corresponding mRNA. Modifications of the antisense sequences may be made as long as the sequences hybridize to and interfere with expression of the corresponding mRNA. In this manner, antisense constructions having at least about 70%, preferably at least about 80%, more preferably at least about 85% sequence identity to the corresponding antisensed sequences may be used. Furthermore, portions of the antisense nucleotides may be used to disrupt the expression of the target gene. Generally, sequences af at least 50 nucleotides, 100 nucleotides, 200 nucleotides, or greater may be used. Thus, antisense DNA sequences may be operably linked to a cardiac tissue-preferred promoter to reduce or inhibit expression of a native protein in cardiac tissue.

In addition to antisense technologies, gene expression can be repressed by double stranded RNA including short-hairpin RNA (shRNA), RNA interference (RNAi), short terminal RNA (stRNA), mikroRNA (miRNA) or short interfering RNA (siRNA) (Schutze N. (2004) Mol Cell Endocrinol. 213, 115-119). These RNA interfering approaches can use RNA of varying sizes, but are generally limited to 15-28 nucleotides and act by an as yet unclear mechanism. This technique has been successfully employed in vitro and in vivo as a means to inhibit gene functions (McCaffrey AP et al. (2002) Nature 418,38-39).

Criteria evaluated for augmented contractility and heart failure progression include, but are not limited to, β-receptor number, β-receptor coupling, adenylyl cyclase activity, cAMP levels at rest, cAMP levels after forskolin administration, PKA activity, PKA protein levels, L-type calcium channel current density, SERCA2a protein levels, and phospholamban mRNA levels, or phospholamban phosphorylation of proteins.

Compounds that can be screened in accordance with the assays of the invention include but are not limited to, libraries of known compounds, including natural products, such as plant or animal extracts, synthetic chemicals, biologically active materials including proteins, peptides such as soluble peptides, including but not limited to members of random peptide libraries and combinatorial chemistry derived molecular library made of D- or L- configuration amino acids, phosphopeptides (including, but not limited to, members of random or partially degenerate, directed phosphopeptide libraries), antibodies (including, but not limited to, polyclonal, monoclonal, chimeric, human, anti-idiotypic or single chain antibodies, and Fab, F(ab')₂ and Fab expression library fragments, and epitope-binding fragments thereof), organic and inorganic molecules.

In addition to the more traditional sources of test compounds, computer modeling and searching technologies permit the rational selection of test compounds by utilizing structural information from the ligand binding sites of proteins of the present invention. Such rational selection of test compounds can decrease the number of test compounds that must be screened in order to identify a therapeutic compound. Knowledge of the sequences of proteins of the present invention allows for the generation of models of their binding sites that can be used to screen for potential ligands. This process can be accomplished in several manners known in the art. A preferred approach involves generating a sequence alignment of the protein sequence to a template (derived from the crystal structures or NMR-based model of a similar protein(s), conversion of the amino acid structures and refining the model by molecular mechanics and visual examination, If a strong sequence alignment cannot be obtained then a model may also be generated by building models of the hydrophobic helices. Mutational data that point towards residue-residue contacts may also be used to position the helices relative to each other so that these contacts are achieved. During this process, docking of the known ligands into the binding site cavity within the helices may also be used to help position the helices by developing interactions that would stabilize the binding of the ligand. The model may be completed by refinement using molecular mechanics and loop building using standard homology modeling techniques. General information regarding modeling can be found in Schoneberg, T. et. al., Molecular and Cellular Endocrinology, 151:181-193 (1999), Flower, D., Biochimica et Biophysica Acta, 1422:207-234 (1999), and Sexton, P.M., Current Opinion in Drug Discovery and Development, 2(5):440-448 (1999).

Once the model is completed, it can be used in conjunction with one of several existing computer programs to narrow the number of compounds to be screened by the screening methods of the present invention, like the DOCK program (UCSF Molecular Design Institute, 533 Parnassus Ave, U-64, Box 0446, San Francisco, California 94143-0446). In several of its variants it can screen databases of commercial and/or proprietary compounds for steric fit and rough electrostatic complementarity to the binding site. Another program that can be used is FLEXX (Tripos Inc., 1699 South Hanley Rd., St. Louis, MO).

As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is known in the art. Except insofar as any conventional media or agent is incompatible with the active compound, such media can be used in the compositions of the invention. Supplementary active compounds can also be incorporated into the compositions. A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose, pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor ELTM (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a carboxypeptidase protein or anti- carboxypeptidase antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For oral administration, the agent can be contained in enteric forms to survive the stomach or further coated or mixed to be released in a particular region of the GI tract by known methods. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel (RTM), or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. "Dosage unit form" as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Anti-cardiomyopathic compounds identified by the methods of this invention may be used in the treatment of humans.

### METHODS

### Example 1. Generation of Transgenic Mice

The *PKCα* gene was targeted for deletion by standard homologous recombination in embryonic stem cells, followed by production of chimeric mice, which were bred and passed the targeted allele into the germline. The exon encoding the ATP binding cassette in *PKCα* was deleted resulting in a null allele with regards to protein expression. For generation of PKCα overexpressing transgenic mice, a cDNA encoding PK*C*α was subcloned into the murine α-myosin heavy chain promoter-containing expression vector and injected into newly fertilized oocytes. The *MLP*, PP1c, and pressure-overload surgical model (TAC) were all described elsewhere (Arber et al. (1997) Cell 88:393-403; Carr et al. (2002) Mol. Cell. Biol. 22:4124-4135; and Liang et al. (2003) EMBO, J. 22:5079-5089). Males were exclusively used in all studies for consistency. All animal experiments were approved by the Institutional Animal Care and Use Committee.

### Example 2. Echocardiographic Analysis

Mice from all genotypes or treatment groups were anesthetized with isoflurane, and echocardiography was performed using a Hewlett Packard 5500 instrument with a 15-MHZ microprobe. Echocardiographic measurements were taken on M-mode in triplicate from four separate mice per group. The isolated ejecting mouse heart preparation used in the present study has been described in detail previously (Gulick et al. (1997) Circ. Res. 80:655-664), as was the close-chested working heart model employed here (Lorenz et al. (1997) Am. J. Physiol. 272:H1137-H1146).

### Example 3. Histological Hypertrophic Marker Gene Analyses

Hearts were collected at the indicated times, fixed in 10% formalin containing PBS, and embedded in paraffin. Serial 5-µm heart sections from each group were analyzed. Samples were stained with hematoxylin and eosin or Masson's trichrome. Cardiac gene expression of hypertrophic molecular markers was assessed by RNA dot-blot analysis as described previously (Jones et al. (1996) J. Clin. Invest 98:1906-1917).

### Example 4. Contractility in Single Adult Rat Cardiac Myocytes after Adenoviral Infection

Ventricular myocytes were isolated from Sprague-Dawley rat hearts (Westfall et al, (1997 Methods Cell Biology 52:307-322), and plated on laminin-coated coverslips in DMEM with 5% serum for hr. Media was then replaced with serum-free DMEM containing a recombinant viral vector. Serum-free DMEM was added after 1 hr, and media was changed every 2 days. About 70-85% of isolated cells are rod shaped, with 1-2 x 10⁶ rod-shaped myocytes per heart. Myocytes used for shortening assays were electrically stimulated in media 199 supplemented with Penicillin/Streptomycin, 10 mM Hepes, 0.2 mg/ml albumin, and 10 mM glutathione (Westfall and Borton, (2003) J. Biol. Chem. 278:33694-33700). Myocytes were transferred to a stimulation chamber with platinum electrodes 1 day after plating, and stimulated at 0.2 Hz with a 2.5 ms pulse at a voltage producing twitches in <25% of myocytes. Media in the stimulation chamber was replaced every 12 hrs. For contractile function studies, coverslips were mounted in a thermo-controlled chamber containing M199 for sarcomere shortening measurements. Sarcomere length was measured via a variable field rate CCD video camera (Ionoptix; Milton, MA), and recorded with sarcomere length detection software. Myocytes were stimulated at 0.2 Hz, and sarcomere shortening was recorded for 60 sec. Measurements of peak shortening, time to peak, time to half maximal relaxation and contraction plus relaxation rates were obtained from the signal average for 10 contractions. Results from these studies were compared by a one-way analysis of variance and a post-hoc Newman-Keuls test.

### Example 5. Electrophysiological Recordings

Cardiac myocytes were dissociated from the ventricles of 3-month-old wildtype or non-transgenic (Ntg) and PKCα-KO mice and electrophysiological recording were performed as described before (Petrashevskaya et al. (2002) Cardiovasc. Res. 54;117-132 and Masaki et al. (1997) Am. J. Physiol. 272:H606-H612). Briefly, the heart was subject to retrograde coronary perfusion with Ca²⁺-free Tyrode's solution for 10 minutes, and with Tyrode's solution (250 µM Ca²⁺) containing collagenase type II (Worthington; 1.0 mg/ml) supplemented with 5 mM taurine and 10 mM BDM (2,3-butane, dione-monoxamine) for 8-12 minutes at 37°C bubbled with 95% O₂ and 5% CO₂. At the end of the perfusion the heart was removed and the ventricular tissues were mechanically minced in low Cl⁻, high K⁺ -KB medium. The minced ventricular tissue was then gently filtered, and stored at 4°C until electrophysiological study. Only Ca²⁺ tolerant cells with clear cross striations and without spontaneous contraction or significant granulation were selected for experiments.

Experiments were performed on the dissociated cardiac myocytes at 20 to 24°C. All current recordings were obtained in the whole cell, voltage-clamp configuration af the patch clamp technique by using 1.60 OD borosilicate glass electrodes (Garner Glass Company). Cell capacitance was calculated by integrating the area under an uncompensated capacity transient elicited by a 25 mV hyperpolarizing test pulse (25 ms) from a holding potential of 0 mV. Resistance was within the range of 2 to 11 MΩ. Most of the data presented in these studies were obtained with electrodes having a resistance of 0.5-3 MΩ. After formation of a high resistance seal between the recording electrode and the myocyte membrane, electrode capacitance was fully compensated electronically before breaking the membrane patch. I_{Ca} currents were elicited by depolarizing voltage steps (380 ms) from -50 mV to +40 mV in 10 mV increments from a holding potential -60 mV. The recorded currents were filtered at 2 kHz through a four-pole low-bass Bessel filter and digitized at 5 kHz. The experiments were controlled using pClamp 5.6 software (Axon Instruments) and analyzed using Clampfit 6.0.3. Ca²⁺ currents were recorded using an external solution containing (in mM): CaCl₂ 1.8, tetraethyl-ammonium chloride (TEA-Cl) 135, 4-aminopyridine (4-AP) 5, glucose 10, HEPES 10, MgCl₂, (pH 7.3). The pipette solution contained (mM): cesium aspartate 100, CsCl 20, MgCl₂ 1, Mg-ATP 2, Na₂-GTP 0.5, EGTA 5, HEPES 5, (pH 7.3 with CsOH). These solutions isolated I_{Ca} from other membrane currents such as Na⁺ and K⁺ channel currents and also Ca²⁺ flux through the Na⁺/Ca²⁺ exchanger.

### Example 6. Calcium Transient Measurements

Isolation of mouse left ventricular myocytes for assessment of calcium transient measurements was carried out as described previously (Chu et al. (1996) Circ. Res. 79:1064-1076). Ca²⁺ transients were measured from cardiomyocytes at room temperature. Briefly, mouse hearts were excised from anesthetized (pentobarbital sodium, 70mg/kg, i.p.) adult mice, mounted in a Langendorff perfusion apparatus, and perfused with Ca²⁺-free Tyrode solution at 37°C for 3 min. The normal Tyrode solution contained 140 mM NaCI, 4 mM KCI, 1 mM MgCl₂, 10mM glucose, and 5mM HEPES, pH 7.4. Perfusion was then switched to the same solution containing 75 units/ml type 1 collagenase (Worthington), and perfusion continued until the heart became flaccid (~10-15min). The left ventricular tissue was excised, minced, pipette-dissociated, and filtered through a 240-µm screen. The cell suspension was then sequentially washed in 25, 100, 200 µM and 1 mM Ca²⁺⁻Tyrode. To obtain intracellular Ca²⁺ signals, cells were incubated with the acetoxymethyl ester form of fura-2 (Fura-2/AM; 2 µM) for 30 min at room temperature and resuspended in 1.8 mM Ca²⁺-Tyrode solution. The myocyte suspension was placed in a Plexiglas chamber, which was positioned on the stage of an inverted epifluorescence microscope (Nikon Diaphot 200), and perfused with 1.8 mM Ca²⁺-Tyrode solution at room temperature (22°C-23°C). Myocyte contraction was field-stimulated by a Grass S5 stimulator (0.5 Hz, square waves), and the cells were alternately excited at 340 and 380nm 5 by Delta Scan dual-beam spectrophotofluorometer (Photon Technology International). Ca²⁺ transients were recorded as the 340/380 nm ratio of the resulting 510 nm emissions, Baseline and amplitude, estimated by the 340/380nm ratio, and the times for 80% decay of the Ca²⁺ signal and tau were acquired. All data were analyzed using software from FeliX and Ionwizard.

### Example 7. PKCα Phosphorylation of I-1 in vitro

PKC kinase reaction mixtures included 10 µM inhibitor-1, 2D mM MOPS, pH 7.2, 25 mM β-glycerol phosphate, 1 mM MgCl₂, 1 mM sodium orthovanadate, 1 mM DTT, 1 mM CaCl₂, 0.1 mg/ml phosphatidylserine, 0.01 mg/ml diacylglycerol, 100 µM ATP, and 0.6 mCi/ml ^{[32P]}ATP. PKC was isolated from rabbit heart muscle (Woodgett and Hunter, (1987) J. Biol. Chem. 262:4836-4843) and recombinant wildtype and Ser-67-Ala inhibitor-1 was isolated from *E. coli* (Bibb et al. (2001) J. Biol. Chem. 276:14490-14497). Reactions were conducted at 30°C and aliquots were removed at specific time points and stopped by addition of protein sample buffer. Stoichiometries were determined by SDS-PAGE and direct quantification of radioactivity.

### Example 8. Primary Cardiomyocyte Cell Culture

Primary cultures of neonatal rat cardiomyocytes were obtained by enzymatic dissociation of 1-2 day-old Sprague-Dawley rat neonates as described previously (De Windt et al. (2000) J. Biol, Chem. 275:13571-13579). Cardiomyocytes were cultured under serum-free conditions in M199 media supplemented with penicillin/streptomycin (100 U/ml) and L-glutamine (2mmol/L).

### Example 9. Replication Deficient Adenoviruses

The characterization of adenovirus-encoding wildtype or dominant negative mutants of PKCα in cardiomyocytes was described previously (Braz et al. (2002) J. Cell Biol. 156:905-919). The dominant negative PKCα cDNA consisted of a lysine to arginine mutation in the ATP binding domain at amino acid position 368. Each recombinant adenovirus was plaque purified, expanded, and titered in HEK293 cells. Typical experiments involved infection of 6 neonatal rat cardiomyocytes at a moi of 100 plaque forming units for 2 h at 37°C in a humidified, 6% CO₂ incubator. Subsequently, the cells were cultured in semin-free M199 media for an additional 24 h before analysis. Under these conditions 95% of the cells showed expression of the recombinant protein.

### Example 10. PKC Translocation Assay and Immunoblot Analysis

Soluble and particulate fractions were prepared as described previously (Braz et al. (2002) J. Cell Biol. 156:905-919). Protein samples were subjected to SDS-PAGE (10% gels), transferred to Hybond-P membrane (Amersham Pharmacia Biotech), blocked in 7% milk, and incubated with primary antibodies against PKCα, β, δ, ε, SERCA2, calsequestrin, PLB, phospho-serine-16 PLB, inhibitor-1, and PP1cα. Phospho-specific I-lantibodies were described previously (Bibb et al. (2001) J. Biol. Chem. 276:14490-14497. Primary antibodies were incubated overnight in 3% milk at 4°C. Secondary antibodies IgG (alkaline phosphatase-conjugated antimouse, -rabbit, or -goat) were incubated for 1 h at room temperature in 0.5-3% milk. Chemifluorescent detection was directly performed with the Vistra ECF reagent (RPN 5785; Amersham Pharmacia Biotech) and scanned with a PhosphorImager or chemiluminescence was performed with ECL (Amersham Pharmacia Biotech) and exposed on film.

### Example II. Immunoprecipitation and Protein Phosphatase Activity Assays

Protein extracts were generated from cardiomyocytes infected with adenovirus encoding β-galactosidase, I-1, PKCα, and PKCα-dn. Extracts were immunoprecipitated with PPIcα conjugated to agarose beads, followed by western blotting against I-1. Preparation of phosphorylated protein substrate and radioactive assay of protein phosphatases were prepared as instructed by the Protein Serine/Threonine Phosphatase (PSP) Assay System (New England BioLabs, Inc.).

### Example 12. Caffeine Induced Calcium Transients

Caffeine induced calcium transients were measured in a total of 37 myocytes from 4 PKCα. null mice and 19 control myocytes from 3 wild type mice. After collagenase digestion, myocytes were loaded with Indo-1 AM (25 µg/ 2 ml) for 12 min at room temperature. Intracellular calcium transients (measured by Indo-1 fluorescence ratio) were recorded at resting state (no electrical stimulation) before and during 20 mM caffeine addition.

### Example 13. Cardiac Functionality Assessment

Hearts were isolated from four wild-type and four PKCα, -/- (PKCα null) transgenic mice. The isolated hearts were infused with PMA at 9 different concentrations ranging from 8 X 10⁻¹¹ through 8 X 10⁻⁷ M. Acute PMA infusion of each concentration occurred for a 7 minute period. The hearts were measured for maximal and minimal dP/dt in systole and diastole respectively. Results from one such experiment are presented in Fig. 36,

### Example 14. PKC Isozyme Abundance Assessment

A standard curve was used to assess the relative abundance of the PKC isozymes in healthy human hearts. Recombinant human protein PKCα, PKCβI, PKCβII, PKCγ, and PKCε generated in bacteria were purchased from a commercial vendor. Three aliquots of known concentrations were prepared.

Adult human ventricular tissue was explanted from six undiseased individuals. Whole cell protein lysates were prepared. The three standard PKC aliquots and the heart proteins were subjected to polyacrylamide gel electrophoresis on the same gel. The proteins were transferred to a membrane. The membrane was blocked and incubated with antibodies specific to the PKCα, PKCβI, PKCβII, PKCγ, and PKCε isozymes. Data from such an experiment are presented in Figure 37.

### Example 15. Cardiac Functionality Assessment

The relatively selective PKCα/β inhibitory compound Ro-32-0432 [2-{8-[(Dimethylamino)methyl]-6,7,8,9-tetrahydropyrido[1,2- a]indol-3-yl}-3-(1-methylindol-3-yl)maleimide, HCl Salt] [3-{8-[(Dimethylamino)methyl]-6,7,8,9-tetrahydropyrido[1,2- a]indol-10-yl}-4-(1-methylindol-3-yl)-1H-pyrrole-2,5-dione, HCl Salt] was used as a means of directly examining the effects of acute PKCα inhibition on cardiac function and contractility using an ex vivo working heart preparation. The working heart preparation separates the inherent pump function of the heart from potential alterations in total vascular resistance as might occur if the drug were infused in vivo.

Working adult wildtype mouse hearts were infused with vehicle control (10% DMSO) or Ro-32-0432 in 10% DMSO at concentrations ranging between 4x10⁻¹⁰ through 4x 10⁻⁶ M. Four animals were analyzed in the Ro-32-0432 group and compared with three animals in the vehicle control group. Values throughout the concentration time course (7 minutes per 10 different incremental concentrations) were summated for statistical purposes, representing an average dosage of approximately 1x10⁻⁸ M. The vehicle control and experimental groups showed heart rates of 363 +/- 15 and 295 +/- 26 beats per minute, respectively, before any treatments were begun. The average heart rate of the vehicle and drug treated groups was 351 +/= 3 and 292 +/- 6 beats per minute, respectively. Despite the lower heart rate, the Ro-32-0432 infused group showed an increase in acute contractile function measured as maximum dP/dt, and an increase in left ventricular pressure developed. The approximate 20% change in acute contractile performance in the Ro-32-0432 treated group is similar to the increase in cardiac function observed in PKCα null mice. The data from one such experiment are presented in Figure 38.

### Example 16. Translocation of a PKCα Indicator Polypeptide

A PKCα indicator was prepared by operably linking a nucleotide sequence encoding PKCα to a nucleotide sequence encoding green fluorescence protein (GFP). An expression cassette comprising the PKCα-GFP nucleotide sequence was prepared. Adenovirus comprising the PKCα-GFP expression cassette was prepared.

Neonatal rat cardiomyocytes were cultured in plastic dishes and incubated until the appropriate density was reached. The cardiomyocytes were infected with an adenovirus encoding PKCα-GFP. The cultures were incubated for 24 hours. After 24 hours the cells were incubated with either DMSO alone (the vehicle treatment) or DMSO and PMA for 60 minutes. The cells were fixed and examined by confocal microscopy. The PMA stimulated cells exhibit a highly localized and punctate staining pattern whereas the vehicle only stimulated cells exhibit a relatively diffuse PKCα-GFP localization.

### Example 17. In Vivo Evaluation of PKCα Inhibitors in the Anesthetized Rat.

Selected PKCα inhibitors are evaluated in both naive rats and rats with myocardial infarction (MI) for effects on cardiac contractility and hemodynamics.

Male, Sprague-Dawley or Lewis rats weighing between 225-500 gm are anesthetized with isoflurane and an MI is induced as follows. A thoracotomy at the fourth or fifth intercostal space is done, the heart is exposed and the pericardium is opened. A 5-0 suture is placed around the left descending coronary artery 2-4 mm from its origin and permanently tied. The ribs, muscle and skin are separately closed and the animal is allowed to recover. Twenty to twenty-three weeks after surgery the animals are used to evaluate the effects of PKCα inhibitors on cardiac contractility and hemodynamics.

The effects of inhibitors on cardiac contractility and hemodynamics are evaluated in naive and MI rats as follows. The animals are anesthetized with isoflurane. A femoral artery is isolated and cannulated for the measurement of systemic blood pressure. A jugular vein is isolated and cannulated for the intravenous infusion of inhibitor. The right carotid artery is isolated and a Millar conductance catheter is inserted to the left ventricle (LV) of the heart. The LV systolic pressure, end-diastolic pressure, +maximum dP/dt, -minimum dP/dt, and heart rate are derived from the LV pressure waveform. Mean arterial blood pressure is derived from the systemic blood pressure waveform. Data are recorded continuously and derived using computerized data acquisition software (Notocord or Powerlab).

After a period of stabilization, PKCα inhibitors are infused at the following infusion doses in naive rats: 0.1, 0.3, 1.0, 3.0, 10, 30, 100, 300 and 1000 nmol/kg/min. The infusion of each dose is allowed to run for at least five minutes. In MI rats, the infusion doses are as follows: 10, 30, 100, 300, and 1000 nmol/kg/min for at least five minutes. Equivalent infusion volumes are administered to separate, vehicle-control naïve and MI animals. At the end of the test infusions, 5.0 µg/kg/min of dobutamine is infused.

### Example 18. Method of Identifying Anti-Cardiomyopathic Compounds

This assay can be used for a variety of cardiomyopathic phenotypes. A PKCα nucleotide sequence of interest is cloned into an expression vector containing a cardiac tissue-preferred promoter. The expression cassette comprising the promoter, operably linked to the nucleotide sequence of interest is digested with a restriction enzyme. The restriction reaction products are electrophoresed on an agarose gel, and the expression cassette is purified from the agarose. The expression cassette is prepared for microinjection according to any method known to one skilled in the art. The expression cassette is used to provide a transgenic mouse. The presence of the transgene is confirmed using Southern blot analysis.

Two cohorts of age-matched transgenic mice are established. The diet of one cohort is supplemented with a compound of interest. The diet of the second cohort is supplemented with a placebo. The two mice cohorts are incubated for an appropriate time and the experiment is terminated. The mice are monitored for a cardiomyopathic phenotype such as hypertrophy using the left ventricle/body mass ratios described elsewhere herein. A cardiomyopathic phenotype presented by the mice of the each cohort is compared. Alternatively, the compound may be administered directly to the animals using established methodologies and technologies, including but not limited to, intra-arterial or intravenous injection of a compound by syringe or osmotic mini-pumps or other means, oral gavage, intraperitoneal injection or subcutaneous injection.

### EXPERIMENTAL RESULTS AND DISCUSSION

In the following figures, data are presented with the standard error of the mean, unless otherwise indicated.
Figure 1. The PKCα locus (also called *Prkcα*) was targeted by homologous recombination in embryonic stem cells so that the exon encoding the catalytic ATP binding cassette was deleted by replacement with the neomycin resistance marker (shown in panel A). Genomic targeting was detected by Southern blotting with EcoRV digested DNA and a 5' probe external to the region of vector homology (shown in panel B), demonstrating correct targeting and deletion of the selected exon. Correctly targeted embryonic stem cells were used to generate germline-containing PKCα. targeted mice using common techniques routinely employed in the previous art. PKCα+/- mice were intercrossed, generating *PKCα-*/*-* progeny at the predicted Mendelian frequencies. Panel C shows western blotting for PKCα protein levels from heart protein extracts derived from wildtype, PKCα+/- and PKCα-/- mice, demonstrating that PKCα protein is completely eliminated in PKCα-/- mice and reduced by approximately 50% in PKCα+/- mice compared with non-targeted wildtype mice.
Figure 2. To evaluate the potential that other PKC isozymes might compensate for the loss of PKCα in the heart, western blotting was performed from hearts from 2 month-old *PKCα-*/mice subjected to pressure-overload by transverse aortic constriction (TAC) for 2 weeks, or sham control animals. Wildtype control animals were also subjected to TAC or sham operations. Protein extracts from these hearts were separated into soluble (S) or particulate (P) fractions and western blotted for select PKC isozymes. The data demonstrate that PKCα-/- mice completely lack PKCα protein, while PKCβ, δ and ε levels or translocation efficiencies were unaffected. These results indicate that alternate PKC isozymes are unlikely to overtly compensate for the loss of PKCα in the heart.
Figure 3. Close-chested invasive hemodynamic assessment of 6 PKCα-/- and 6 non-targeted wildtype mice demonstrated a 15-20% increase in maximum dP/dt at baseline, with a corresponding parallel increase in performance following β-adrenergic receptor stimulation with dobutamine. These results indicate that PKCα-/- mice have hypercontractile hearts in vivo.
Figure 4. To assess the intrinsic function of the heart apart from potential hemodynamic compensatory responses, an ex vivo anterograde working heart preparation was performed at 2 and 10 months of age from PKCα-/- or wildtype mice (4 hearts in each group). Each heart was paced at approximately 400 beats per minute to ensure equal assessment of functional capacity. *PKCα-*/*-* hearts showed a 15% and 32% increase in maximum dP/dt at 2 and 10 months, respectively, compared to age-matched, wildtype littermate controls (panel A). A corresponding increase in left ventricular pressure development was also observed in *PKCα-*/*-* mice (panel B). These results further indicate that PKCα-/- mice have hypercontractile hearts and the defects are not compensated by other mechanisms found in the whole animal.
Figure 5. Close-chested invasive hemodynamic assessment of 6 PKCα-/- and 6 non-targeted wildtype mice demonstrated no change in heart rate (panel A) or mean arterial blood pressure (panel B). These results indicate that the increase in contractility observed in PKCα-/hearts is not due to a secondary alteration in blood pressure or heart rate.
Figure 6. To assess the gain-of-function phenotype associated with PKCα protein ablation in the heart, transgenic mice were generated that overexpress the wildtype PKCα cDNA under the control of the cardiac-specific α-myosin heavy chain promoter (panel A). Quantitative western blotting from heart protein extracts of wildtype mice or the PKCα-overexpressing transgenic mice demonstrated 5-fold overexpression of PKCα protein in transgenic hearts, without any compensatory changes in PKCβ, δ, or ε (panel B, upper). Western blotting of cardiac protein extracts derived from wildtype, PKCα transgenic, or PKCα-/- mice showed increased autophosphorylation of PKCα, suggesting greater activity due to the transgene (panel B, lower). PKCα-/- heart extract was used as a migration control in the western blotting procedure. These results indicate that PKCα transgenic mice have significantly greater PKCα activity in the heart.
Figure 7. PKCα transgenic mice manifest signs of cardiomyopathy. By 4 months of age, PKCα transgenic mice show reduced fractional shortening as measured by echocardiography compared to age and strain-matched wildtype controls, suggesting that increased PKCα activity diminishes cardiac contractile performance in vivo (panel A). This conclusion is also supported by assessment of maximal dP/dt as assessed by an ex vivo working heart preparation (panel B), which also shows reduced cardiac functional performance in PKCα transgenic mice compared with wildtype controls. Four animals were used in each group (A & B) in the above experiments.
Figure 8. PKCα transgenic mice did not manifest signs of cardiac hypertrophy until 6 and 8 months of age, a time slightly after reduced contractile performance was noted in Figure 7. The gradual manifestation of cardiac hypertrophy by 6 and 8 months of age is a consequence of reduced contractile performance, that together indicates that enhanced PKCα. activity in the heart produces cardiomyopathy. Four animals were used in each group.
Figure 9. While *PKCα* gene-targeted and transgenic mice demonstrated an antithetic cardiac contractility phenotype, the potential for secondary effects associated with a chronic alteration in PKCα activity cannot be disregarded. To address this concern an acute model of PKCα activation or inhibition was instituted in wildtype adult rat cardiac myocytes, followed by examination of single cell contractile responsiveness. Adenoviral-mediated gene transfer of wildtype or dominant negative PKCα reduced and enhanced myocyte contractility, respectively, as measured by peak shortening (P<0.05). Maximal shortening velocity was also similarly affected, with values of 4.04 ±0.23 µm/sec in control adult myocytes compared to 3.16±0.25 µm/sec and 5.48±0.36 µm/sec in wildtype and dominant negative PKCα adenoviral-infected myocytes, respectively (P<0.05). These data indicate that acute alterations in PKCα activity impact myocyte contractility, consistent with the genetic mouse models presented in Figures 1-8.
Figure 10. *PKCα-I-* hearts showed a hyperphosphorylation of phospholamban (PLB) resulting in retarded migration by western blotting and an increase in direct phosphorylation at serine-16, without a change in SERCA2 or calsequestrin protein levels (panels A,B). The pentameric form of PLB is shown to more represent differences in migration. Interestingly, the observed profile of PLB hyperphosphorylation was also associated with reduced PLB protein levels so that the PLB/SERCA2 protein ratio was reduced by 50-70%, which is predicted to render SERCA2 more active (panel A). Direct measurement of PLB phosphorylation at serine 16, the site know to alter the contractile effectiveness of PLB, was increased in the hearts of PKCα-/mice compared with wildtype (Wt) control hearts (panels C,D). These results indicate a potential mechanism whereby loss of PKCα protein enhances cardiac contractile performance through diminished PLB effectiveness in inhibiting SERCA2a activity.
Figure 11. The overall regulatory paradigm between PKCα and PLB was also observed in acutely infected adult rat cardiomyocytes. Specifically, dominant negative PKCα expression by adenoviral-mediated gene transfer was associated with increased phosphorylation of PLB at serine-16. Collectively, these results indicate that alterations in PKCα signaling impacts PLB phosphorylation status and protein levels, suggesting a mechanism whereby PKCα ablation or overexpression might affect contractility.
Figure 12. No change in PLB or SERCA2 mRNA levels were detected between wildtype and *PKCα-*/*-* hearts by RNA dot blotting (panel A) or semiquantitative RT-PCR (panel B), suggesting that the observed down-regulation of PLB protein presented in Figure 10 results from a post-transcriptional mechanism. This decrease in PLB protein levels is hypothesized to result from decreased protein stability due its net dissociation form the stabilizing SERCA2 complex in the SR.
Figure 13. PKCα transgenic mice, which have more PKCα activity and protein in the heart, showed an antithetic alteration in PLB compared to the PKCα-/- mice. Specifically, PLB phosphorylation was reduced in the heart, while total protein was increased by 2.1-fold (P<0.05) (panels A-D). The observed dephosphorylated state of PLB, in conjunction with an increase in total protein, would significantly inhibit SERCA2 activity. Thus, overexpression of PKCα reduces cardiac contractility. The pentameric form of PLB is shown to demonstrate the shift in protein migration.
Figure 14. Alterations in PLB phosphorylation should directly alter SERCA2 function, thus effecting calcium loading within the sarcoplasmic reticulum and the magnitude of the calcium transient. Adult cardiac myocytes isolated from *PKC*α-/- mice showed enhanced calcium transients, suggesting greater calcium loading within the sarcoplasmic reticulum (panel A). Fura-2 loaded *PKC*α*-*/*-* cells demonstrated a 52% increase in the peak release of calcium, as well as 17% faster calcium re-uptake (T₈₀) corresponding to a 20% reduction in the time constant Tau (n=36 cells from 6 wildtype mice and 33 cells from 4 *PKCα*-/- mice) (panel B). These data are consistent with enhanced SERCA2a function and larger calcium loads within the sarcoplasmic reticulum, thus reflecting a hyperdynamic state of the myocardium.
Figure 15. These results suggest that the augmented calcium transient observed in *PKC*α-/- cardiac myocytes is due to increased loading of calcium within the sarcoplasmic reticulum. Direct measurement of peak calcium release induced by caffeine administration in Indo-1-loaded cardiomyocytes demonstrated significantly greater sarcoplasmic reticulum calcium loads from *PKC*α-/- mice compared with wildtype (Wt) controls (P<0.05) (panels A shows a representative calcium tracing, B shows the quantitative data on peak caffeine-induced calcium release).
Figure 16. The observed increase in the calcium transient presented in Figure 14 could also be due, in part, to augmentation in L-type calcium current within the sarcolemma. However, direct measurement of mean *I_{Ca}* density did not vary between wildtype and *PKCα-*/*-* cardiac myocytes.
Figure 17. The alterations observed in PLB phosphorylation, without a corresponding change in β-adrenergic receptor signaling or protein kinase A activity suggested a potential role for a phosphatase that acts on PLB. To investigate this potential effector pathway, PP1- and PP2A-specific phosphatase assays were performed from wildtype hearts and *PKCα-*/*-* hearts (N=4 hearts each). Total protein phosphatase activity was decreased approximately 18% in *PKCα-*/hearts, while PP1-specific activity was decreased by greater than 30% and PP2A-specific activity was not significantly different. These results indicate that loss of PKCα is associated with a decrease in PP1 activity within the heart.
Figure 18. Reciprocal to the data shown in PKCα-/- mouse hearts, PKCα overexpressing transgenic mice showed a significant increase in PP1 activity in the heart, but no change in PP2A activity. These results indicate that increased PKCα activity within the heart is associated with a specific increase in PF 1 activity. Data are expressed as the relative phosphatase activity.
Figure 19. Consistent with the data presented in Figures 17 and 18, acute adenoviral infection of cultured cardiomyocytes showed a 60% reduction in PP1 activity with expression of a dominant negative PKCα mutant, and a 30% augmentation in PP1 activity with wildtype PKCα overexpression (from triplicate experiments). That acute alterations in PKCα correspond with an alteration in PP1 activity suggests that PKCα might directly regulate PP1 activity, through a mechanism that will be elaborated in subsequent figures.
Figure 20. PP 1 activity is regulated by a class of inhibitory proteins, such as inhibitory protein-1 (I-1). I-1 directly binds PP1 resulting in the inhibition of PP1 activity, although I-1's ability to bind PP1 depends on its phosphorylation status from inducible signals. To examine the hypothesis that PKCα might directly phosphorylate 1-1, thus regulating its association with PP1, an in vitro phosphorylation experiment was performed with bacterial generated I-1 and purified PKC in the presence of ³²P-ATP. Wildtype I-1 protein was directly phosphorylated in vitro in a time-dependent manner at stoichiometric levels by PKC. Analysis of putative PKC phosphorylation sites within I-1 revealed a consensus motif at serine-67. Recombinant S67A mutant I-1 protein showed approximately 50% less phosphorylation by PKC compared with equal amounts of wildtype protein.
Figure 21. To further examine the potential mechanism whereby PP1 activity was altered by PKCα, a series of I-1 immunoprecipitation experiments was performed from adenoviral-infected cardiomyocytes subjected to PP1c pull-down followed by I-1 western blotting (the input lanes were not immunoprecipitated). The data demonstrate that wildtype PKCα overexpression specifically reduced the ability of I-1 to interact with PP1c by approximately 50%, while dominant negative PKCα (dn) augmented complex formation by greater than 70%. Total PP1c levels did not vary in each of the immunoprecipitation reactions.
Figure 22. The ability of I-1 to interact with and inhibit PP1 is also regulated by protein kinase A-mediated phosphorylation of threonine-35 in I-1. Phosphorylation at this site renders I-1 a more potent inhibitor of PP1, thus reducing its activity, opposite to the effect associated with phosphorylation ofserine-67 by PKCα. We investigated the effect of wildtype or dominant negative PKCα expression on I-I phosphorylation at either threonine-35 or serine-67 using phospho-specific antibodies generated against each site. Cultured cardiomyocytes were infected with AdI-1 (Ad = adenovirus) to increase the sensitivity of the assay, together with Adβgal, AdPKCα-wt or AdPKCα-dn. No change in threonine-35 phosphorylation was observed in response to PKCα modulation. However, AdPKCα-dn expression significantly decreased 1-1 serine-67 phosphorylation by more than 70%, while AdPKCα-wt augmented phosphorylation by greater than 60%.
Figure 23. The data presented in Figures 20-22 were extended in vivo using *PKC*α transgenic and gene-targeted mice in which endogenous 1-1 phosphorylation was analyzed from heart extracts. Western blotting with serine-67 phospho-specific antisera demonstrated a significant reduction in phosphorylation from *PKCα-l-* hearts (more than 50%), while PKCα transgenic hearts had increased phosphorylation (2-fold) (P<0.05).
Figure 24. Consistent with the data shown in Figures 20-23, western blotting with protein extracts obtained from dilated failing human hearts also showed an increase in I-1 senne-67 phosphorylation compared with normal donor human hearts (panel B). These data are also consistent with a general increase in PKCα protein levels as assessed by western blotting from the same protein extracts (P<0.05) (panel A). These results indicate that failing, cardiomyopathic human hearts show an increase in PKCα levels and serine 67 phosphorylation in I-1.
Figure 25. Confocal immunohistochemistry of PKCα protein in adult rat cardiomyocytes in culture shows PMA-induced translocation to the membrane and Z-lines. In unstimulated cells, PKCα is localized throughout the cell, but acute stimulation with PMA causes a rapid translocation to structures that are coincident with an enrichment af PLB and SERCA2 at the z-line within the sarcoplasmic reticulum. These data indicate that PKCα, once activated, translocates to the proper intracellular localization to affect calcium handling within the sarcoplasmic reticulum.
Figure 26. Here we tested the hypothesis that the relatively mild hypercontractile status observed in *PKCα* gene-targeted mice might benefit a failing heart. *PKCα-*/*-* mice and wildtype littermate controls were subjected to long-term aortic banding-induced heart failure. Mice underwent TAC within the thoracic cavity beginning at 8 weeks of age for a period of 12 weeks, after which cardiac function was assessed by working heart preparation. Hearts from wildtype mice showed a 50% reduction in maximal dP/dt and a 35% reduction in LVP compared to sham operated controls of the same age, while *PKC*α null mice did not show a significant decrease in either parameter (N=4 hearts in each cohort). These results indicate that PKCα-/- mice are resistant to pressure overload-induced cardiac decompensation and loss of contractility.
Figure 27. Echocardiography was also performed to further examine contractility affects following long-term aortic banding-induced heart failure as described in Figure 26. *PKCα* null mice and wildtype littermate controls were subjected to TAC within the thoracic cavity beginning at 8 weeks of age for a period of 12 weeks, after which cardiac function was assessed by echocardiography. Hearts from wildtype mice subjected to 12 weeks of TAG showed a more prominent increase in left ventricular end diastolic (LVED) and systolic (LVES) ventricular chamber dimensions compared with PKCα-/- subjected to the same stimulus (panel A). Cardiac left ventricular fractional shortening (FS) was also more prominently depressed in wildtype TAC mice compare with PKCα-/- mice, which showed much less loss of ventricular performance (panel B). These results further indicate that PKCα-/- mice are resistant to pressure overload-induced cardiac decompensation and loss of contractility in vivo.
Figure 28. A mouse model of dilated cardiomyopathy due to ablation of the *muscle lim protein (MLP)* gene was also analyzed as a second heart failure model. By echocardiography, 2 month-old *MLP* null mice showed reduced functional capacity and greater left ventricular chamber dilation compared with wildtype controls or PKCα-/- mice (panels A, B). However, *MLP* null mice that were null for *PKCα* showed a significant improvement in heart failure symptoms, such as less ventricular dilation (LVED and LVES) and preserved fractional shortening (panels A,B). These results indicate that loss of PKCα prevents cardiac dysfunction and remodeling in another mouse model of cardiomyopathy and heart failure in vivo.
Figure 29. Contractility was also assessed in MLP-/- mice using the isolated ex vivo working heart preparation. These data showed a significant reduction in cardiac contractility in MLP-/- mice that was prevented in mice that were also null for PKCα (double nulls), as measured by changes in maximum dP/dt or left ventricular pressure developed (LVP). These results further indicate that loss of PKCα prevents cardiac dysfunction in the MLP mouse model of cardiomyopathy and heart failure ex vivo.
Figure 30. The prevention of heart failure and reductions in cardiac contractility by deletion of PKCα within the MLP-/- background suggested that other aspects of cardiomyopathy might be reduced. Compared with MLP-/- mice, the double null mice (also missing PKCα), showed a loss in reactive hypertrophy that typifies the *MLP* null phenotype. Thus, enhancing contractility through PKCα deletion prevented the manifestations of dilated cardiomyopathy related to increases in heart weight (HW) to body weight (BW) ratio increases.
Figure 31. Consistent with the data presented in Figure 30, single MLP-/- mice had histological disease associated with a dilated and enlarged myocardium in longitudinal section, while the double null mice (also missing PKCα) showed essentially no pathology. These results further support the contention that PKCα deletion prevents the manifestations of dilated cardiomyopathy related to histopathology and gross morphological changes.
Figure 32. Transgenic mice expressing 3-fold more PP1 catalytic subunit in the heart are known to have reduced functional capacity and cardiomyopathy by 3 months of age. Given our data that suggest PKCα can directly regulates PP1 activity in the heart (Figures 18-24), we reasoned that loss of PKCα would partially inhibit the increased activity associated with moderate overexpression of PP1. *PKCα* null mice crossed with PP1 transgenic mice demonstrated a significant reduction in PP1 activity in the heart. Hearts derived from PP1 transgenic mice showed an approximate increase in PP1 activity of 2.5 fold compared with hearts from wildtype mice. Once again, hearts from PKCα-/- mice showed a significant reduction in cardiac PP1 activity. No change in PP2A was observed. These results indicate that loss of PKCα reduces the effectiveness of overexpressed PP1 in the heart.
Figure 33. By 3 months of age, PP1 transgenic mice showed significant reductions in ventricular performance as assessed by echocardiography. However, deletion of PKCα within the PP1 transgenic background, which was shown in Figure 32 to reduce activity of PP1, effectively prevented the loss of ventricular performance. These results indicate that PKCα can prevent cardiomyopathic effects and contractile deficits observed in PP1 transgenic mice in vivo.
Figure 34. At 3 months of age, PP1 transgenic mice also have reduced contractility as measured with an ex vivo working heart preparation. However, deletion of PKCα within the PP1 transgenic background, similarly prevented the loss of contractility as measured by changes in maximum dP/dt, minimum dP/dt, and left ventricular pressure developed (LVP). These results further support the conclusion that PKCα can reverse the cardiomyopathic effects and contractile deficits observed in PP1 transgenic mice ex vivo.
Figure 35. To demonstrate the benefits of PKCα inhibition on mortality resulting from heart failure and cardiomyopathy, death was also quantified as an end-point. The 12 week TAC experiment described in Figures 26 and 27 was also monitored for animal deaths in both control wildtype mice, as well as PKCα-/- mice. The data show that significantly more deaths were observed in wildtype mice subjected to TAC over the 12 week time course of TAC compared with PKCα-/- mice subjected to TAC. No deaths were observed in sham control mice of either genotype. These results indicate that loss of PKCα protects mice from TAC-induced heart failure and ultimately, an untimely demise. In addition, mortality was also assessed in the MLP-/- mice. Figure 35B indicates that MLP-/- mice have a high mortality rate compared to the other groups presented. The high mortality rate in MLP-/- mice is attenuated in mice lacking both MLP and PKCα (double nulls). The data indicate that PKCα ablation/inhibition in the setting of MLP ablation and heart failure, provides a survival benefit.
Figure 36. To more carefully assess the potential role of PKCα as a regulator of cardiac contractility we used acute administration of PMA to wildtype or FKCα-/- hearts in the ex vivo working heart preparation. The PKC-activating class of compounds referred to as phorbol esters were employed to elicit acute, PKC-dependent alterations in cardiac contractility. Here isolated hearts were infused with PMA at 9 different concentrations ranging from 8x10⁻¹¹ through 8x10⁻⁷ M (panels A,B). The data show that acute PMA infusion has essentially no effect on the contractile performance of wildtype mouse hearts at concentrations ranging from 8x10⁻¹¹ through 8x10⁻⁹ M, with respect to either Maximum dP/dt or Minimum dP/dt (panels A,B). However, concentrations higher than 8x10⁻⁹ M produced a marked decrease in functional performance in wildtype mouse hearts, suggesting that PKC activation could reduce cardiac contractility in this preparation (panels A,B). However, PKCα null hearts subjected to the same concentrations of PMA showed an immediate positive inotropic effect to law doses of PMA, and only a mild depression in functional performance at the highest concentrations of PMA (Figure 36 A,B). These results indicate that PMA-induced depression of cardiac contractility directly depends on PKCα. Such data support a critical role for PKCα as an acute negative regulatory of contractility, distinct from other PKC isozymes that are also activated by PMA.
Figure 37. Since PKCα may serve as a novel target for altering cardiac contractility acutely, and hence affect heart failure, it was of interest to examine the relative abundance of PKCα versus the other classic PKC isozymes from the human heart. Recombinant human protein standards (generated in bacteria) were purchased from a commercial vendor so that a standard curve of protein content versus signal intensity by western blotting could be generated for PKCα, βI, βII, γ and ε. These protein standards were run on the same gel and subjected to antibody detection by western blotting as whole cell protein samples derived from 6 normal human hearts (panels A,B). The data demonstrate that PKCα is expressed at significantly higher levels than the other PKC isozymes that were analyzed in the human heart (Panels A,B). These results suggest that PKCα is a prominent PKC isoform in the human heart
Figure 38. The relatively selective PKCα/β inhibitory compound Ro-32-0432 [2-{8-[(Dimethylamino)methyl]-6,7,8,9-tetrahydropyrido[1,2-a]indol-3-yl}-3-(1-methylindol-3-yl)maleimide, HCI Salt] was used as a means of directly examining the effects of acute PKCα inhibition on cardiac function and contractility using an ex vivo working heart preparation. Adult wildtype mouse hearts were infused vehicle control (10% DMSO) or Ro-32-0432 in 10% DMSO at concentrations ranging between 4x10⁻¹⁰ through 4x10⁻⁶ M. All values throughout the concentration time course (7 minutes per 10 different incremental concentrations) were summated for statistical purposes, representing an average dosage of approximately Ix10⁻⁸ M. The vehicle control and experimental groups showed heart rates of 363 +/- 15 and 295 +/- 26 beats per minute, respectively, before any treatments were begun. The average heart rate of the vehicle and drug treated groups was 351 1 +/- 3 and 292 +/- 6 beats per minute, respectively. Despite the lower heart rate, the Ro-32-0432 infused group showed a 20% increase in acute contractile function, measured as maximum dP/dt, and a 20% increase in left ventricular pressure developed (P<0,05) (panels A,B). Four animals were analyzed in the Ro-32-0432 group and compared with three animals in the vehicle control group. The approximate 20% change in acute contractile performance in the Ro-32-0432 treated group is similar to the increase in cardiac function observed in PKCα null mice discussed earlier in this application. Collectively, these results indicate the acute inhibition of PKCα, using Ro-32-0432, effectively augments cardiac function and contractility.
Figure 39. PKC isozyme translocation is often associated with activation, and PKC inhibitory agents can block this translocation event. A PKCα-green fluorescent protein (GFP) fusion expressing adenovirus was generated as a means of carefully monitoring PKCα translocation, or inhibition of translocation in neonatal cardiomyocyte cultures. In response to vehicle treatment (DMSO), PKCα-GFP was unaffected compared to untreated, showing a fairly diffuse localization throughout the cell, with a mild sarcomeric organization. However, 60 minutes of PMA stimulation caused a robust redistribution of PKCα-GFP, so that the diffuse background of localization was replaced with a highly localized and punctate staining pattern with less overall fluorescence. The net effect of such redistribution is a change in local fluorescence characteristics in each cell, which could be easily detected in a large-scale screening assay. Thus, the appropriate PKCα inhibitory compound could be quickly identified based on PKCα-GFP cellular redistribution.
Figures 40A and 40B. In order to demonstrate the ability of a PKCα inhibitor to modulate contractility in vivo, a PKC inhibitor, LY333531, (S)-13[(monomethylamino)methyl]-10,11,14,15-tetrahydro-4,9:16,21-dimetheno-1*H*, 13*H*-dibenzo [E,K] pyrrolo-[3,4-*H*][1,4,13]oxadiaza-cyclohexidine-1,3(2*H*)-dione (Burkey JL et al.(2002) Xenobiotica. 32,1045-1052), was administered in normal rats (n=3) as described in the experimental section. LY333531 was dissolved in 20 % Sulfobutyl ether-B-cyclodextrin sodium salt (Captisol) in a 50 mM acetate buffer at pH 5.0. The compound was infused for 5 minutes at each concentration in Figure 40. At the 1000 nmol/kg/min dose, LY333531 demonstrated a significant increase in maximum dP/dt (Figure 40A) and minimum dP/dt (Figure 40B). Figures 40A shows maximum dP/dt and Figure 40B shows minimum dP/dt including no compound (baseline; B/L) and following infusion of 0,1, 0.3, 1,3, 10, 30, 100, 300 and 1000 nmol/kg/min of LY333531, which are indicated on the abscissa. The drug was then stopped for 5 minutes (P/D) and dobutamine (Dob) was administered at 5.0 µg/kg/min for 5 minutes. In Figure 40B, values for minimum dP/dt are expressed as the absolute or numeric value for simplicity. At the 1000nmol/kg/min dose, maximum dP/dt was increased 28% while minimum dP/dt was increased 17% compared to baseline measurements, consistent with the data in the PKCα null mice and administration of Ro-32-0432 in isolated work-performing heart preparations. In Figure 40A and 40B, the asterisk indicates a statistically significant difference (P<0.05) from baseline values, as determined by a one-way analysis of variance (ANOVA) with a Dunnett's Multiple Comparisons post-hoc test. This increase in cardiac contraction and relaxation at 1000 nmol/kg/min occurred in the absence of effects on heart rate (Baseline: 315±17 beats per minute vs. 1000 nmol/kg/min: 294±8 beats per minute, not statistically significant) or left ventricular systolic blood pressure (Baseline: 90±3 mmHg vs. 1000 nmol/kg/min: 105±6 beats per minute, not statistically significant). These data indicate that PKCα inhibition in normal rats result in positive cardiac inotropy (contraction) and lusitropy (relaxation).. LY338522, an active metabolite of LY333531, has also shown to be effective in inhibiting the PKC isoforms (Burkey JL et al.(2002) Xenobiotica. 32, 1045-1052).
Figure 41. In order to demonstrate the efficacy of PKCα in vivo in a myocardial infarction model, Ro-31-8220, 3-[1-[3-(Amidinothio)propyl-1H-indol-3-yl]-3-(1-methyl-1H-indol-3-yl)maleimide, Bisindolylmaleimide IX, Methanesulfonate, a known PKC inhibitor (Han Z et al. (2000) Cell Death Differ. 7, 521-530) was infused in rats (n=4) which underwent surgery to induce a myocardial infarction (MI rats). Ro-31-8220 was dissolved in 20 % Sulfobutyl ether-B-cycladextrin sodium salt (Captisol) in a 50 mM acetate buffer at pH 5.0. Ro-31-8220 was delivered in vivo as described in the experimental section. Infusion of Ro-31-8220 resulted in a dose dependent enhancement of the percent increase in maximum dP/dt (21%) which reached statistical significance (P<0.05) at the 300 nmol/kg/min dose (Figure 41; One-way ANOVA and Dunnett's Multiple Comparisons post-hoc test). Figure 41 shows the percent increase in maximum dP/dt from baseline (B/L) following infusion of 10, 30, 100, 300 and 1000 nmol/kg/min of Ro-81-8220, which are indicated on the abscissa. The drug was then stopped for 5 minutes (P/D) and dobutamine (Dob) was administered at 5.0 µg/kg/min for 5 minutes. These data indicate that inhibition of PKCα results in a positive inotropic effect in a rat model of heart failure. The inotropic benefit observed with infusion of Ro-31-8220 was greater than that seen with dobutamine, a clinically administered inotrope in ADHF. These data suggest that delivery of a PKCα inhibitor to human's inflicted with myocardial dysfunction in contraction or relaxation, such as that observed in ADHF, would provide a functional benefit in these patients, a desired outcome of medical treatment.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more".

All documents cited are, in relevant part, incorporated herein by reference; the citation of any document is not to be construed as an admission that it is prior art with respect to the present invention

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope.

## Claims

1. The use of a protein kinase C-α modulating compound in the manufacture of a medicament for treating or preventing a disorder modulated by cardiac contractility in a mammal.

2. The use of a protein kinase C-α modulating compound in the manufacture of a medicament for treating or preventing a cardiomyopathy in a mammal.

3. The use of a protein kinase C-α modulating compound in the manufacture of a medicament for treating or preventing an acute heart failure in a mammal in need thereof.

4. The use of a protein kinase C-α modulating compound according to any of claims 1, 2, or 3, wherein the protein kinase C-α modulating compound is a protein kinase C-α inhibitor selected from the group consisting of Ro-32-0432, LY333531 and Ro-31-8220.

5. A protein kinase C-α modulating compound for use in treating or preventing a disorder modulated by cardiac contractility in a mammal.

6. A protein kinase C-α modulating compound for use in treating or preventing a cardiomyopathy in a mammal.

7. A protein kinase C-α modulating compound for use in treating or preventing an acute heart failure in a mammal in need thereof.

8. A protein kinase C-α modulating compound for use according to any of claims 5, 6, or 7, wherein the protein kinase C-α modulating compound is a protein kinase C-α inhibitor selected from the group consisting of Ro-32-0432, LY333531 and Ro-31-8220.

9. A method for identifying compounds that modulate cardiac contractility, comprising the steps of :
(a) contacting a compound with protein kinase C-α protein;
(b) determining whether the compound binds protein kinase C-α; and
(c) identifying those compounds that bind protein kinase C-α as modulators of cardiac contractility.

10. A method for identifying compounds that modulate cardiomyopathy, comprising the steps of :
(a) contacting a compound with protein kinase C-α protein;
(b) determining whether the compound binds protein kinase C-α ; and
(c) identifying those compounds that bind protein kinase C-α as compounds as modulators of cardiomyopathy.

11. The method of claim 9 or claim 10, wherein the protein kinase C-α protein is expressed in a cell and the effect of the modulator is measured as change in the protein kinase C-α activity as compared to a cell that is not contacted with the compound.

12. The method of claim 9 or 11, further comprising the steps of :
(a) selecting those compounds that modulate activity of protein kinase C-α protein, and further determining whether those compounds modulate cardiac contractility in a cardiac contractility model system; and
(b) identifying those test compounds that modulate cardiac contractility in the cardiac contractility model system as candidate compounds for modulating cardiac contractility.

13. The method of claim 10 or 11, further comprising the steps of :
(a) selecting those compounds that modulate activity of protein kinase C-α protein, and further determining whether those compounds modulate cardiomyopathy in a cardiomyopathy model system; and
(b) identifying those test compounds that modulate cardiomyopathy in the cardiomyopathy model system as candidate compounds for modulating cardiomyopathy.

14. The use of a transgenic mouse, said mouse comprising at least one stably incorporated expression cassette in the genome of at least one cell, said expression cassette comprising a cardiac tissue-preferred regulatory sequence operably linked to a nucleotide sequence encoding a polypeptide having protein kinase C-α acitivity, or a cell or tissue thereof, as the model system in the method of any of claims 11, 12, or 13.

15. A transgenic mouse comprising at least one disrupted protein kinase C-α gene in the genome of at least one cell, the disruption being sufficient to decrease protein kinase C-α expression levels.


SEQUENCE LISTING

<110>  Children's Hospital Medical Center
       UNIVERSITY OF CINCINNATI

<120>  REGULATION OF CARDIAC CONTRACTILITY AND HEART FAILURE PROPENSITY

<130>  P02-0264/EP/2

<150>  EP04809768.7
<151>  2004-09-17

<160>  15

<170>  PatentIn version 3.3

<210>  1
<211>  2841
<212>  DNA
<213>  Oryctolagus cuniculus


<220>
<221>  CDS
<222>  (205)..(2223)

<400>  1
gcgagcgaga gagagccagc gagcggctcc ggctccgcgc cgcgccgcgc ccgctcgcct      60

ctccccggcc gccgccgcct ccagcccgcg ccccgcgccg gggtcgcccc tcgccccgcc     120

gcccctcccc cgcccgcccc cgcccacccc ggccctcgcc ggctgccgct ccccggcgga     180

ggcaagaggt ggttgggggg gacc atg gct gac gtt ttc ccg gcc aac gac        231
                           Met Ala Asp Val Phe Pro Ala Asn Asp
                           1               5

tcc acg gcg tct cag gac gtg gcc aac cgc ttc gcc cgc aaa ggg gcg       279
Ser Thr Ala Ser Gln Asp Val Ala Asn Arg Phe Ala Arg Lys Gly Ala
10                  15                  20                  25

ctg agg cag aag aac gtg cac gag gtg aaa gac cac aaa ttc atc gcg       327
Leu Arg Gln Lys Asn Val His Glu Val Lys Asp His Lys Phe Ile Ala
                30                  35                  40

cgc ttc ttc aag cag ccc acc ttc tgc agc cac tgc acc gac ttc atc       375
Arg Phe Phe Lys Gln Pro Thr Phe Cys Ser His Cys Thr Asp Phe Ile
            45                  50                  55

tgg ggg ttt ggg aag caa ggc ttc cag tgc caa gtt tgc tgt ttt gtg       423
Trp Gly Phe Gly Lys Gln Gly Phe Gln Cys Gln Val Cys Cys Phe Val
        60                  65                  70

gtt cac aag agg tgc cat gaa ttt gtt acc ttt tct tgt ccg ggt gcg       471
Val His Lys Arg Cys His Glu Phe Val Thr Phe Ser Cys Pro Gly Ala
    75                  80                  85

gat aag gga cct gac act gat gac ccc agg agc aag cac aag ttt aaa       519
Asp Lys Gly Pro Asp Thr Asp Asp Pro Arg Ser Lys His Lys Phe Lys
90                  95                  100                 105

atc cac acc tac ggg agc ccc acc ttc tgc gat cac tgt ggg tca ctg       567
Ile His Thr Tyr Gly Ser Pro Thr Phe Cys Asp His Cys Gly Ser Leu
                110                 115                 120

ctg tac gga ctc atc cac cag ggg atg aaa tgt gac acc tgc gac atg       615
Leu Tyr Gly Leu Ile His Gln Gly Met Lys Cys Asp Thr Cys Asp Met
            125                 130                 135

aac gtt cac aag cag tgt gtg atc aat gtc ccc agc ctg tgt ggg atg 663
Asn Val His Lys Gln Cys Val Ile Asn Val Pro Ser Leu Cys Gly Met
        140                 145                 150

gac cat acg gag aag agg gga cgg atc tac ctg aag gcc gag gtc acc 711
Asp His Thr Glu Lys Arg Gly Arg Ile Tyr Leu Lys Ala Glu Val Thr
    155                 160                 165

gat gaa aag ctc cac gtc acg gta cga gat gca aaa aac ctg atc ccc 759
Asp Glu Lys Leu His Val Thr Val Arg Asp Ala Lys Asn Leu Ile Pro
170                 175                 180                 185

atg gat ccc aac ggg ctt tca gat ccc tac gtg aag ctg aaa ctc att 807
Met Asp Pro Asn Gly Leu Ser Asp Pro Tyr Val Lys Leu Lys Leu Ile
                190                 195                 200

cct gat ccc aag aat gaa agc aaa caa aaa acc aaa acc atc cgc tcc 855
Pro Asp Pro Lys Asn Glu Ser Lys Gln Lys Thr Lys Thr Ile Arg Ser
            205                 210                 215

acg ctg aac ccg cag tgg aac gaa tcc ttt acc ttc aaa tta aaa ccc 903
Thr Leu Asn Pro Gln Trp Asn Glu Ser Phe Thr Phe Lys Leu Lys Pro
        220                 225                 230

tcc gac aaa gac cga cga ctg tct gta gaa atc tgg gac tgg gac cga 951
Ser Asp Lys Asp Arg Arg Leu Ser Val Glu Ile Trp Asp Trp Asp Arg
    235                 240                 245

aca aca agg aat gac ttc atg ggg tcg ctc tcc ttt ggc gtc tcg gag 999
Thr Thr Arg Asn Asp Phe Met Gly Ser Leu Ser Phe Gly Val Ser Glu
250                 255                 260                 265

ctc atg aag atg cca gcc agt gga tgg tac aag ttg ctg aac caa gaa 1047
Leu Met Lys Met Pro Ala Ser Gly Trp Tyr Lys Leu Leu Asn Gln Glu
                270                 275                 280

gaa ggt gaa tac tac aat gtg ccc att ccg gaa ggg gac gaa gat ggg 1095
Glu Gly Glu Tyr Tyr Asn Val Pro Ile Pro Glu Gly Asp Glu Asp Gly
            285                 290                 295

aac gtg gag ctc agg cag aaa ttc gag aaa gcc aag ctg ggg ccc gct 1143
Asn Val Glu Leu Arg Gln Lys Phe Glu Lys Ala Lys Leu Gly Pro Ala
        300                 305                 310

ggc aac aag gtg atc agt ccc tcg gaa gac agg aag cag ccg tcc aac 1191
Gly Asn Lys Val Ile Ser Pro Ser Glu Asp Arg Lys Gln Pro Ser Asn
    315                 320                 325

aac ctg gac cga gtg aag ctc acc gac ttc aac ttc ctc atg gtc ctg 1239
Asn Leu Asp Arg Val Lys Leu Thr Asp Phe Asn Phe Leu Met Val Leu
330                 335                 340                 345

gga aaa ggc agt ttt ggg aag gtg atg ctg gcg gac agg aag ggc acg 1287
Gly Lys Gly Ser Phe Gly Lys Val Met Leu Ala Asp Arg Lys Gly Thr
                350                 355                 360

gaa gag ctg tac gcc atc aag atc ctg aag aag gac gtg gtg att cag 1335
Glu Glu Leu Tyr Ala Ile Lys Ile Leu Lys Lys Asp Val Val Ile Gln
            365                 370                 375

gat gac gac gtg gag tgc acc atg gtg gag aag cgg gtt ctg gcc ctg 1383
Asp Asp Asp Val Glu Cys Thr Met Val Glu Lys Arg Val Leu Ala Leu
        380                 385                 390

atg gac aag ccg ccc ttc ctg aca cag ctg cac tcc tgc ttc cag acc 1431
Met Asp Lys Pro Pro Phe Leu Thr Gln Leu His Ser Cys Phe Gln Thr
    395                 400                 405

gtg gac cgg ctg tac ttt gtc atg gaa tac gtc aac ggc gga gac ctc 1479

Val Asp Arg Leu Tyr Phe Val Met Glu Tyr Val Asn Gly Gly Asp Leu
410                 415                 420                 425

atg tac cac atc cag caa gtg gga aag ttc aag gag cca caa gca gta      1527
Met Tyr His Ile Gln Gln Val Gly Lys Phe Lys Glu Pro Gln Ala Val
                430                 435                 440

ttc tat gca gca gag att tcc att ggg ctg ttc ttc ctt cac aaa aga      1575
Phe Tyr Ala Ala Glu Ile Ser Ile Gly Leu Phe Phe Leu His Lys Arg
            445                 450                 455

ggg atc atc tat agg gac ctg aag cta gat aac gtc atg ctg gac tcg      1623
Gly Ile Ile Tyr Arg Asp Leu Lys Leu Asp Asn Val Met Leu Asp Ser
        460                 465                 470

gaa gga cac atc aaa atc gct gac ttt ggg atg tgc aag gaa cac atg      1671
Glu Gly His Ile Lys Ile Ala Asp Phe Gly Met Cys Lys Glu His Met
    475                 480                 485

atg gac ggg gtc acc acc agg acc ttc tgt ggg act cca gat tac atc      1719
Met Asp Gly Val Thr Thr Arg Thr Phe Cys Gly Thr Pro Asp Tyr Ile
490                 495                 500                 505

gcc ccg gag atc atc gct tat cag ccg tac ggc aaa tcc gtg gac tgg      1767
Ala Pro Glu Ile Ile Ala Tyr Gln Pro Tyr Gly Lys Ser Val Asp Trp
                510                 515                 520

tgg gcc tac ggc gtc ctg ctg tat gag atg ctg gct ggg cag cct cca      1815
Trp Ala Tyr Gly Val Leu Leu Tyr Glu Met Leu Ala Gly Gln Pro Pro
            525                 530                 535

ttt gat ggc gag gac gaa gac gag ctg ttt cag tcc atc atg gag cac      1863
Phe Asp Gly Glu Asp Glu Asp Glu Leu Phe Gln Ser Ile Met Glu His
        540                 545                 550

aac gtt tcc tac ccc aaa tcc ttg tcc aag gag gcc gtc tcc atc tgc      1911
Asn Val Ser Tyr Pro Lys Ser Leu Ser Lys Glu Ala Val Ser Ile Cys
    555                 560                 565

aag ggg ctg atg acc aaa cac ccg gcc aag cgg ctg ggc tgc ggg ccc      1959
Lys Gly Leu Met Thr Lys His Pro Ala Lys Arg Leu Gly Cys Gly Pro
570                 575                 580                 585

gag gga gag cgg gac gtg agg gaa cac gcc ttc ttc cgg agg atc gac      2007
Glu Gly Glu Arg Asp Val Arg Glu His Ala Phe Phe Arg Arg Ile Asp
                590                 595                 600

tgg gag aaa ctg gag aac agg gag atc cag ccg ccg ttc aag ccc aaa      2055
Trp Glu Lys Leu Glu Asn Arg Glu Ile Gln Pro Pro Phe Lys Pro Lys
            605                 610                 615

gtg tgt ggc aaa gga gca gaa aac ttc gac aag ttc ttc acg cga ggg      2103
Val Cys Gly Lys Gly Ala Glu Asn Phe Asp Lys Phe Phe Thr Arg Gly
        620                 625                 630

cag ccc gtc gtc acg ccg ccg gat caa ctg gtc atc gct aac ata gac      2151
Gln Pro Val Val Thr Pro Pro Asp Gln Leu Val Ile Ala Asn Ile Asp
    635                 640                 645

cag tct gat ttc gaa ggg ttc tcg tat gtc aac ccc cag ttc gtg cac      2199
Gln Ser Asp Phe Glu Gly Phe Ser Tyr Val Asn Pro Gln Phe Val His
650                 655                 660                 665

ccg atc cta cag agc tca gta tga aagtcgccgg cgagacccag cgtgcccacc      2253
Pro Ile Leu Gln Ser Ser Val
                670

cctccagccc ccagcacccc tagcggcggg aagtgatcct taaccctaaa attttaaggc      2313

cacggcctta tgtttttgct ccacatggag gcctgaaaat tgtagggtta tagtcccaag 2373

gcgaccagct atccagggtc tctctcttaa aaccaagaac attatgttag tggatacgca 2433

aatcgtgaac agcggccggt ttgatcatgt gggagtcgca cctggctgta ggccaacccc 2493

tcctaggtag aaagcaggcc ctcgcccctc tttttttttt tttttttttt ggtacgattt 2553

ggtatacttc ccatatcccg tgtctggctg gattctctcc atgaggctcc gcccaagcag 2613

agatgcgaaa gtgaacctgc cccagcctgg tgggccggaa gcacctccaa ccaggaggta 2673

aaggaatggg gagcccggag aggggcgggg gaggtctgcg agctgccgac cacggtctgt 2733

gcctcggtgg aaacgaccga ccccctgggt gtgagaggcc gggacaagac tacttactat 2793

tccccgacat cgacacggcg tcaccaactc tagcccagct tccaaaac 2841

<210> 2
<211> 672
<212> PRT
<213> Oryctolagus cuniculus

<400> 2

Met Ala Asp Val Phe Pro Ala Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Thr Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Asp Gly Asn Val Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Lys
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Met Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys His
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Val Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ser Val
            660                 665                 670

<210>  3
<211>  2465
<212>  DNA
<213>  Mus musculus

<220>
<221>  CDS
<222>  (163)..(2181)

<400>  3
gcgagcgaga gagagccagc gagcggctcc ggctccgcgc cgcgccgcgc ccgctcgcct      60

ctccccggcc gccgccgcct ccagcccgcg ccccgcgccg gggtcgcccc tcgccccgcc 120

cgcagcgagc cagagagccg gagagagcag agagagcggc tc ggc tcc cag ctc 174
Gly Ser Gln Leu
1

gag tgg cgc agg ccg ccc ggt ctc cgg ccc gcg cac acg atc tac acg 222
Glu Trp Arg Arg Pro Pro Gly Leu Arg Pro Ala His Thr Ile Tyr Thr
5                   10                  15                  20

cac tgg cgc ggc cgc cac cgc tct gtg tcc ggc gga ggc agg agg tgg 270
His Trp Arg Gly Arg His Arg Ser Val Ser Gly Gly Gly Arg Arg Trp
                25                  30                  35

ttg ggg gga acc atg gct gac gtt tac ccg gcc aac gac tcc acg gcg 318
Leu Gly Gly Thr Met Ala Asp Val Tyr Pro Ala Asn Asp Ser Thr Ala
            40                  45                  50

tct cag gac gtg gcc aac cgc ttc gcc cgc aaa ggg gcg ctg agg cag 366
Ser Gln Asp Val Ala Asn Arg Phe Ala Arg Lys Gly Ala Leu Arg Gln
        55                  60                  65

aag aac gtg cat gag gtg aaa gac cac aaa ttc atc gcc cgc ttc ttc 414
Lys Asn Val His Glu Val Lys Asp His Lys Phe Ile Ala Arg Phe Phe
    70                  75                  80

aag caa ccc acc ttc tgc agc cac tgc acc gac ttc atc tgg ggg ttt 462
Lys Gln Pro Thr Phe Cys Ser His Cys Thr Asp Phe Ile Trp Gly Phe
85                  90                  95                  100

ggg aaa caa ggc ttc cag tgc caa gtt tgc tgt ttt gtg gtt cat aag 510
Gly Lys Gln Gly Phe Gln Cys Gln Val Cys Cys Phe Val Val His Lys
                105                 110                 115

agg tgc cat gag ttc gtt acg ttc tct tgt ccg ggt gcg gat aag gga 558
Arg Cys His Glu Phe Val Thr Phe Ser Cys Pro Gly Ala Asp Lys Gly
            120                 125                 130

cct gac act gac gac ccc agg agc aag cac aag ttc aaa atc cac aca 606
Pro Asp Thr Asp Asp Pro Arg Ser Lys His Lys Phe Lys Ile His Thr
        135                 140                 145

tac gga agc cct acc ttc tgt gat cac tgt ggg tcc ctg ctc tat gga 654
Tyr Gly Ser Pro Thr Phe Cys Asp His Cys Gly Ser Leu Leu Tyr Gly
    150                 155                 160

ctt atc cac caa ggg atg aaa tgt gac acc tgc gac atg aat gtt cac 702
Leu Ile His Gln Gly Met Lys Cys Asp Thr Cys Asp Met Asn Val His
165                 170                 175                 180

aag cag tgt gtg atc aat gtc cct agc ctc tgc gga atg gat cac aca 750
Lys Gln Cys Val Ile Asn Val Pro Ser Leu Cys Gly Met Asp His Thr
                185                 190                 195

gag aag agg ggg cgg att tat ctg aag gct gag gtc act gat gaa aag 798
Glu Lys Arg Gly Arg Ile Tyr Leu Lys Ala Glu Val Thr Asp Glu Lys
            200                 205                 210

ctc cac gtc acg gta cga gat gca aaa aat cta atc cct atg gat cca 846
Leu His Val Thr Val Arg Asp Ala Lys Asn Leu Ile Pro Met Asp Pro
        215                 220                 225

aat ggg ctt tcg gat cct tat gtg aag ctg aaa ctt atc cct gac ccc 894
Asn Gly Leu Ser Asp Pro Tyr Val Lys Leu Lys Leu Ile Pro Asp Pro
    230                 235                 240

aag aat gag agc aaa cag aaa acc aaa acc atc cgc tcc aca ctg aat 942
Lys Asn Glu Ser Lys Gln Lys Thr Lys Thr Ile Arg Ser Thr Leu Asn
245                 250                 255                 260

cct cag tgg aat gag tcc ttc acg ttc aaa tta aaa cct tca gac aaa 990
Pro Gln Trp Asn Glu Ser Phe Thr Phe Lys Leu Lys Pro Ser Asp Lys
                265                 270                 275

gac cgg cga ctg tct gta gaa atc tgg gac tgg gat cgg acg act cgg 1038
Asp Arg Arg Leu Ser Val Glu Ile Trp Asp Trp Asp Arg Thr Thr Arg
            280                 285                 290

aat gac ttc atg gga tcc ctt tcc ttt ggt gtc tca gag cta atg aag 1086
Asn Asp Phe Met Gly Ser Leu Ser Phe Gly Val Ser Glu Leu Met Lys
        295                 300                 305

atg ccg gcc agt gga tgg tat aaa ctg ctc aac caa gaa gag ggc gaa 1134
Met Pro Ala Ser Gly Trp Tyr Lys Leu Leu Asn Gln Glu Glu Gly Glu
    310                 315                 320

tat tac aat gtg ccc att cca gaa gga gat gaa gaa ggc aac atg gaa 1182
Tyr Tyr Asn Val Pro Ile Pro Glu Gly Asp Glu Glu Gly Asn Met Glu
325                 330                 335                 340

ctc agg cag aag ttt gag aaa gcc aag cta ggc cct gct ggt aac aaa 1230
Leu Arg Gln Lys Phe Glu Lys Ala Lys Leu Gly Pro Ala Gly Asn Lys
                345                 350                 355

gtc atc agc cct tca gaa gac aga aag caa cca tcc aac aac ctg gac 1278
Val Ile Ser Pro Ser Glu Asp Arg Lys Gln Pro Ser Asn Asn Leu Asp
            360                 365                 370

aga gtg aaa ctc aca gac ttc aac ttc ctc atg gtg ctg ggg aag ggg 1326
Arg Val Lys Leu Thr Asp Phe Asn Phe Leu Met Val Leu Gly Lys Gly
        375                 380                 385

agt ttt ggg aag gtg atg ctt gct gac agg aag gga acg gag gaa ctg 1374
Ser Phe Gly Lys Val Met Leu Ala Asp Arg Lys Gly Thr Glu Glu Leu
    390                 395                 400

tac gcc atc aag atc ctg aag aag gac gtg gtg atc cag gac gac gac 1422
Tyr Ala Ile Lys Ile Leu Lys Lys Asp Val Val Ile Gln Asp Asp Asp
405                 410                 415                 420

gtg gag tgc acc atg gtg gag aag cgc gtg ctg gcc ctg ctg gac aag 1470
Val Glu Cys Thr Met Val Glu Lys Arg Val Leu Ala Leu Leu Asp Lys
                425                 430                 435

ccg cca ttt ctg aca cag ctg cac tcc tgc ttc cag aca gtg gac cgg 1518
Pro Pro Phe Leu Thr Gln Leu His Ser Cys Phe Gln Thr Val Asp Arg
            440                 445                 450

ctg tac ttc gtc atg gaa tac gtc aac ggc ggg gac ctc atg tac cac 1566
Leu Tyr Phe Val Met Glu Tyr Val Asn Gly Gly Asp Leu Met Tyr His
        455                 460                 465

att cag caa gtc gga aaa ttt aag gag cca caa gca gta ttc tac gca 1614
Ile Gln Gln Val Gly Lys Phe Lys Glu Pro Gln Ala Val Phe Tyr Ala
    470                 475                 480

gcc gag atc tcc atc gga ctg ttc ttc ctt cat aaa aga ggg atc att 1662
Ala Glu Ile Ser Ile Gly Leu Phe Phe Leu His Lys Arg Gly Ile Ile
485                 490                 495                 500

tac agg gat ctg aag ctg gac aat gtc atg ctg gac tca gaa ggg cac 1710
Tyr Arg Asp Leu Lys Leu Asp Asn Val Met Leu Asp Ser Glu Gly His
                505                 510                 515

atc aaa atc gcc gac ttc ggg atg tgc aag gaa cac atg atg gat gga 1758
Ile Lys Ile Ala Asp Phe Gly Met Cys Lys Glu His Met Met Asp Gly
            520                 525                 530

gtc acg acc agg acc ttc tgt ggg act ccg gac tac att gcc cca gag     1806
Val Thr Thr Arg Thr Phe Cys Gly Thr Pro Asp Tyr Ile Ala Pro Glu
        535                 540                 545

ata atc gct tac cag ccg tac ggg aag tct gta gat tgg tgg gcg tac     1854
Ile Ile Ala Tyr Gln Pro Tyr Gly Lys Ser Val Asp Trp Trp Ala Tyr
    550                 555                 560

ggt gtg ctg ctg tac gag atg cta gcc ggg cag cct ccg ttt gat ggt     1902
Gly Val Leu Leu Tyr Glu Met Leu Ala Gly Gln Pro Pro Phe Asp Gly
565                 570                 575                 580

gaa gat gaa gat gaa ctg ttt cag tct ata atg gag cac aac gtg tcc     1950
Glu Asp Glu Asp Glu Leu Phe Gln Ser Ile Met Glu His Asn Val Ser
                585                 590                 595

tac ccc aaa tcc ttg tcc aag gaa gcc gtc tcc atc tgc aaa gga ctt     1998
Tyr Pro Lys Ser Leu Ser Lys Glu Ala Val Ser Ile Cys Lys Gly Leu
            600                 605                 610

atg acc aaa cac cct gcc aag cgg ctg ggc tgc ggg ccc gag gga gag     2046
Met Thr Lys His Pro Ala Lys Arg Leu Gly Cys Gly Pro Glu Gly Glu
        615                 620                 625

agg gat gtc aga gag cat gcc ttc ttc agg agg atc gac tgg gag aaa     2094
Arg Asp Val Arg Glu His Ala Phe Phe Arg Arg Ile Asp Trp Glu Lys
    630                 635                 640

ctg gag aac agg gag atc caa cca cca ttc aag ccc aaa gtg tgt ggc     2142
Leu Glu Asn Arg Glu Ile Gln Pro Pro Phe Lys Pro Lys Val Cys Gly
645                 650                 655                 660

aaa gga gca gaa aac ttt gac aag ttc ttc acg cga gga cagcctgtct     2191
Lys Gly Ala Glu Asn Phe Asp Lys Phe Phe Thr Arg Gly
                665                 670

taacaccacc agatcagctg gtgattgcta acatagacca atctgatttt gaagggttct     2251

cgtatgtcaa cccccagttt gtgcacccaa tcttgcaaag tgcagtatga aactcaaaaa     2311

atgaaagatc aatgctccca gcccccagca tccccagcag tgggaagtga tccttaacca     2371

taaaatttag gctatggctt tgtatttgtt ccacacagag gcctggaatt ctaggggaca     2431

ttagtcaatt acattatcac cttttcctca cccc                                 2465

<210>  4
<211>  673
<212>  PRT
<213>  Mus musculus

<400>  4

Gly Ser Gln Leu Glu Trp Arg Arg Pro Pro Gly Leu Arg Pro Ala His
1               5                   10                  15

Thr Ile Tyr Thr His Trp Arg Gly Arg His Arg Ser Val Ser Gly Gly
            20                  25                  30

Gly Arg Arg Trp Leu Gly Gly Thr Met Ala Asp Val Tyr Pro Ala Asn
        35                  40                  45

Asp Ser Thr Ala Ser Gln Asp Val Ala Asn Arg Phe Ala Arg Lys Gly
    50                  55                  60

Ala Leu Arg Gln Lys Asn Val His Glu Val Lys Asp His Lys Phe Ile
65                  70                  75                  80

Ala Arg Phe Phe Lys Gln Pro Thr Phe Cys Ser His Cys Thr Asp Phe
                85                  90                  95

Ile Trp Gly Phe Gly Lys Gln Gly Phe Gln Cys Gln Val Cys Cys Phe
            100                 105                 110

Val Val His Lys Arg Cys His Glu Phe Val Thr Phe Ser Cys Pro Gly
        115                 120                 125

Ala Asp Lys Gly Pro Asp Thr Asp Asp Pro Arg Ser Lys His Lys Phe
    130                 135                 140

Lys Ile His Thr Tyr Gly Ser Pro Thr Phe Cys Asp His Cys Gly Ser
145                 150                 155                 160

Leu Leu Tyr Gly Leu Ile His Gln Gly Met Lys Cys Asp Thr Cys Asp
                165                 170                 175

Met Asn Val His Lys Gln Cys Val Ile Asn Val Pro Ser Leu Cys Gly
            180                 185                 190

Met Asp His Thr Glu Lys Arg Gly Arg Ile Tyr Leu Lys Ala Glu Val
        195                 200                 205

Thr Asp Glu Lys Leu His Val Thr Val Arg Asp Ala Lys Asn Leu Ile
    210                 215                 220

Pro Met Asp Pro Asn Gly Leu Ser Asp Pro Tyr Val Lys Leu Lys Leu
225                 230                 235                 240

Ile Pro Asp Pro Lys Asn Glu Ser Lys Gln Lys Thr Lys Thr Ile Arg
                245                 250                 255

Ser Thr Leu Asn Pro Gln Trp Asn Glu Ser Phe Thr Phe Lys Leu Lys
            260                 265                 270

Pro Ser Asp Lys Asp Arg Arg Leu Ser Val Glu Ile Trp Asp Trp Asp
        275                 280                 285

Arg Thr Thr Arg Asn Asp Phe Met Gly Ser Leu Ser Phe Gly Val Ser
    290                 295                 300

Glu Leu Met Lys Met Pro Ala Ser Gly Trp Tyr Lys Leu Leu Asn Gln
305                 310                 315                 320

Glu Glu Gly Glu Tyr Tyr Asn Val Pro Ile Pro Glu Gly Asp Glu Glu
                325                 330                 335

Gly Asn Met Glu Leu Arg Gln Lys Phe Glu Lys Ala Lys Leu Gly Pro
            340                 345                 350

Ala Gly Asn Lys Val Ile Ser Pro Ser Glu Asp Arg Lys Gln Pro Ser
        355                 360                 365

Asn Asn Leu Asp Arg Val Lys Leu Thr Asp Phe Asn Phe Leu Met Val
    370                 375                 380

Leu Gly Lys Gly Ser Phe Gly Lys Val Met Leu Ala Asp Arg Lys Gly
385                 390                 395                 400

Thr Glu Glu Leu Tyr Ala Ile Lys Ile Leu Lys Lys Asp Val Val Ile
                405                 410                 415

Gln Asp Asp Asp Val Glu Cys Thr Met Val Glu Lys Arg Val Leu Ala
            420                 425                 430

Leu Leu Asp Lys Pro Pro Phe Leu Thr Gln Leu His Ser Cys Phe Gln
        435                 440                 445

Thr Val Asp Arg Leu Tyr Phe Val Met Glu Tyr Val Asn Gly Gly Asp
    450                 455                 460

Leu Met Tyr His Ile Gln Gln Val Gly Lys Phe Lys Glu Pro Gln Ala
465                 470                 475                 480

Val Phe Tyr Ala Ala Glu Ile Ser Ile Gly Leu Phe Phe Leu His Lys
                485                 490                 495

Arg Gly Ile Ile Tyr Arg Asp Leu Lys Leu Asp Asn Val Met Leu Asp
            500                 505                 510

Ser Glu Gly His Ile Lys Ile Ala Asp Phe Gly Met Cys Lys Glu His
        515                 520                 525

Met Met Asp Gly Val Thr Thr Arg Thr Phe Cys Gly Thr Pro Asp Tyr
    530                 535                 540

Ile Ala Pro Glu Ile Ile Ala Tyr Gln Pro Tyr Gly Lys Ser Val Asp
545                 550                 555                 560

Trp Trp Ala Tyr Gly Val Leu Leu Tyr Glu Met Leu Ala Gly Gln Pro
                565                 570                 575

Pro Phe Asp Gly Glu Asp Glu Asp Glu Leu Phe Gln Ser Ile Met Glu
            580                 585                 590

His Asn Val Ser Tyr Pro Lys Ser Leu Ser Lys Glu Ala Val Ser Ile
        595                 600                 605

Cys Lys Gly Leu Met Thr Lys His Pro Ala Lys Arg Leu Gly Cys Gly

610                 615                 620

Pro Glu Gly Glu Arg Asp Val Arg Glu His Ala Phe Phe Arg Arg Ile
625                 630                 635                 640

Asp Trp Glu Lys Leu Glu Asn Arg Glu Ile Gln Pro Pro Phe Lys Pro
                645                 650                 655

Lys Val Cys Gly Lys Gly Ala Glu Asn Phe Asp Lys Phe Phe Thr Arg
            660                 665                 670

Gly

<210>  5
<211>  2245
<212>  DNA
<213>  homo sapiens

<220>
<221>  CDS
<222>  (28)..(2046)

<400>  5
ggagcaagag gtggttgggg ggggacc atg gct gac gtt ttc ccg ggc aac gac      54
                              Met Ala Asp Val Phe Pro Gly Asn Asp
                              1               5

tcc acg gcg tct cag gac gtg gcc aac cgc ttc gcc cgc aaa ggg gcg      102
Ser Thr Ala Ser Gln Asp Val Ala Asn Arg Phe Ala Arg Lys Gly Ala
10                  15                  20                  25

ctg agg cag aag aac gtg cac gag gtg aag gac cac aaa ttc atc gcg      150
Leu Arg Gln Lys Asn Val His Glu Val Lys Asp His Lys Phe Ile Ala
                30                  35                  40

cgc ttc ttc aag cag ccc acc ttc tgc agc cac tgc acc gac ttc atc      198
Arg Phe Phe Lys Gln Pro Thr Phe Cys Ser His Cys Thr Asp Phe Ile
            45                  50                  55

tgg ggg ttt ggg aaa caa ggc ttc cag tgc caa gtt tgc tgt ttt gtg      246
Trp Gly Phe Gly Lys Gln Gly Phe Gln Cys Gln Val Cys Cys Phe Val
        60                  65                  70

gtc cac aag agg tgc cat gaa ttt gtt act ttt tct tgt ccg ggt gcg      294
Val His Lys Arg Cys His Glu Phe Val Thr Phe Ser Cys Pro Gly Ala
    75                  80                  85

gat aag gga ccc gac act gat gac ccc agg agc aag cac aag ttc aaa      342
Asp Lys Gly Pro Asp Thr Asp Asp Pro Arg Ser Lys His Lys Phe Lys
90                  95                  100                 105

atc cac act tac gga agc ccc acc ttc tgc gat cac tgt ggg tca ctg      390
Ile His Thr Tyr Gly Ser Pro Thr Phe Cys Asp His Cys Gly Ser Leu
                110                 115                 120

ctc tat gga ctt atc cat caa ggg atg aaa tgt gac acc tgc gat atg      438
Leu Tyr Gly Leu Ile His Gln Gly Met Lys Cys Asp Thr Cys Asp Met
            125                 130                 135

aac gtt cac aag caa tgc gtc atc aat gtc ccc agc ctc tgc gga atg      486
Asn Val His Lys Gln Cys Val Ile Asn Val Pro Ser Leu Cys Gly Met
        140                 145                 150

gat cac act gag aag agg ggg cgg att tac cta aag gct gag gtt gct 534
Asp His Thr Glu Lys Arg Gly Arg Ile Tyr Leu Lys Ala Glu Val Ala
    155                 160                 165

gat gaa aag ctc cat gtc aca gta cga gat gca aaa aat cta atc cct 582
Asp Glu Lys Leu His Val Thr Val Arg Asp Ala Lys Asn Leu Ile Pro
170                 175                 180                 185

atg gat cca aac ggg ctt tca gat cct tat gtg aag ctg aaa ctt att 630
Met Asp Pro Asn Gly Leu Ser Asp Pro Tyr Val Lys Leu Lys Leu Ile
                190                 195                 200

cct gat ccc aag aat gaa agc aag caa aaa acc aaa acc atc cgc tcc 678
Pro Asp Pro Lys Asn Glu Ser Lys Gln Lys Thr Lys Thr Ile Arg Ser
            205                 210                 215

aca cta aat ccg cag tgg aat gag tcc ttt aca ttc aaa ttg aaa cct 726
Thr Leu Asn Pro Gln Trp Asn Glu Ser Phe Thr Phe Lys Leu Lys Pro
        220                 225                 230

tca gac aaa gac cga cga ctg tct gta gaa atc tgg gac tgg gat cga 774
Ser Asp Lys Asp Arg Arg Leu Ser Val Glu Ile Trp Asp Trp Asp Arg
    235                 240                 245

aca aca agg aat gac ttc atg gga tcc ctt tcc ttt gga gtt tcg gag 822
Thr Thr Arg Asn Asp Phe Met Gly Ser Leu Ser Phe Gly Val Ser Glu
250                 255                 260                 265

ctg atg aag atg ccg gcc agt gga tgg tac aag ttg ctt aac caa gaa 870
Leu Met Lys Met Pro Ala Ser Gly Trp Tyr Lys Leu Leu Asn Gln Glu
                270                 275                 280

gaa ggt gag tac tac aac gta ccc att ccg gaa ggg gac gag gaa gga 918
Glu Gly Glu Tyr Tyr Asn Val Pro Ile Pro Glu Gly Asp Glu Glu Gly
            285                 290                 295

aac atg gaa ctc agg cag aaa ttc gag aaa gcc aaa ctt ggc cct gct 966
Asn Met Glu Leu Arg Gln Lys Phe Glu Lys Ala Lys Leu Gly Pro Ala
        300                 305                 310

ggc aac aaa gtc atc agt ccc tct gaa gac agg aaa caa cct tcc aac 1014
Gly Asn Lys Val Ile Ser Pro Ser Glu Asp Arg Lys Gln Pro Ser Asn
    315                 320                 325

aac ctt gac cga gtg aaa ctc acg gac ttc aat ttc ctc atg gtg ttg 1062
Asn Leu Asp Arg Val Lys Leu Thr Asp Phe Asn Phe Leu Met Val Leu
330                 335                 340                 345

gga aag ggg agt ttt gga aag gtg atg ctt gcc gac agg aag ggc aca 1110
Gly Lys Gly Ser Phe Gly Lys Val Met Leu Ala Asp Arg Lys Gly Thr
                350                 355                 360

gaa gaa ctg tat gca atc aaa atc ctg aag aag gat gtg gtg att cag 1158
Glu Glu Leu Tyr Ala Ile Lys Ile Leu Lys Lys Asp Val Val Ile Gln
            365                 370                 375

gat gat gac gtg gag tgc acc atg gta gaa aag cga gtc ttg gcc ctg 1206
Asp Asp Asp Val Glu Cys Thr Met Val Glu Lys Arg Val Leu Ala Leu
        380                 385                 390

ctt gac aaa ccc ccg ttc ttg acg cag ctg cac tcc tgc ttc cag aca 1254
Leu Asp Lys Pro Pro Phe Leu Thr Gln Leu His Ser Cys Phe Gln Thr
    395                 400                 405

gtg gat cgg ctg tac ttc gtc atg gaa tat gtc aac ggt ggg gac ctc 1302
Val Asp Arg Leu Tyr Phe Val Met Glu Tyr Val Asn Gly Gly Asp Leu
410                 415                 420                 425

atg tac cac att cag caa gta gga aaa ttt aag gaa cca caa gca gta     1350
Met Tyr His Ile Gln Gln Val Gly Lys Phe Lys Glu Pro Gln Ala Val
                430                 435                 440

ttc tat gcg gca gag att tcc atc gga ttg ttc ttt ctt cat aaa aga     1398
Phe Tyr Ala Ala Glu Ile Ser Ile Gly Leu Phe Phe Leu His Lys Arg
            445                 450                 455

gga atc att tat agg gat ctg aag tta gat aac gtc atg ttg gat tca     1446
Gly Ile Ile Tyr Arg Asp Leu Lys Leu Asp Asn Val Met Leu Asp Ser
        460                 465                 470

gaa gga cat atc aaa att gct gac ttt ggg atg tgc aag gaa cac atg     1494
Glu Gly His Ile Lys Ile Ala Asp Phe Gly Met Cys Lys Glu His Met
    475                 480                 485

atg gat gga gtc acg acc agg acc ttc tgt ggg act cca gat tat atc     1542
Met Asp Gly Val Thr Thr Arg Thr Phe Cys Gly Thr Pro Asp Tyr Ile
490                 495                 500                 505

gcc cca gag ata atc gct tat cag ccg tat gga aaa tct gtg gac tgg     1590
Ala Pro Glu Ile Ile Ala Tyr Gln Pro Tyr Gly Lys Ser Val Asp Trp
                510                 515                 520

tgg gcc tat ggc gtc ctg ttg tat gaa atg ctt gcc ggg cag cct cca     1638
Trp Ala Tyr Gly Val Leu Leu Tyr Glu Met Leu Ala Gly Gln Pro Pro
            525                 530                 535

ttt gat ggt gaa gat gaa gac gag cta ttt cag tct atc atg gag cac     1686
Phe Asp Gly Glu Asp Glu Asp Glu Leu Phe Gln Ser Ile Met Glu His
        540                 545                 550

aac gtt tcc tat cca aaa tcc ttg tcc aag gag gct gtt tct atc tgc     1734
Asn Val Ser Tyr Pro Lys Ser Leu Ser Lys Glu Ala Val Ser Ile Cys
    555                 560                 565

aaa gga ctg atg acc aaa cac cca gcc aag cgg ctg ggc tgt ggg cct     1782
Lys Gly Leu Met Thr Lys His Pro Ala Lys Arg Leu Gly Cys Gly Pro
570                 575                 580                 585

gag ggg gag agg gac gtg aga gag cat gcc ttc ttc cgg agg atc gac     1830
Glu Gly Glu Arg Asp Val Arg Glu His Ala Phe Phe Arg Arg Ile Asp
                590                 595                 600

tgg gaa aaa ctg gag aac agg gag atc cag cca cca ttc aag ccc aaa     1878
Trp Glu Lys Leu Glu Asn Arg Glu Ile Gln Pro Pro Phe Lys Pro Lys
            605                 610                 615

gtg tgt ggc aaa gga gca gag aac ttt gac aag ttc ttc aca cga gga     1926
Val Cys Gly Lys Gly Ala Glu Asn Phe Asp Lys Phe Phe Thr Arg Gly
        620                 625                 630

cag ccc gtc tta aca cca cct gat cag ctg gtt att gct aac ata gac     1974
Gln Pro Val Leu Thr Pro Pro Asp Gln Leu Val Ile Ala Asn Ile Asp
    635                 640                 645

cag tct gat ttt gaa ggg ttc tcg tat gtc aac ccc cag ttt gtg cac     2022
Gln Ser Asp Phe Glu Gly Phe Ser Tyr Val Asn Pro Gln Phe Val His
650                 655                 660                 665

ccc atc tta cag agt gca gta tga aactcaccag cgagaacaaa cacctcccca     2076
Pro Ile Leu Gln Ser Ala Val
                670

gcccccagcc ctccccgcag tggaagtgaa tccttaaccc taaaatttta aggccacggc     2136

ttgtgtctga ttccatatgg aggcctgaaa attgtagggt tattagtcca aatgtgatca     2196

actgttcagg gtctctctct tacaaccaag aacattatct tagtggaag                 2245

<210>  6
<211>  672
<212>  PRT
<213>  homo sapiens

<400>  6

Met Ala Asp Val Phe Pro Gly Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Ala Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Glu Gly Asn Met Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Lys
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Leu Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu

515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys His
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Leu Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ala Val
            660                 665                 670

<210>  7
<211>  2019
<212>  DNA
<213>  Oryctolagus cuniculus

<220>
<221>  CDS
<222>  (1)..(2019)

<400>  7
atg gct gac gtt ttc ccg gcc aac gac tcc acg gcg tct cag gac gtg        48
Met Ala Asp Val Phe Pro Ala Asn Asp Ser Thr Ala Ser Gln Asp Val
1               5                   10                  15

gcc aac cgc ttc gcc cgc aaa ggg gcg ctg agg cag aag aac gtg cac        96
Ala Asn Arg Phe Ala Arg Lys Gly Ala Leu Arg Gln Lys Asn Val His
            20                  25                  30

gag gtg aaa gac cac aaa ttc atc gcg cgc ttc ttc aag cag ccc acc       144
Glu Val Lys Asp His Lys Phe Ile Ala Arg Phe Phe Lys Gln Pro Thr
        35                  40                  45

ttc tgc agc cac tgc acc gac ttc atc tgg ggg ttt ggg aag caa ggc       192
Phe Cys Ser His Cys Thr Asp Phe Ile Trp Gly Phe Gly Lys Gln Gly
    50                  55                  60

ttc cag tgc caa gtt tgc tgt ttt gtg gtt cac aag agg tgc cat gaa       240
Phe Gln Cys Gln Val Cys Cys Phe Val Val His Lys Arg Cys His Glu
65                  70                  75                  80

ttt gtt acc ttt tct tgt ccg ggt gcg gat aag gga cct gac act gat 288
Phe Val Thr Phe Ser Cys Pro Gly Ala Asp Lys Gly Pro Asp Thr Asp
                85                  90                  95

gac ccc agg agc aag cac aag ttt aaa atc cac acc tac ggg agc ccc 336
Asp Pro Arg Ser Lys His Lys Phe Lys Ile His Thr Tyr Gly Ser Pro
            100                 105                 110

acc ttc tgc gat cac tgt ggg tca ctg ctg tac gga ctc atc cac cag 384
Thr Phe Cys Asp His Cys Gly Ser Leu Leu Tyr Gly Leu Ile His Gln
        115                 120                 125

ggg atg aaa tgt gac acc tgc gac atg aac gtt cac aag cag tgt gtg 432
Gly Met Lys Cys Asp Thr Cys Asp Met Asn Val His Lys Gln Cys Val
    130                 135                 140

atc aat gtc ccc agc ctg tgt ggg atg gac cat acg gag aag agg gga 480
Ile Asn Val Pro Ser Leu Cys Gly Met Asp His Thr Glu Lys Arg Gly
145                 150                 155                 160

cgg atc tac ctg aag gcc gag gtc acc gat gaa aag ctc cac gtc acg 528
Arg Ile Tyr Leu Lys Ala Glu Val Thr Asp Glu Lys Leu His Val Thr
                165                 170                 175

gta cga gat gca aaa aac ctg atc ccc atg gat ccc aac ggg ctt tca 576
Val Arg Asp Ala Lys Asn Leu Ile Pro Met Asp Pro Asn Gly Leu Ser
            180                 185                 190

gat ccc tac gtg aag ctg aaa ctc att cct gat ccc aag aat gaa agc 624
Asp Pro Tyr Val Lys Leu Lys Leu Ile Pro Asp Pro Lys Asn Glu Ser
        195                 200                 205

aaa caa aaa acc aaa acc atc cgc tcc acg ctg aac ccg cag tgg aac 672
Lys Gln Lys Thr Lys Thr Ile Arg Ser Thr Leu Asn Pro Gln Trp Asn
    210                 215                 220

gaa tcc ttt acc ttc aaa tta aaa ccc tcc gac aaa gac cga cga ctg 720
Glu Ser Phe Thr Phe Lys Leu Lys Pro Ser Asp Lys Asp Arg Arg Leu
225                 230                 235                 240

tct gta gaa atc tgg gac tgg gac cga aca aca agg aat gac ttc atg 768
Ser Val Glu Ile Trp Asp Trp Asp Arg Thr Thr Arg Asn Asp Phe Met
                245                 250                 255

ggg tcg ctc tcc ttt ggc gtc tcg gag ctc atg aag atg cca gcc agt 816
Gly Ser Leu Ser Phe Gly Val Ser Glu Leu Met Lys Met Pro Ala Ser
            260                 265                 270

gga tgg tac aag ttg ctg aac caa gaa gaa ggt gaa tac tac aat gtg 864
Gly Trp Tyr Lys Leu Leu Asn Gln Glu Glu Gly Glu Tyr Tyr Asn Val
        275                 280                 285

ccc att ccg gaa ggg gac gaa gat ggg aac gtg gag ctc agg cag aaa 912
Pro Ile Pro Glu Gly Asp Glu Asp Gly Asn Val Glu Leu Arg Gln Lys
    290                 295                 300

ttc gag aaa gcc aag ctg ggg ccc gct ggc aac aag gtg atc agt ccc 960
Phe Glu Lys Ala Lys Leu Gly Pro Ala Gly Asn Lys Val Ile Ser Pro
305                 310                 315                 320

tcg gaa gac agg aag cag ccg tcc aac aac ctg gac cga gtg aag ctc 1008
Ser Glu Asp Arg Lys Gln Pro Ser Asn Asn Leu Asp Arg Val Lys Leu
                325                 330                 335

acc gac ttc aac ttc ctc atg gtc ctg gga aaa ggc agt ttt ggg aag 1056
Thr Asp Phe Asn Phe Leu Met Val Leu Gly Lys Gly Ser Phe Gly Lys
            340                 345                 350

gtg atg ctg gcg gac agg aag ggc acg gaa gag ctg tac gcc atc agg     1104
Val Met Leu Ala Asp Arg Lys Gly Thr Glu Glu Leu Tyr Ala Ile Arg
        355                 360                 365

atc ctg aag aag gac gtg gtg att cag gat gac gac gtg gag tgc acc     1152
Ile Leu Lys Lys Asp Val Val Ile Gln Asp Asp Asp Val Glu Cys Thr
    370                 375                 380

atg gtg gag aag cgg gtt ctg gcc ctg atg gac aag ccg ccc ttc ctg     1200
Met Val Glu Lys Arg Val Leu Ala Leu Met Asp Lys Pro Pro Phe Leu
385                 390                 395                 400

aca cag ctg cac tcc tgc ttc cag acc gtg gac cgg ctg tac ttt gtc     1248
Thr Gln Leu His Ser Cys Phe Gln Thr Val Asp Arg Leu Tyr Phe Val
                405                 410                 415

atg gaa tac gtc aac ggc gga gac ctc atg tac cac atc cag caa gtg     1296
Met Glu Tyr Val Asn Gly Gly Asp Leu Met Tyr His Ile Gln Gln Val
            420                 425                 430

gga aag ttc aag gag cca caa gca gta ttc tat gca gca gag att tcc     1344
Gly Lys Phe Lys Glu Pro Gln Ala Val Phe Tyr Ala Ala Glu Ile Ser
        435                 440                 445

att ggg ctg ttc ttc ctt cac aaa aga ggg atc atc tat agg gac ctg     1392
Ile Gly Leu Phe Phe Leu His Lys Arg Gly Ile Ile Tyr Arg Asp Leu
    450                 455                 460

aag cta gat aac gtc atg ctg gac tcg gaa gga cac atc aaa atc gct     1440
Lys Leu Asp Asn Val Met Leu Asp Ser Glu Gly His Ile Lys Ile Ala
465                 470                 475                 480

gac ttt ggg atg tgc aag gaa cac atg atg gac ggg gtc acc acc agg     1488
Asp Phe Gly Met Cys Lys Glu His Met Met Asp Gly Val Thr Thr Arg
                485                 490                 495

acc ttc tgt ggg act cca gat tac atc gcc ccg gag atc atc gct tat     1536
Thr Phe Cys Gly Thr Pro Asp Tyr Ile Ala Pro Glu Ile Ile Ala Tyr
            500                 505                 510

cag ccg tac ggc aaa tcc gtg gac tgg tgg gcc tac ggc gtc ctg ctg     1584
Gln Pro Tyr Gly Lys Ser Val Asp Trp Trp Ala Tyr Gly Val Leu Leu
        515                 520                 525

tat gag atg ctg gct ggg cag cct cca ttt gat ggc gag gac gaa gac     1632
Tyr Glu Met Leu Ala Gly Gln Pro Pro Phe Asp Gly Glu Asp Glu Asp
    530                 535                 540

gag ctg ttt cag tcc atc atg gag cac aac gtt tcc tac ccc aaa tcc     1680
Glu Leu Phe Gln Ser Ile Met Glu His Asn Val Ser Tyr Pro Lys Ser
545                 550                 555                 560

ttg tcc aag gag gcc gtc tcc atc tgc aag ggg ctg atg acc aaa cac     1728
Leu Ser Lys Glu Ala Val Ser Ile Cys Lys Gly Leu Met Thr Lys His
                565                 570                 575

ccg gcc aag cgg ctg ggc tgc ggg ccc gag gga gag cgg gac gtg agg     1776
Pro Ala Lys Arg Leu Gly Cys Gly Pro Glu Gly Glu Arg Asp Val Arg
            580                 585                 590

gaa cac gcc ttc ttc cgg agg atc gac tgg gag aaa ctg gag aac agg     1824
Glu His Ala Phe Phe Arg Arg Ile Asp Trp Glu Lys Leu Glu Asn Arg
        595                 600                 605

gag atc cag ccg ccg ttc aag ccc aaa gtg tgt ggc aaa gga gca gaa     1872
Glu Ile Gln Pro Pro Phe Lys Pro Lys Val Cys Gly Lys Gly Ala Glu
    610                 615                 620

aac ttc gac aag ttc ttc acg cga ggg cag ccc gtc gtc acg ccg ccg     1920

Asn Phe Asp Lys Phe Phe Thr Arg Gly Gln Pro Val Val Thr Pro Pro
625                 630                 635                 640

gat caa ctg gtc atc gct aac ata gac cag tct gat ttc gaa ggg ttc      1968
Asp Gln Leu Val Ile Ala Asn Ile Asp Gln Ser Asp Phe Glu Gly Phe
                645                 650                 655

tcg tat gtc aac ccc cag ttc gtg cac ccg atc cta cag agc tca gta      2016
Ser Tyr Val Asn Pro Gln Phe Val His Pro Ile Leu Gln Ser Ser Val
            660                 665                 670

tga                                                                  2019

<210>  8
<211>  672
<212>  PRT
<213>  Oryctolagus cuniculus

<400>  8

Met Ala Asp Val Phe Pro Ala Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Thr Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Asp Gly Asn Val Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Arg
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Met Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala

465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys His
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Val Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ser Val
            660                 665                 670

<210>  9
<211>  147
<212>  DNA
<213>  Mus musculus

<220>
<221>  CDS
<222>  (2)..(145)

<400>  9
g ttt gag aaa gcc aag cta ggc cct gct ggt aac aaa gtc atc agc cct      49
  Phe Glu Lys Ala Lys Leu Gly Pro Ala Gly Asn Lys Val Ile Ser Pro
  1               5                   10                  15

tca gaa gac aga aag caa cca tcc aac aac ctg gac aga gtg aaa ctc        97
Ser Glu Asp Arg Lys Gln Pro Ser Asn Asn Leu Asp Arg Val Lys Leu
            20                  25                  30

aca gac ttc aac ttc ctc atg gtg ctg ggg aag ggg agt ttt ggg aag      145
Thr Asp Phe Asn Phe Leu Met Val Leu Gly Lys Gly Ser Phe Gly Lys
        35                  40                  45

gt                                                                   147

<210>  10
<211>  48
<212>  PRT
<213>  Mus musculus

<400>  10

Phe Glu Lys Ala Lys Leu Gly Pro Ala Gly Asn Lys Val Ile Ser Pro
1               5                   10                  15

Ser Glu Asp Arg Lys Gln Pro Ser Asn Asn Leu Asp Arg Val Lys Leu
            20                  25                  30

Thr Asp Phe Asn Phe Leu Met Val Leu Gly Lys Gly Ser Phe Gly Lys
        35                  40                  45

<210>  11
<211>  32
<212>  DNA
<213>  Mus musculus

<400>  11
gactgtcgag tcgaccgagg tgtccctgta gt                                   32

<210>  12
<211>  29
<212>  DNA
<213>  Mus musculus

<400>  12
caatagccag tcgactcgat ccacagtca                                       29

<210>  13
<211>  33
<212>  DNA
<213>  Mus musculus

<400>  13
tagtcaacaa tcgatggacg gtacttctgc gca                                  33

<210>  14
<211>  30
<212>  DNA
<213>  Mus musculus

<400>  14
acttgtcata tcgatgaaat gagttaccca                                      30

<210>  15
<211>  5443
<212>  DNA
<213>  Mus musculus

<400>  15
   ggatcctgca aggtcacaca agggtctcca cccaccaggt gccctagtct caatttcagt 60
   ttccatgcct tgttctcaca atgctggcct ccccagagct aatttggact ttgtttttat 120
   ttcaaaaggg cctgaatgag gagtagatct tgtgctaccc agctctaagg gtgcccgtga 180
   agccctcaga cctggagcct ttgcaacagc cctttaggtg gaagcagaat aaagcaattt 240
   tccttaaagc caaaatcctg cctctagact cttcttctct gacctcggtc cctgggctct 300
   agggtgggga ggtggggctt ggaagaagaa ggtggggaag tggcaaaagc cgatccctag 360
   ggccctgtga agttcggagc cttccctgta cagcactggc tcatagatcc tcctccagcc 420
   aaacatagca agaagtgata cctcctttgt gacttcccca ggcccagtac ctgtcaggtt 480
   gaaacaggat ttagagaagc ctctgaactc acctgaactc tgaagctcat ccaccaagca 540
   agcacctagg tgccactgct agttagtatc ctacgctgat aatatgcaga gctgggccac 600
   agaagtcctg gggtgtagga actgaccagt gacttttcag tcggcaaagg tatgaccccc 660
   tcagcagatg tagtaatgtc cccttagatc ccatcccagg caggtctcta agaggacatg 720
   ggatgagaga tgtagtcatg tggcattcca aacacagcta tccacagtgt cccttgcccc 780
   ttccacttag ccaggaggac agtaacctta gcctatcttt cttcctcccc atcctcccag 840
   gacacacccc ctggtctgca gtattcattt cttccttcac gtcccctctg tgacttccat 900
   ttgcaaggct tttgacctct gcagctgctg gaagatagag tttggcccta ggtgtggcaa 960
   gccatctcaa gagaaagcag acaacagggg gaccagattt tggaaggatc aggaactaaa 1020
   tcactggcgg gcctgggggt agaaaaaaga gtgagtgagt ccgctccagc taagccaagc 1080
   tagtccccga gatactctgc cacagctggg ctgctcgggg tagctttagg aatgtgggtc 1140
   tgaaagacaa tgggattgga agacatctct ttgagtctcc cctcaacccc acctacagac 1200
   acactcgtgt gtggccagac tcctgttcaa cagccctctg tgttctgacc actgagctag 1260
   gcaaccagag catgggccct gtgctgagga tgaagagttg gttaccaata gcaaaaacag 1320
   caggggaggg agaacagaga acgaaataag gaaggaagaa ggaaaggcca gtcaatcaga 1380
   tgcagtcaga agagatggga agccaacaca cagcttgagc agaggaaaca gaaaagggag 1440
   agattctggg cataaggagg ccacagaaag aagagcccag gccccccaag tctcctcttt 1500
   ataccctcat cccgtctccc aattaagccc actcttcttc ctagatcaga cctgagctgc 1560
   agcgaagaga cccgtaggga ggatcacact ggatgaagga gatgtgtgga gaagtccagg 1620
   gcaacctaag agccagagcc taaaagagca agagataaag gtgcttcaaa ggtggccagg 1680
   ctgtgcacac agagggtcga ggactggtgg tagagcctca agataaggat gatgctcaga 1740
   atgggcgggg ggggggattc tggggggggg agagagaagg tgagaaggag cctggaacag 1800
   agaatctgga agcgctggaa acgataccat aaagggaaga acccaggcta cctttagatg 1860
   taaatcatga aagacaggga gaagggaagc tggagagagt agaaggaccc cggggcaaga 1920
   catggaagca aggacaagcc aggttgagcg ctccgtgaaa tcagcctgct gaaggcagag 1980
   ccctggtatg agcaccagaa cagcagaggc tagggttaat gtcgagacag ggaacagaag 2040
   gtagacacag gaacagacag agacggggga gccaggtaac aaaggaatgg tccttctcac 2100
   ctgtggccag agcgtccatc tgtgtccaca tactctagaa tgttcatcag actgcagggc 2160
   tggcttggga ggcagctgga aagagtatgt gagagccagg ggagacaagg gggcctagga 2220
   aaggaagaag agggcaaacc aggccacaca agagggcaga gcccagaact gagttaactc 2280
   cttccttgtt gcatcttcca taggaggcag tgggaactct gtgaccacca tcccccatga 2340
   gcccccacta cccataccaa gtttggcctg agtggcattc taggttccct gaggacagag 2400
   cctggccttt gtctcttgga cctgacccaa gctgacccaa tgttctcagt accttatcat 2460
   gccctcaaga gcttgagaac caggcagtga catattaggc catgggctaa ccctggagct 2520
   tgcacacagg agcctcaagt gacctccagg gacacagctg cagacaggtg gcctttatcc 2580
   ccaaagagca accatttggc ataggtggct gcaaatggga atgcaaggtt gaatcaggtc 2640
   ccttcaagaa tactgcatgc aagacctaag acccctggag agaggggtat gctcctgccc 2700
   ccacccacca taaggggagt gaactatcct agggggctgg cgaccttggg gagacaccac 2760
   attactgaga gtgctgagcc cagaaaaact gaccgccctg tgtcctgccc acctccacac 2820
   tctagagcta tattgagagg tgacagtaga tagggtggga gctggtagca gggagagtgt 2880
   tcctgggtgt gagggtgtag gggaaagcca gagcagggga gtctggcttt gtctcctgaa 2940
   cacaatgtct acttagttat aacaggcatg acctgctaaa gacccaacat ctacgacctc 3000
   tgaaaagaca gcagccctgg aggacagggg ttgtctctga gccttgggtg cttgatggtg 3060
   ccacaaagga gggcatgagt gtgagtataa ggccccagga gcgttagaga agggcacttg 3120
   ggaaggggtc agtctgcaga gcccctatcc atggaatctg gagcctgggg ccaactggtg 3180
   taaatctctg ggcctgccag gcattcaaag cagcacctgc atcctctggc agcctgggga 3240
   ggcggaaggg agcaaccccc cacttatacc ctttctccct cagccccagg attaacacct 3300
   ctggccttcc cccttcccac ctcccatcag gagtggaggg ttgcagaggg agggtaaaaa 3360
   cctacatgtc caaacatcat ggtgcacgat atatggatca gtatgtgtag aggcaagaaa 3420
   ggaaatctgc aggcttaact gggttaatgt gtaaagtctg tgtgcatgtg tgtgtgtctg 3480
   actgaaaacg ggcatggctg tgcagctgtt cagttctgtg cgtgaggtta ccagactgca 3540
   ggtttgtgtg taaattgccc aaggcaaagt gggtgaatcc cttccatggt ttaaagagat 3600
   tggatgatgg cctgcatctc aaggaccatg gaaaatagaa tggacactct atatgtgtct 3660
   ctaagctaag gtagcaaggt ctttggagga cacctgtcta gagatgtggg caacagagac 3720
   tacagacagt atctgtacag agtaaggaga gagaggaggg ggtgtagaat tctcttacta 3780
   tcaaagggaa actgagtcgt gcacctgcaa agtggatgct ctccctagac atcatgactt 3840
   tgtctctggg gagccagcac tgtggaactt caggtctgag agagtaggag gctcccctca 3900
   gcctgaagct atgcagatag ccagggttga aagggggaag ggagagcctg ggatgggagc 3960
   ttgtgtgttg gaggcagggg acagatatta agcctggaag agaaggtgac ccttacccag 4020
   ttgttcaact cacccttcag attaaaaata actgaggtaa gggcctgggt aggggaggtg 4080
   gtgtgagacg ctcctgtctc tcctctatct gcccatcggc cctttgggga ggaggaatgt 4140
   gcccaaggac taaaaaaagg ccatggagcc agaggggcga gggcaacaga cctttcatgg 4200
   gcaaaccttg gggccctgct gtcctcctgt cacctccaga gccaagggat caaaggagga 4260
   ggagccagga caggagggaa gtgggaggga gggtcccagc agaggactcc aaatttaggc 4320
   agcaggcata tgggatggga tataaagggg ctggagcact gagagctgtc agagatttct 4380
   ccaacccagg taagagggag tttcgggtgg gggctcttca cccacaccag acctctcccc 4440
   acctagaagg aaactgcctt tcctggaagt ggggttcagg ccggtcagag atctgacagg 4500
   gtggccttcc accagcctgg gaagttctca gtggcaggag gtttccacaa gaaacactgg 4560
   atgccccttc ccttacgctg tcttctccat cttcctcctg gggatgctcc tccccgtctt 4620
   ggtttatctt ggctcttcgt cttcagcaag atttgccctg tgctgtccac tccatctttc 4680
   tctactgtct ccgtgccttg ccttgccttc ttgcgtgtcc ttcctttcca cccatttctc 4740
   acttcacctt ttctcccctt ctcatttgta ttcatccttc cttccttcct tccttccttc 4800
   cttccttcct tccttccttc ctttctccct tccttccttc cttccttcct tccttccttc 4860
   cttccttcct gtgtcagagt gctgagaatc acacctgggg ttcccaccct tatgtaaaca 4920
   atcttccagt gagccacagc ttcagtgctg ctgggtgctc tcttaccttc ctcaccccct 4980
   ggcttgtcct gttccatcct ggtcaggatc tctagattgg tctcccagcc tctgctactc 5040
   ctcttcctgc ctgttcctct ctctgtccag ctgcgccact gtggtgcctc gttccagctg 5100
   tggtccacat tcttcaggat tctctgaaaa gttaaccagg tgagaatgtt tcccctgtag 5160
   acagcagatc acgattctcc cggaagtcag gcttccagcc ctctctttct ctgcccagct 5220
   gcccggcact cttagcaaac ctcaggcacc cttaccccac atagacctct gacagagaag 5280
   caggcacttt acatggagtc ctggtgggag agccataggc tacggtgtaa aagaggcagg 5340
   gaagtggtgg tgtaggaaag tcaggacttc acatagaagc ctagcccaca ccagaaatga 5400
   cagacagatc cctcctatct cccccataag agtttgagtc gac 5443